(19) Europäisches Patentamt
European Patent Office
Office européen des brevets

(11) EP 1 396 538 A1

(12) **EUROPEAN PATENT APPLICATION**
published in accordance with Art. 158(3) EPC

(43) Date of publication:
**10.03.2004 Bulletin 2004/11**

(21) Application number: **02736063.5**

(22) Date of filing: **12.06.2002**

(51) Int Cl.7: **C12N 15/09**, C12Q 1/68

(86) International application number:
**PCT/JP2002/005832**

(87) International publication number:
**WO 2002/101042 (19.12.2002 Gazette 2002/51)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **12.06.2001 JP 2001177737**
**20.08.2001 JP 2001249689**

(71) Applicant: **TAKARA BIO INC.**
**Otsu-shi, Shiga 520-2193 (JP)**

(72) Inventors:
- **SAGAWA, Hiroaki**
  **Kusatsu-shi, Shiga 525-0025 (JP)**
- **UEMORI, Takashi**
  **Otsu-shi, Shiga 520-2141 (JP)**
- **MUKAI, Hiroyuki**
  **Moriyama-shi, Shiga 524-0102 (JP)**
- **YAMAMOTO, Junko**
  **Moriyama-shi, Shiga 524-0044 (JP)**
- **TOMONO, Jun;**
  **Kusatsu-shi, Shiga 525-0025 (JP)**
- **KOBAYASHI, Eiji**
  **Otsu-shi, Shiga 520-2153 (JP)**
- **ENOKI, Tatsuji**
  **Otsu-shi, Shiga 520-0865 (JP)**
- **ASADA, Kiyozo**
  **Koka-gun, Shiga 520-3333 (JP)**
- **KATO, Ikunoshin**
  **Koka-gun, Shiga 529-18518 (JP)**

(74) Representative: **Grund, Martin, Dr. et al**
**Dr. Volker Vossius**
**Patent- & Rechtsanwaltskanzlei**
**Geibelstrasse 6**
**81679 München (DE)**

(54) **METHOD OF STABILIZING REAGENT FOR AMPLIFYING OR DETECTING NUCLEIC ACID AND STORAGE METHOD**

(57) A method of stabilizing a reaction reagent for highly sensitively and specifically amplifying a target nucleic acid in a sample with the use of a chimeric oligonucleotide primer and a method of storing the same over a long time; and a method of highly sensitively detecting a pathogenic microorganism and a virus.

EP 1 396 538 A1

SEQUENCE LISTING

<110> TAKARA BIO INC.

<120> A stabilization method and a preservation method for a reagent for nucleic acid amplification or detection reaction

<130> 663232

<150> JP 2001-177737
<151> 2001-06-12

<150> JP 2001-249689
<151> 2001-08-20

<160> 173

<210> 1
<211> 25
<212> DNA
<213> Artificial Sequence

<220>
<223> Designed oligonucleotide primer designated as pUC19 upper 150 to amplify a portion of plasmid pUC19.

<400> 1
ggtgtcacgc tcgtcgtttg gtatg 25

<210> 2

<211> 25

<212> DNA

<213> Artificial Sequence

<220>

<223> Designed chimeric oligonucleotide primer designated as pUC19 lower NN to amplify a portion of plasmid pUC19.

<400> 2

gataacactg cggccaactt acttc 25

<210> 3

<211> 22

<212> DNA

<213> Artificial Sequence

<220>

<223> Designed oligonucleotide primer designated as MCS-F to amplify a long DNA fragment

<400> 3

ccattcaggc tgcgcaactg tt 22

<210> 4

<211> 22

<212> DNA

<213> Artificial Sequence

<220>

<223> Designed oligonucleotide primer designated as MCS-R to amplify a long DNA fragment

<400> 4

tggcacgaca ggtttcccga ct 22

<210> 5

<211> 30

<212> DNA

<213> Artificial Sequence

<220>

<223> Designed oligonucleotide primer designated as MF2 to amplify a portion of pUC19 plasmid DNA.

<400> 5

ggatgtgctg caaggcgatt aagttgggta 30

<210> 6

<211> 30

<212> DNA

<213> Artificial Sequence

<220>

<223> Designed oligonucleotide primer designated as MR1 to amplify a portion of pUC19 plasmid DNA.

<400> 6

tttacacttt atgcttccgg ctcgtatgtt 30

<210> 7

<211> 21

<212> DNA

<213> Artificial Sequence

<220>

<223> Designed chimeric oligonucleotide primer designated as K-F-1033-2 to amplify a portion of Mycobacterium tuberculosis DNA. "nucleotides 19 to 21 are ribonucleotides-other nucleotides are deoxyribonucleotides."

<400> 7

cagtacacat cgatccgguu c 21

<210> 8

<211> 22

<212> DNA

<213> Artificial Sequence

<220>

<223> Designed chimeric oligonucleotide primer designated as K-R-1133-2 to amplify a portion of Mycobacterium tuberculosis DNA. "nucleotides 20 to 22 are ribonucleotides-other nucleotides are deoxyribonucleotides."

<400> 8

gatcgtctcg gctagtgcau ug 22

<210> 9

<211> 23

<212> DNA

<213> Artificial Sequence

<220>

<223> Designed chimeric oligonucleotide primer to amplify a portion of INOS-encoding sequence from mouse. "nucleotides 21 to 23 are ribonucleotides-other nucleotides are deoxyribonucleotides"

<400> 9

ctcatgccat tgagttcatc aac 23

<210> 10

<211> 22

<212> DNA

<213> Artificial Sequence

<220>

<223> Designed chimeric oligonucleotide primer to amplify a portion of INOS-encoding sequence from mouse. "nucleotides 20 to 22 are ribonucleotides-other nucleotides are deoxyribonucleotides"

<400> 10

gctggtaggt tcctgttgtu uc 22

<210> 11

<211> 24

<212> RNA

<213> Artificial Sequence

<220>

<223> Designed oligonucleotide probe designated as MTIS to detect a DNA fragment amplifying a portion of nucleotide sequence from Mycobacterium tuberculosis.

<400> 11
accuaugugu cgaccugggc aggg 24

<210> 12
<211> 20
<212> RNA
<213> Artificial Sequence

<220>

<223> Designed oligonucleotide probe designated as MTIS-2 to detect a DNA fragment amplifying a portion of nucleotide sequence from Mycobacterium tuberculosis.

<400> 12
gaccucaccu augugucgac 20

<210> 13
<211> 45
<212> DNA
<213> Artificial Sequence

<220>

<223> Designed oligonucleotide primer designated as SP6-HCV-F to amplify a portion of HCV.

<400> 13
ccatttaggt gacactatag aatactgatg ggggcgacac tccac 45

<210> 14
<211> 45
<212> DNA
<213> Artificial Sequence

<220>
<223> Designed oligonucleotide primer designated as T7-HCV-R to amplify a portion of HCV

<400> 14
agctctaata cgactcacta tagggtcgca agcaccctat caggc 45

<210> 15
<211> 18
<212> DNA
<213> Artificial Sequence

<220>
<223> Designed chimeric oligonucleotide primer designated as HCV-A2-S to amplify a portion of HCV. "nucleotides 16 to 18 are ribonucleotides-other nucleotides are deoxyribonucleotides."

<400> 15
ctttcttgga tcaacccg 18

<210> 16

<211> 20

<212> DNA

<213> Artificial Sequence

<220>

<223> Designed chimeric oligonucleotide primer designated as HCV-A2-A to amplify a portion of HCV. "nucleotides 18 to 20 are ribonucleotides-other nucleotides are deoxyribonucleotides."

<400> 16

aacactactc ggctagcagu 20

<210> 17

<211> 20

<212> DNA

<213> Artificial Sequence

<220>

<223> Designed chimeric oligonucleotide primer designated as CT2F to amplify a portion of Chlamydia trachomatis cryptic plasmid DNA."nucleotides 18 to 20 are ribonucleotides-other nucleotides are deoxyribonucleotides."

<400> 17

ctggatttat cggaaaccuu 20

<210> 18

<211> 18

<212> DNA

<213> Artificial Sequence

<220>

<223> Designed chimeric oligonucleotide primer designated as CT2R to amplify a portion of Chlamydia trachomatis cryptic plasmid DNA."nucleotides 16 to 18 are ribonucleotides-other nucleotides are deoxyribonucleotides."

<400> 18

aggcctctga aacgacuu 18

<210> 19

<211> 22

<212> DNA

<213> Artificial Sequence

<220>

<223> Designed chimeric oligonucleotide primer designated as CT-FB19-3 to amplify a portion of Chlamydia trachomatics cryptic plasmid DNA. "nucleotides 20 to 22 are ribonucleotides-other nucleotides are deoxyribonucleotides."

<400> 19

catacggttt tcctcgatga uu 22

<210> 20

<211> 23

<212> DNA

<213> Artificial Sequence

<220>

<223> Designed chimeric oligonucleotide primer designated as CT-RB23-2 to amplify a portion of Chlamydia trachomatics cryptic plasmid DNA. "nucleotides 21 to 23 are ribonucleotides-other nucleotides are deoxyribonucleotides."

<400> 20

gatctacgca atggattttc auu 23

<210> 21

<211> 22

<212> DNA

<213> Artificial Sequence

<220>

<223> Designed chimeric oligonucleotide primer designated as K-F-1033(68) to amplify a portion of Mycobacterium tuberculosis DNA."nucleotides 20 to 22 are ribonucleotides-other nucleotides are deoxyribonucleotides."

<400> 21

gtacacatcg atccggttca gc 22

<210> 22

<211> 22

<212> DNA

<213> Artificial Sequence

<220>

<223> Designed chimeric oligonucleotide primer designated as K-R-1133(68) to amplify a portion of Mycobacterium tuberculosis DNA."nucleotides 20 to 22 are ribonucleotides-other nucleotides are deoxyribonucleotides."

<400> 22

gttgatcgtc tcggctagtg ca 22

<210> 23

<211> 560

<212> DNA

<213> Mycobacterium avium

<400> 23

gacgaacgct ggcggcgtgc ttaacacatg caagtcgaac ggaaaggcct cttcggaggt 60
actcgagtgg cgaacgggtg agtaacacgt gggcaatctg ccctgcactt cgggataagc 120
ctgggaaact gggtctaata ccggatagga cctcaagacg catgtcttct ggtggaaagc 180
ttttgcggtg tgggatgggc ccgcggccta tcagcttgtt ggtggggtga cggcctacca 240
aggcgacgac gggtagccgg cctgagaggg tgtccggcca cactgggact gagatacggc 300
ccagactcct acgggaggca gcagtgggga atattgcaca atgggcgcaa gcctgatgca 360
gcgacgccgc gtgggggatg acggccttcg ggttgtaaac ctctttcacc atcgacgaag 420
gtccgggttt tctcggattg acggtaggtg gagaagaagc accggccaac tacgtgccag 480
cagccgcggt aatacgtagg gtgcgagcgt tgtccggaat tactgggcgt aaagagctcg 540
taggtggttt gtcgcgttgt 560

<210> 24

<211> 560

<212> DNA

<213> Mycobacterium intracellulare

<400> 24

atcctggctc aggacgaacg ctggcggcgt gcttaacaca tgcaagtcga acggaaaggc 60

cccttcgggg gtactcgagt ggcgaacggg tgagtaacac gtgggcaatc tgccctgcac 120

ttcgggataa gcctgggaaa ctgggtctaa taccggatag gacctttagg cgcatgtctt 180

taggtggaaa gcttttgcgg tgtgggatgg gcccgcggcc tatcagcttg ttggtggggt 240

gatggcctac caaggcgacg acgggtagcc ggcctgagag ggtgtccggc cacactggga 300

ctgagatacg gcccagactc ctacgggagg cagcagtggg gaatattgca caatgggcgc 360

aagcctgatg cagcgacgcc gcgtggggga tgacggcctt cgggttgtaa acctctttca 420

ccatcgacga aggtccgggt tttctcggat tgacggtagg tggagaagaa gcaccggcca 480

actacgtgcc agcagccgcg gtaatacgta gggtgcgagc gttgtccgga attactgggc 540

gtaaagagct cgtaggtggt 560

<210> 25

<211> 20

<212> DNA

<213> Artificial Sequence

<220>

<223> Designed chimeric oligonucleotide primer designated as Myco-F-1 to amplify a portion of Mycobacterium 16S rRNA. "Nucleotides 18 to 20 are ribonucleotides-other nucleotides are deoxyribonucleotides."

<400> 25

caatctgccc tgcacttcgg 20

<210> 26
<211> 18
<212> DNA
<213> Artificial Sequence

<220>
<223> Designed chimeric oligonucleotide primer designated as Myco-F-2 to amplify a portion of Mycobacterium 16S rRNA. "Nucleotides 16 to 18 are ribonucleotides-other nucleotides are deoxyribonucleotides."

<400> 26
caatctgccc tgcacuuc 18

<210> 27
<211> 18
<212> DNA
<213> Artificial Sequence

<220>
<223> Designed chimeric oligonucleotide primer designated as Myco-F-3 to amplify a portion of Mycobacterium 16S rRNA. "Nucleotides 16 to 18 are ribonucleotides-other nucleotides are deoxyribonucleotides."

<400> 27
gtgggcaatc tgcccugc 18

<210> 28
<211> 19
<212> DNA

<213> Artificial Sequence

<220>
<223> Designed chimeric oligonucleotide primer designated as Myco-R-1 to amplify a portion of Mycobacterium 16S rRNA. "Nucleotides 17 to 19 are ribonucleotides-other nucleotides are deoxyribonucleotides."

<400> 28
cccacaccgc aaaagcuuu 19

<210> 29
<211> 18
<212> DNA
<213> Artificial Sequence

<220>
<223> Designed chimeric oligonucleotide primer designated as Myco-R-1-2 to amplify a portion of Mycobacterium 16S rRNA. "Nucleotides 16 to 18 are ribonucleotides-other nucleotides are deoxyribonucleotides."

<400> 29
accgcaaaag ctttccac 18

<210> 30
<211> 21
<212> DNA
<213> Artificial Sequence

<220>

<223> Designed chimeric oligonucleotide primer designated as Myco-R-2I to amplify a portion of 16S rRNA-encoding sequence from Mycobacterium intracellulare. "Nucleotides 19 to 21 are ribonucleotides-other nucleotides are deoxyribonucleotides."

<400> 30

accgcaaaag ctttccaccu a 21

<210> 31

<211> 21

<212> DNA

<213> Artificial Sequence

<220>

<223> Designed chimeric oligonucleotide primer designated as Myco-R-2A to amplify a portion of 16S rRNA-encoding sequence from Mycobacterium avium. "Nucleotides 19 to 21 are ribonucleotides-other nucleotides are deoxyribonucleotides."

<400> 31

accgcaaaag ctttccacca g 21

<210> 32

<211> 14

<212> DNA

<213> Artificial Sequence

<220>

<223> Designed oligonucleotide probe as avium-probe to detect a DNA fragment amplifing a portion of 16S rRNA-encoding sequence from Mycobacterium avium.

<400> 32
atgcgtcttg aggt 14

<210> 33
<211> 14
<212> DNA
<213> Artificial Sequence

<220>
<223> Designed oligonucleotide probe as intracellulare-probe to detect a DNA fragment amplifing a portion of 16S rRNA-encoding sequence from Mycobacterium intracellurare.

<400> 33
atgcgcctaa aggt 14

<210> 34
<211> 560
<212> DNA
<213> Neisseria gonorrhorae

<400> 34
cgctctgccg ctctaactcg gctgccaagc tcgctagctg ctgcgctaaa ctcgtgtttt 60
cctgctctag ctctgccaac ctttcgccca agtgcgttaa ggctttcatc attcgctgct 120
cgattgctgc gtgattgctc tctaattccg ctaacgcgtc cagcattcgc ttctcggtcg 180
ctacgcatac ccgcgttgct ttgctgttct cgactgggca attttccagt gtcaaacctt 240

tggtcttggt ttccaacagg tctagggtgc gctctgcttc ggctctctgc tgtttcaagt 300
cgtccagctc gttcttgacg ctccatatcg ctatgaacag ccctgctatg actatcaacc 360
ctgccgccga tatacctagc aagctccaca gatagggctt gaatactgcc ttgctcatgc 420
gtaactgccg ggcgtttata tcggcggtta ttttctgctc gctttgcttc aatgcctcgt 480
tgatattttt ccgtaacgtc tctaagtctg ctttcgtttg ttgctctatg ctggcggctt 540
cggtgcgtga tgtctgctcg 560

<210> 35
<211> 560
<212> DNA
<213> Neisseria gonorrhorae

<400> 35
gttgatagcg gtagcaataa actaccctac tctttcgcta tccataccga taaaaataat 60
cataatcccc attgtcattt gatattttca gaacgccaac ttgacggcat agaccgtaca 120
gccgagcagt tttttaaacg tgctaatact aaatccccag aaaagggcgg agcgatgaaa 180
acggcagatt ttcgagatcg tgagtttatc caatctgtcc gaaaaacgtg gagagagcaa 240
gctaatcaag ccttagagca atacggatat gccgcacgaa ttgacgaacg tagctacaag 300
gaacaaggca tagagcaagc cccaagagca agaattgaca gggtaacgtg gcaagaattg 360
aaccgattag agcaagaaga acgccaaatc gtgcaagagc ttgcacttaa aggacaagaa 420
attaacaaag aaaaatccta cttgcagaaa atcgaagaaa aacaggctca aggaatgggc 480
aaatatgaat ccaaattcgc agctgcgttt tctaaattat cggaaagtgc cctaaaacac 540
gatttaagca acgaaaaaga 560

<210> 36
<211> 560
<212> DNA
<213> Neisseria gonorrhorae

<400> 36

gccgatttgg atattgattg gaaattccca aaaagaacac attccgaaga caggttgaat 60
tgggaaaagt atgtaacggg ggaatactgg gaaaaacaca acgaacccaa aagattcaat 120
aaagatattg ctgaaaagtt acaaaaaaaa tacggtatat tcgaaccaga aaaaaaacct 180
tggcaaacgg taagggatac cttgtccgac atcccgcatc ctttggggaa tcataaaatt 240
acaggacatg aatataggga tggcgcaaga atttatcccg gacacacagg aagcgggata 300
gacgaaccgt ccaaaaccat taaagcaggt gggcatggcg ttcccggcgg agaaaatatg 360
attcgttatg atgatggaac agtcagatac tttaccagct atgaagcaaa acttcttcaa 420
acattccctg aagaatttgt catttctgga gcttggggag aagcaatgcg acaaattggc 480
aatgcggttc ctgtcaaatt gtcggaaatt ttaggcaaac atctgatggg ggtgttgtcg 540
gagaaaagca gcctgcacaa 560

<210> 37
<211> 490
<212> DNA
<213> Neisseria gonorrhorae

<400> 37

tgtgattcag gtgaagggat gaaagatatc agaatactag atgcgtgctg tgggtctaga 60
atgttttggt tcgataaaaa ggaaccacat acgacataca tggatagacg tgaagaagaa 120
tttgaaattc acaaaaagaa aatcaatgtc aagccagata ttgttgcaga ttttcgagac 180
atgccatttg atgatgaaac atttaacctt gttgtatttg atccgccaca ccttctctgg 240
gcaggtcaga aatcattcat gcgtgcgcaa tatggtcaac tagacttgtt gacttggaga 300
ttagacttgc agcaaggttt tgaagaatgt ttcagagtat taaaaagagg tggaacactt 360
atttttaagt ggtcggatgc tcaagtaaat gttaaggaaa ttttggaatt agtcccacaa 420
aaaccacttt ttgggcaaca acgtgggaca actcactgga tggcttttat gaaattttag 480
gaggtattga 490

<210> 38

<211> 22

<212> DNA

<213> Artificial Sequence

<220>

<223> Designed chimeric oligonucleotide primer designated as NEI-5103 to amplify a portion of pJDB4 plasmid DNA from Neisseria gonorrhoeae. "Nucleotides 20 to 22 are ribonucleotides-other nucleotides are deoxyribonucleotides."

<400> 38

taatcaagcc ttagagcaau ac 22

<210> 39

<211> 22

<212> DNA

<213> Artificial Sequence

<220>

<223> Designed chimeric oligonucleotide primer designated as NEI-5150 to amplify a portion of pJDB4 plasmid DNA from Neisseria gonorrhoeae. "Nucleotides 20 to 22 are ribonucleotides-other nucleotides are deoxyribonucleotides."

<400> 39

atgccttgtt ccttgtagcu ac 22

<210> 40

<211> 19

<212> DNA

<213> Artificial Sequence

<220>

<223> Designed chimeric oligonucleotide primer designated as NEI-CppB-F1 to amplify a portion of CppB gene from Neisseria gonorrhoeae. "Nucleotides 17 to 19 are ribonucleotides-other nucleotides are deoxyribonucleotides."

<400> 40

gctctaactc ggctgccaa 19

<210> 41

<211> 20

<212> DNA

<213> Artificial Sequence

<220>

<223> Designed chimeric oligonucleotide primer designated as NEI-CppB-F2 to amplify a portion of CppB gene from Neisseria gonorrhoeae. "Nucleotides 18 to 20 are ribonucleotides-other nucleotides are deoxyribonucleotides."

<400> 41

gctctaactc ggctgccaag 20

<210> 42

<211> 18

<212> DNA

<213> Artificial Sequence

<220>

<223> Designed chimeric oligonucleotide primer designated as NEI-CppB-F3 to amplify a portion of CppB gene from Neisseria gonorrhoeae. "Nucleotides 16 to 18 are ribonucleotides-other nucleotides are deoxyribonucleotides."

<400> 42

gctctaactc ggctgcca 18

<210> 43

<211> 20

<212> DNA

<213> Artificial Sequence

<220>

<223> Designed chimeric oligonucleotide primer designated as NEI-CppB-R1 to amplify a portion of CppB gene from Neisseria gonorrhoeae. "Nucleotides 18 to 20 are ribonucleotides-other nucleotides are deoxyribonucleotides."

<400> 43

tagagcagga aaacacgagu 20

<210> 44

<211> 19

<212> DNA

<213> Artificial Sequence

<220>

<223> Designed chimeric oligonucleotide primer designated as NEI-CppB-R2 to amplify a portion of CppB gene from Neisseria gonorrhoeae. "Nucleotides 17 to 19 are ribonucleotides-other nucleotides are deoxyribonucleotides."

<400> 44
tagagcagga aaacacgag 19

<210> 45
<211> 21
<212> DNA
<213> Artificial Sequence

<220>

<223> Designed chimeric oligonucleotide primer designated as NEI-CppB-R3 to amplify a portion of CppB gene from Neisseria gonorrhoeae. "Nucleotides 19 to 21 are ribonucleotides-other nucleotides are deoxyribonucleotides."

<400> 45
tagagcagga aaacacgagu u 21

<210> 46
<211> 20
<212> DNA
<213> Artificial Sequence

<220>

<223> Designed chimeric oligonucleotide primer designated as pJDB F-1 to amplify a portion of CppB gene from Neisseria gonorrhoeae. "Nucleotides 18 to 20 are ribonucleotides-other nucleotides are deoxyribonucleotides."

<400> 46
ctttgcttca atgcctcguu 20

<210> 47
<211> 21
<212> DNA
<213> Artificial Sequence

<220>

<223> Designed chimeric oligonucleotide primer designated as pJDB F-2 to amplify a portion of CppB gene from Neisseria gonorrhoeae. "Nucleotides 19 to 21 are ribonucleotides-other nucleotides are deoxyribonucleotides."

<400> 47
ctttgcttca atgcctcguu g 21

<210> 48
<211> 15
<212> DNA
<213> Artificial Sequence

<220>

<223> Designed chimeric oligonucleotide primer designated as pJDB R-1 to amplify a portion of CppB gene from Neisseria gonorrhoeae. "Nucleotides 13 to 15 are ribonucleotides-other nucleotides are deoxyribonucleotides."

<400> 48
catcacgcac cgaag 15

<210> 49
<211> 16
<212> DNA
<213> Artificial Sequence

<220>
<223> Designed chimeric oligonucleotide primer designated as pJDB R-2 to amplify a portion of CppB gene from Neisseria gonorrhoeae. "Nucleotides 14 to 16 are ribonucleotides-other nucleotides are deoxyribonucleotides."

<400> 49
catcacgcac cgaagc 16

<210> 50
<211> 17
<212> DNA
<213> Artificial Sequence

<220>
<223> Designed chimeric oligonucleotide primer designated as pJDB R-3 to amplify a portion of CppB gene from Neisseria gonorrhoeae. "Nucleotides 15 to 17 are ribonucleotides-other nucleotides are deoxyribonucleotides."

<400> 50
catcacgcac cgaagcc 17

<210> 51
<211> 21
<212> DNA
<213> Artificial Sequence

<220>
<223> Designed chimeric oligonucleotide primer designated as M.Ngo F-1 to amplify a portion of M.NgoMIII gene from Neisseria gonorrhoeae. "Nucleotides 19 to 21 are ribonucleotides-other nucleotides are deoxyribonucleotides."

<400> 51
acacacagga agcgggatag a 21

<210> 52
<211> 20
<212> DNA
<213> Artificial Sequence

<220>
<223> Designed chimeric oligonucleotide primer designated as M.Ngo F-2 to amplify a portion of M.NgoMIII gene from Neisseria gonorrhoeae. "Nucleotides 18 to 20 are ribonucleotides-other nucleotides are deoxyribonucleotides."

<400> 52
acacacagga agcgggauag 20

<210> 53
<211> 19
<212> DNA
<213> Artificial Sequence

<220>
<223> Designed chimeric oligonucleotide primer designated as M.Ngo F-3 to amplify a portion of M.NgoMIII gene from Neisseria gonorrhoeae. "Nucleotides 17 to 19 are ribonucleotides-other nucleotides are deoxyribonucleotides."

<400> 53
acacacagga agcgggaua 19

<210> 54
<211> 20
<212> DNA
<213> Artificial Sequence

<220>
<223> Designed chimeric oligonucleotide primer designated as M.Ngo R-1 to amplify a portion of M.NgoMIII gene from Neisseria gonorrhoeae. "Nucleotides 18 to 20 are ribonucleotides-other nucleotides

are deoxyribonucleotides."

<400> 54
acgccatgcc cacctgcuuu 20

<210> 55
<211> 19
<212> DNA
<213> Artificial Sequence

<220>
<223> Designed chimeric oligonucleotide primer designated as M.Ngo R-2 to amplify a portion of M.NgoMIII gene from Neisseria gonorrhoeae. "Nucleotides 17 to 19 are ribonucleotides-other nucleotides are deoxyribonucleotides."

<400> 55
acgccatgcc cacctgcuu 19

<210> 56
<211> 18
<212> DNA
<213> Artificial Sequence

<220>
<223> Designed chimeric oligonucleotide primer designated as M.Ngo R-3 to amplify a portion of M.NgoMIII gene from Neisseria gonorrhoeae. "Nucleotides 16 to 18 are ribonucleotides-other nucleotides are deoxyribonucleotides."

<400> 56

acgccatgcc cacctgcu 18

<210> 57

<211> 17

<212> DNA

<213> Artificial Sequence

<220>

<223> Designed chimeric oligonucleotide primer designated as M.Ngo R-4 to amplify a portion of M.NgoMIII gene from Neisseria gonorrhoeae. "Nucleotides 19 to 21 are ribonucleotides-other nucleotides are deoxyribonucleotides."

<400> 57

acgccatgcc caccugc 17

<210> 58

<211> 20

<212> DNA

<213> Artificial Sequence

<220>

<223> Designed chimeric oligonucleotide primer designated as Cytosine F-1 to amplify a portion of methyltransferase gene from Neisseria gonorrhoeae. "Nucleotides 18 to 20 are ribonucleotides-other nucleotides are deoxyribonucleotides."

<400> 58

tatttgatcc gccacaccuu 20

<210> 59

<211> 21

<212> DNA

<213> Artificial Sequence

<220>

<223> Designed chimeric oligonucleotide primer designated as Cytosine F-2 to amplify a portion of methyltransferase gene from Neisseria gonorrhoeae. "Nucleotides 19 to 21 are ribonucleotides-other nucleotides are deoxyribonucleotides."

<400> 59

tatttgatcc gccacaccuu c 21

<210> 60

<211> 19

<212> DNA

<213> Artificial Sequence

<220>

<223> Designed chimeric oligonucleotide primer designated as Cytosine F-3 to amplify a portion of methyltransferase gene from Neisseria gonorrhoeae. "Nucleotides 17 to 19 are ribonucleotides-other nucleotides are deoxyribonucleotides."

<400> 60

tatttgatcc gccacaccu 19

<210> 61
<211> 20
<212> DNA
<213> Artificial Sequence

<220>
<223> Designed chimeric oligonucleotide primer designated as Cytosine R-1 to amplify a portion of methyltransferase gene from Neisseria gonorrhoeae. "Nucleotides 18 to 20 are ribonucleotides-other nucleotides are deoxyribonucleotides."

<400> 61
tctagttgac catattgcgc 20

<210> 62
<211> 21
<212> DNA
<213> Artificial Sequence

<220>
<223> Designed chimeric oligonucleotide primer designated as Cytosine R-2 to amplify a portion of methyltransferase gene from Neisseria gonorrhoeae. "Nucleotides 19 to 21 are ribonucleotides-other nucleotides are deoxyribonucleotides."

<400> 62
tctagttgac catattgcgc a 21

<210> 63
<211> 22
<212> DNA
<213> Artificial Sequence

<220>
<223> Designed chimeric oligonucleotide primer designated as Cytosine R-3 to amplify a portion of methyltransferase gene from Neisseria gonorrhoeae. "Nucleotides 20 to 22 are ribonucleotides-other nucleotides are deoxyribonucleotides."

<400> 63
tctagttgac catattgcgc ac 22

<210> 64
<211> 20
<212> DNA
<213> Artificial Sequence

<220>
<223> Designed oligonucleotide probe as NEI-5130 probe to detect a DNA fragment amplifing a portion of pJDB4 plasmid DNA from Neisseria gonorrhoeae.

<400> 64
gatatgccgc acgaattgac 20

<210> 65

<211> 20

<212> DNA

<213> Artificial Sequence

<220>

<223> Designed oligonucleotide probe as NEI-CppB probe-1 to detect a DNA fragment amplifing a portion of CppB-encoding sequence from Neisseria gonorrhoeae.

<400> 65

ctcgctagct gctgcgctaa 20

<210> 66

<211> 20

<212> DNA

<213> Artificial Sequence

<220>

<223> Designed oligonucleotide probe as NEI-CppB probe-2 to detect a DNA fragment amplifing a portion of CppB-encoding sequence from Neisseria gonorrhoeae.

<400> 66

tccgtaacgt ctctaagtct 20

<210> 67

<211> 19

<212> DNA

<213> Artificial Sequence

<220>

<223> Designed oligonucleotide probe as M.Ngo-probe to detect a DNA fragment amplifing a portion of M.NgoMIII gene-encoding sequence from Neisseria gonorrhoeae.

<400> 67

cgaaccgtcc aaaaccatt 19

<210> 68

<211> 20

<212> DNA

<213> Artificial Sequence

<220>

<223> Designed oligonucleotide probe as Cytosine-probe to detect a DNA fragment amplifing a portion of methyltransferase-encoding sequence from Neisseria gonorrhoeae.

<400> 68

ctctgggcag gtcagaaatc 20

<210> 69

<211> 560

<212> DNA

<213> Hepatitis B virus

<400> 69

ccttcccatg gctgctcggg tgtgctgcca actggatcct gcgcgggacg tcctttgtct 60

acgtcccgtc ggcgctgaat cccgcggacg acccgtctcg gggccgtttg ggcctctacc 120
gtcccttgct ttctctgccg ttccagccga ccacggggcg cacctctctt tacgcggtct 180
ccccgtctgt gccttctcat ctgccggacc gtgtgcactt cgcttcacct ctgcacgtcg 240
catggagacc accgtgaacg gccaccaggt cttgcccaag ctcttacata agaggactct 300
tggactctca gcaatgtcaa caaccgacct tgaggcatac ttcaaagact gtttgtttaa 360
agactgggag gagttggggg aggagattag gttaaaggtc tttgtactag gaggctgtag 420
gcataaattg gtctgttcac cagcaccatg caactttttc acctctgcct aatcatctca 480
tgttcatgtc ctactgttca agcctccaag ctgtgccttg ggtggctttg gggcatggac 540
attgacccgt ataaagaatt 560

<210> 70
<211> 560
<212> DNA
<213> Hepatitis B virus

<400> 70
ccttcccatg gctgctcggg tgtgctgcca actggatcct gcgcgggacg tcctttgtct 60
acgtcccgtc ggcgctgaat cccgcggacg acccgtctcg gggccgtttg ggcctctacc 120
gtcccttgct ttctctgccg ttccagccga ccacggggcg cacctctctt tacgcggtct 180
ccccgtctgt gccttctcat ctgccggacc gtgtgcactt cgcttcacct ctgcacgtcg 240
catggagacc accgtgaacg gccaccaggt cttgcccaag ctcttacata agaggactct 300
tggactctca gcaatgtcaa caaccgacct tgaggcatac ttcaaagact gtgtgtttaa 360
agactgggag gagttggggg aggagattag gttaaaggtc tttgtactag gaggctgtag 420
gcataaattg gtctgttcac cagcaccatg caactttttc acctctgcct aatcatctca 480
tgttcatgtc ctactgttca agcctccaag ctgtgccttg ggtggctttg gggcatggac 540
attgacccgt ataaagaatt 560

<210> 71
<211> 24

<212> DNA

<213> Artificial Sequence

<220>

<223> Designed chimeric oligonucleotide primer designated as HBV-F-1 to amplify a portion of X-protein gene from Hepatitis B virus. "Nucleotides 22 to 24 are ribonucleotides-other nucleotides are deoxyribonucleotides."

<400> 71

gaggactctt ggactctcag caau 24

<210> 72

<211> 20

<212> DNA

<213> Artificial Sequence

<220>

<223> Designed chimeric oligonucleotide primer designated as HBV-F-2 to amplify a portion of X-protein gene from Hepatitis B virus. "Nucleotides 18 to 20 are ribonucleotides-other nucleotides are deoxyribonucleotides."

<400> 72

ctcttggact ctcagcaaug 20

<210> 73

<211> 21

<212> DNA

<213> Artificial Sequence

<220>

<223> Designed chimeric oligonucleotide primer designated as HBV-F-3 to amplify a portion of X-protein gene from Hepatitis B virus. "Nucleotides 19 to 21 are ribonucleotides-other nucleotides are deoxyribonucleotides."

<400> 73

gaggcatact tcaaagacug u 21

<210> 74

<211> 24

<212> DNA

<213> Artificial Sequence

<220>

<223> Designed chimeric oligonucleotide primer designated as HBV-F-5 to amplify a portion of X-protein gene from Hepatitis B virus. "Nucleotides 22 to 24 are ribonucleotides-other nucleotides are deoxyribonucleotides."

<400> 74

tacttcaaag actgtgtgtt taaa 24

<210> 75

<211> 22

<212> DNA

<213> Artificial Sequence

<220>

<223> Designed chimeric oligonucleotide primer designated as HBV-R-1 to amplify a portion of X-protein gene from Hepatitis B virus. "Nucleotides 20 to 22 are ribonucleotides-other nucleotides are deoxyribonucleotides."

<400> 75

tcctcccagt ctttaaacam ac 22

<210> 76

<211> 23

<212> DNA

<213> Artificial Sequence

<220>

<223> Designed chimeric oligonucleotide primer designated as HBV-R-2 to amplify a portion of X-protein gene from Hepatitis B virus. "Nucleotides 21 to 23 are ribonucleotides-other nucleotides are deoxyribonucleotides."

<400> 76

ccagtcttta aacamacagt cuu 23

<210> 77

<211> 21

<212> DNA

<213> Artificial Sequence

<220>

<223> Designed chimeric oligonucleotide primer designated as HBV-R-3 to amplify a portion of X-protein gene from Hepatitis B virus. "Nucleotides 19 to 21 are ribonucleotides-other nucleotides are deoxyribonucleotides."

<400> 77

gcctcctagt acaaagaccu u 21

<210> 78

<211> 24

<212> DNA

<213> Artificial Sequence

<220>

<223> Designed chimeric oligonucleotide primer designated as HBV-R-4 to amplify a portion of X-protein gene from Hepatitis B virus. "Nucleotides 22 to 24 are ribonucleotides-other nucleotides are deoxyribonucleotides."

<400> 78

ctagtacaaa gacctttaac cuaa 24

<210> 79

<211> 20

<212> DNA

<213> Artificial Sequence

<220>

<223> Designed oligonucleotide probe as HBV-probe1 to detect a DNA fragment amplifing a portion of X-protein-encoding sequence from Hepatitis B virus.

<400> 79
caaccgacct tgaggcatac 20

<210> 80
<211> 20
<212> DNA
<213> Artificial Sequence

<220>
<223> Designed oligonucleotide probe as HBV-probe2 to detect a DNA fragment amplifing a portion of X-protein-encoding sequence from Hepatitis B virus.

<400> 80
gactgggagg agttggggga 20

<210> 81
<211> 18
<212> DNA
<213> Artificial Sequence

<220>
<223> Designed chimeric oligonucleotide primer designated as HCV-A2-S to amplify a portion of Hepatitis C virus. "Nucleotides 16 to 18 are ribonucleotides-other nucleotides are deoxyribonucleotides."

<400> 81
ctttcttgga tcaacccg 18

<210> 82
<211> 20
<212> DNA
<213> Artificial Sequence

<220>
<223> Designed chimeric oligonucleotide primer designated as HCV-A2-A to amplify a portion of Hepatitis C virus. "Nucleotides 18 to 20 are ribonucleotides-other nucleotides are deoxyribonucleotides."

<400> 82
aacactactc ggctagcagu 20

<210> 83
<211> 17
<212> DNA
<213> Artificial Sequence

<220>
<223> Designed chimeric oligonucleotide primer designated as HCV-A4-S to amplify a portion of Hepatitis C virus. "Nucleotides 15 to 17 are ribonucleotides-other nucleotides are deoxyribonucleotides."

<400> 83
ctttcttgga tcaaccc 17

<210> 84

<211> 18

<212> DNA

<213> Artificial Sequence

<220>

<223> Designed chimeric oligonucleotide primer designated as HCV-A4-A to amplify a portion of Hepatitis C virus. "Nucleotides 16 to 18 are ribonucleotides-other nucleotides are deoxyribonucleotides."

<400> 84

ccaacactac tcggcuag 18

<210> 85

<211> 19

<212> DNA

<213> Artificial Sequence

<220>

<223> Designed chimeric oligonucleotide primer designated as HCV-A4-S19 to amplify a portion of Hepatitis C virus. "Nucleotides 17 to 19 are ribonucleotides-other nucleotides are deoxyribonucleotides."

<400> 85

tcctttcttg gatcaaccc 19

<210> 86

<211> 19

<212> DNA

<213> Artificial Sequence

<220>

<223> Designed chimeric oligonucleotide primer designated as HCV-A4-A19 to amplify a portion of Hepatitis C virus. "Nucleotides 17 to 19 are ribonucleotides-other nucleotides are deoxyribonucleotides."

<400> 86

cccaacacta ctcggcuag 19

<210> 87

<211> 20

<212> DNA

<213> Artificial Sequence

<220>

<223> Designed oligonucleotide probe as HCV-C probe to detect a DNA fragment amplifing a Hepatitis C virus.

<400> 87

ctcaatgcct ggagatttgg 20

<210> 88

<211> 19

<212> DNA

<213> Artificial Sequence

<220>

<223> Designed chimeric oligonucleotide primer designated as HCV-F1 to amplify a portion of Hepatitis C virus. "Nucleotides 17 to 19 are ribonucleotides-other nucleotides are deoxyribonucleotides."

<400> 88
ctagccgagt agtgttggg 19

<210> 89
<211> 18
<212> DNA
<213> Artificial Sequence

<220>
<223> Designed chimeric oligonucleotide primer designated as HCV-R1 to amplify a portion of Hepatitis C virus. "Nucleotides 16 to 18 are ribonucleotides-other nucleotides are deoxyribonucleotides."

<400> 89
tcgcaagcac cctatcag 18

<210> 90
<211> 18
<212> DNA
<213> Artificial Sequence

<220>
<223> Designed chimeric oligonucleotide primer designated as HCV-F2 to amplify a portion of Hepatitis C virus. "Nucleotides 16 to 18 are ribonucleotides-other nucleotides are deoxyribonucleotides."

<400> 90
ctagccgagt agtgtugg 18

<210> 91
<211> 19
<212> DNA
<213> Artificial Sequence

<220>
<223> Designed chimeric oligonucleotide primer designated as HCV-R2 to amplify a portion of Hepatitis C virus. "Nucleotides 17 to 19 are ribonucleotides-other nucleotides are deoxyribonucleotides."

<400> 91
tcgcaagcac cctatcagg 19

<210> 92
<211> 20
<212> DNA
<213> Artificial Sequence

<220>
<223> Designed oligonucleotide probe as HCV-D probe to detect a DNA fragment amplifing a Hepatitis C virus.

<400> 92
gcgaaaggcc ttgtggtact 20

<210> 93

<211> 44

<212> DNA

<213> Artificial Sequence

<220>

<223> Designed oligonucleotide primer designated as SP6-HIV-F to amplify a portion of gag sequence from HIV.

<400> 93

ccatttaggt gacactatag aataccagag gagctctctc gacg 44

<210> 94

<211> 20

<212> DNA

<213> Artificial Sequence

<220>

<223> Designed oligonucleotide primer designated as HIV-R to amplify a portion of gag sequence from HIV.

<400> 94

ccaacagccc tttttcctag 20

<210> 95

<211> 20

<212> DNA

<213> Artificial Sequence

<220>

<223> Designed chimeric oligonucleotide primer designated as HIV-F1 to amplify a portion of gag sequence from HIV. "Nucleotides 18 to 20 are ribonucleotides-other nucleotides are deoxyribonucleotides."

<400> 95

atgggtgcga gagcgucart 20

<210> 96

<211> 22

<212> DNA

<213> Artificial Sequence

<220>

<223> Designed chimeric oligonucleotide primer designated as HIV-F2 to amplify a portion of gag sequence from HIV. "Nucleotides 20 to 22 are ribonucleotides-other nucleotides are deoxyribonucleotides."

<400> 96

tagaagaaat gatgacagca ug 22

<210> 97

<211> 22

<212> DNA

<213> Artificial Sequence

<220>

<223> Designed chimeric oligonucleotide primer designated as HIV-F3 to amplify a portion of gag from sequence HIV. "Nucleotides 20 to 22 are ribonucleotides-other nucleotides are deoxyribonucleotides."

<400> 97
gaagaaatga tgacagcatg uc 22

<210> 98
<211> 22
<212> DNA
<213> Artificial Sequence

<220>
<223> Designed chimeric oligonucleotide primer designated as HIV-F4 to amplify a portion of gag from sequence HIV. "Nucleotides 20 to 22 are ribonucleotides-other nucleotides are deoxyribonucleotides."

<400> 98
aytagaagaa atgatgacag ca 22

<210> 99
<211> 20
<212> DNA
<213> Artificial Sequence

<220>
<223> Designed chimeric oligonucleotide primer designated as HIV-R1 to amplify a portion of gag sequence from HIV. "Nucleotides 18 to 20 are ribonucleotides-other nucleotides are deoxyribonucleotides."

<400> 99

ctccctgctt gcccatacua 20

<210> 100
<211> 22
<212> DNA
<213> Artificial Sequence

<220>
<223> Designed chimeric oligonucleotide primer designated as HIV-R2 to amplify a portion of gag from sequence HIV. "Nucleotides 20 to 22 are ribonucleotides-other nucleotides are deoxyribonucleotides."

<400> 100
cattgcctca gccaaaactc uu 22

<210> 101
<211> 22
<212> DNA
<213> Artificial Sequence

<220>
<223> Designed chimeric oligonucleotide primer designated as HIV-R3 to amplify a portion of gag sequence from HIV. "Nucleotides 20 to 22 are ribonucleotides-other nucleotides are deoxyribonucleotides."

<400> 101
ctcattgcct cagccaaaac uc 22

<210> 102

<211> 22

<212> DNA

<213> Artificial Sequence

<220>

<223> Designed chimeric oligonucleotide primer designated as HIV-R4 to amplify a portion of gag sequence from HIV. "Nucleotides 20 to 22 are ribonucleotides-other nucleotides are deoxyribonucleotides."

<400> 102

ttgcttcagc caaaactctu gc 22

<210> 103

<211> 22

<212> DNA

<213> Artificial Sequence

<220>

<223> Designed chimeric oligonucleotide primer designated as HIV-R4M to amplify a portion of gag sequence from HIV. "Nucleotides 20 to 22 are ribonucleotides-other nucleotides are deoxyribonucleotides."

<400> 103

ttgcytcagc caaaacyctu gc 22

<210> 104

<211> 21

<212> DNA

<213> Artificial Sequence

<220>

<223> Designed oligonucleotide probe as HIV-A probe to detect a DNA fragment amplifing a portion of gag sequence from HIV.

<400> 104

gggaaaatta gatgcatggg a 21

<210> 105

<211> 20

<212> DNA

<213> Artificial Sequence

<220>

<223> Designed oligonucleotide probe as HIV-B probe to detect a DNA fragment amplifing a portion of gag sequence from HIV.

<400> 105

ggagtgggag gaccyrgcca 20

<210> 106

<211> 22

<212> DNA

<213> Artificial Sequence

<220>

<223> Designed oligonucleotide primer designated as coa-PCR-F to amplify a portion of coagulase gene from Staphylococcus aureus.

<400> 106

ctattttaga aggtcttgaa gg 22

<210> 107

<211> 20

<212> DNA

<213> Artificial Sequence

<220>

<223> Designed oligonucleotide primer designated as coa-PCR-R to amplify a portion of coagulase gene from Staphylococcus aureus.

<400> 107

gtattcacgg atacctgtac 20

<210> 108

<211> 18

<212> DNA

<213> Artificial Sequence

<220>

<223> Designed chimeric oligonucleotide primer designated as coa-F1 to amplify a portion of coagulase gene from Staphylococcus aureus. "Nucleotides 16 to 18 are ribonucleotides-other nucleotides are deoxyribonucleotides."

<400> 108

caagcaactg aaacaaca 18

<210> 109

<211> 19

<212> DNA

<213> Artificial Sequence

<220>

<223> Designed chimeric oligonucleotide primer designated as coa-F2 to amplify a portion of coagulase gene from Staphylococcus aureus. "Nucleotides 17 to 19 are ribonucleotides-other nucleotides are deoxyribonucleotides."

<400> 109

tattgaagtt aaacctcaa 19

<210> 110

<211> 20

<212> DNA

<213> Artificial Sequence

<220>

<223> Designed chimeric oligonucleotide primer designated as coa-F3 to amplify a portion of coagulase gene from Staphylococcus aureus. "Nucleotides 18 to 20 are ribonucleotides-other nucleotides are deoxyribonucleotides."

<400> 110

aacttgaaat aaaaccacaa 20

<210> 111

<211> 20

<212> DNA

<213> Artificial Sequence

<220>

<223> Designed chimeric oligonucleotide primer designated as coa-F4 to amplify a portion of coagulase gene from Staphylococcus aureus. "Nucleotides 18 to 20 are ribonucleotides-other nucleotides are deoxyribonucleotides."

<400> 111

aaataaaacc acaaggtacu 20

<210> 112

<211> 18

<212> DNA

<213> Artificial Sequence

<220>

<223> Designed chimeric oligonucleotide primer designated as coa-F5 to amplify a portion of coagulase gene from Staphylococcus aureus. "Nucleotides 16 to 18 are ribonucleotides-other nucleotides are deoxyribonucleotides."

<400> 112

gtagctcatc taaacuug 18

<210> 113

<211> 18

<212> DNA

<213> Artificial Sequence

<220>

<223> Designed chimeric oligonucleotide primer designated as coa-R1 to amplify a portion of coagulase gene from Staphylococcus aureus. "Nucleotides 16 to 18 are ribonucleotides-other nucleotides are deoxyribonucleotides."

<400> 113

gttttgttaa attgcggu 18

<210> 114

<211> 18

<212> DNA

<213> Artificial Sequence

<220>

<223> Designed chimeric oligonucleotide primer designated as coa-R2 to amplify a portion of coagulase gene from Staphylococcus aureus. "Nucleotides 16 to 18 are ribonucleotides-other nucleotides are deoxyribonucleotides."

<400> 114

atacttaggt gttttguu 18

<210> 115

<211> 20

<212> DNA

<213> Artificial Sequence

<220>

<223> Designed chimeric oligonucleotide primer designated as coa-R3 to amplify a portion of coagulase gene from Staphylococcus aureus. "Nucleotides 18 to 20 are ribonucleotides-other nucleotides are deoxyribonucleotides."

<400> 115

tgtttcagtt gcttgagguu 20

<210> 116

<211> 20

<212> DNA

<213> Artificial Sequence

<220>

<223> Designed chimeric oligonucleotide primer designated as coa-R4 to amplify a portion of coagulase gene from Staphylococcus aureus. "Nucleotides 18 to 20 are ribonucleotides-other nucleotides are deoxyribonucleotides."

<400> 116

ttctgttgtt tcagttgcuu 20

<210> 117

<211> 18

<212> DNA

<213> Artificial Sequence

<220>

<223> Designed chimeric oligonucleotide primer designated as coa-R5 to amplify a portion of coagulase gene from Staphylococcus aureus. "Nucleotides 16 to 18 are ribonucleotides-other nucleotides are deoxyribonucleotides."

<400> 117

tgttgtttca gttgcuug 18

<210> 118

<211> 20

<212> DNA

<213> Artificial Sequence

<220>

<223> Designed oligonucleotide probe as coa-A probe to detect a DNA fragment amplifing a coagulase gene from Staphylococcus aureus.

<400> 118

tcaaggagaa tcaagtgata 20

<210> 119

<211> 19

<212> DNA

<213> Artificial Sequence

<220>

<223> Designed oligonucleotide probe as coa-B probe to detect a DNA fragment amplifing a coagulase gene from Staphylococcus aureus.

<400> 119
agcttctcaa tatggtccg 19

<210> 120
<211> 560
<212> DNA
<213> Chlamydia trachomatis

<400> 120
atgcgttgtt aggtaaagct ctgatatttg aagactctac tgagtatatt ctgaggcagc 60
ttgctaatta tgagtttaag tgttctcatc ataaaaacat attcatagta tttaaatact 120
taaaagacaa tggattacct ataactgtag actcggcttg ggaagagctt ttgcggcgtc 180
gtatcaaaga tatggacaaa tcgtatctcg ggttaatgtt gcatgatgct ttatcaaatg 240
acaagcttag atccgtttct catacggttt tcctcgatga tttgagcgtg tgtagcgctg 300
aagaaaattt gagtaatttc attttccgct cgtttaatga gtacaatgaa aatccattgc 360
gtagatctcc gtttctattg cttgagcgta taaagggaag gcttgacagt gctatagcaa 420
agactttttc tattcgcagc gctagaggcc ggtctattta tgatatattc tcacagtcag 480
aaattggagt gctggctcgt ataaaaaaaa gacgagcaac gttctctgag aatcaaaatt 540
ctttctttga tgccttccca 560

<210> 121
<211> 19
<212> DNA
<213> Artificial Sequence

<220>
<223> Designed chimeric oligonucleotide primer designated as CT-FB19 to amplify a portion of plasmid DNA from Chlamydia trachomatis . "Nucleotides 17 to 19 are ribonucleotides-other nucleotides are deoxyribonucleotides."

<400> 121
tccgtttctc atacgguuu 19

<210> 122
<211> 22
<212> DNA
<213> Artificial Sequence

<220>
<223> Designed chimeric oligonucleotide primer designated as CT-FB19-3 to amplify a portion of plasmid DNA from Chlamydia trachomatis . "Nucleotides 20 to 22 are ribonucleotides-other nucleotides are deoxyribonucleotides."

<400> 122
catacggttt tcctcgatga uu 22

<210> 123
<211> 21
<212> DNA
<213> Artificial Sequence

<220>
<223> Designed chimeric oligonucleotide primer designated as CT-FB19-3-21 to amplify a portion of plasmid DNA from Chlamydia trachomatis . "Nucleotides 19 to 21 are ribonucleotides-other nucleotides are deoxyribonucleotides."

<400> 123

catacggttt tcctcgatga u 21

<210> 124

<211> 23

<212> DNA

<213> Artificial Sequence

<220>

<223> Designed chimeric oligonucleotide primer designated as CT-FB19-3-23 to amplify a portion of plasmid DNA from Chlamydia trachomatis . "Nucleotides 21 to 23 are ribonucleotides-other nucleotides are deoxyribonucleotides."

<400> 124

catacggttt tcctcgatga uuu 23

<210> 125

<211> 21

<212> DNA

<213> Artificial Sequence

<220>

<223> Designed chimeric oligonucleotide primer designated as CT-RB21 to amplify a portion of plasmid DNA from Chlamydia trachomatis . "Nucleotides 19 to 21 are ribonucleotides-other

nucleotides are deoxyribonucleotides."

<400> 125

agaaacggag atctacgcaa u 21

<210> 126

<211> 23

<212> DNA

<213> Artificial Sequence

<220>

<223> Designed chimeric oligonucleotide primer designated as CT-RB23-2 to amplify a portion of plasmid DNA from Chlamydia trachomatis . "Nucleotides 21 to 23 are ribonucleotides-other nucleotides are deoxyribonucleotides."

<400> 126

gatctacgca atggattttc auu 23

<210> 127

<211> 24

<212> DNA

<213> Artificial Sequence

<220>

<223> Designed chimeric oligonucleotide primer designated as CT-RB23-2-24 to amplify a portion of plasmid DNA from Chlamydia trachomatis . "Nucleotides 22 to 24 are ribonucleotides-other nucleotides are deoxyribonucleotides."

<400> 127

gatctacgca atggattttc auug 24

<210> 128

<211> 20

<212> DNA

<213> Artificial Sequence

<220>

<223> Designed oligonucleotide probe as CT-probe to detect a DNA fragment amplifing a portion of plasmid DNA from Chlamydia trachomatis.

<400> 128

tgagcgtgtg tagcgctgaa 20

<210> 129

<211> 560

<212> DNA

<213> Chlamydia trachomatis

<400> 129

gtcctctagt acaaacaccc ccaatattgt gatataatta aaattatatt catattctgt 60

tgccagaaaa aacactttta ggctatatta gagccaatct tctttgaagc gttgtcttct 120

cgagaagatt tatcgtacgc aaatatcatc tttgcggttg cgtgtcctgt gaccttcatt 180

atgtcggagt ctgagcaccc taggcgtttg tactccgtca cagcggttgc tcgaagcacg 240

tgcggggtta tcttaaaagg gattgcagct tgtagtcctg cttgagagaa cgtgcgggcg 300

atttgcctta accccaccat ttttccggag cgagttacga agacaaaacc tcttcgttga 360

ccgatgtact cttgtagaaa gtgcataaac ttctgaggat aagttataat aatcctcttt 420

tctgtctgac ggttcttaag ctgggagaaa gaaatggtag cttgttggaa acaaatctga 480

ctaatctcca agcttaagac ttcagaggag cgtttacctc cttggagcat tgtctgggcg 540

atcaaccaat cccgggcatt 560

<210> 130

<211> 20

<212> DNA

<213> Artificial Sequence

<220>

<223> Designed chimeric oligonucleotide primer designated as CT-F1212-20 to amplify a portion of plasmid DNA from Chlamydia trachomatis . "Nucleotides 18 to 20 are ribonucleotides-other nucleotides are deoxyribonucleotides."

<400> 130

gtgtcctgtg accttcauua 20

<210> 131

<211> 21

<212> DNA

<213> Artificial Sequence

<220>

<223> Designed chimeric oligonucleotide primer designated as CT-F1212-21 to amplify a portion of plasmid DNA from Chlamydia trachomatis . "Nucleotides 19 to 21 are ribonucleotides-other nucleotides are deoxyribonucleotides."

<400> 131

gtgtcctgtg accttcatua u 21

<210> 132

<211> 20

<212> DNA

<213> Artificial Sequence

<220>

<223> Designed chimeric oligonucleotide primer designated as CT-F1212-22 to amplify a portion of plasmid DNA from Chlamydia trachomatis . "Nucleotides 20 to 22 are ribonucleotides-other nucleotides are deoxyribonucleotides."

<400> 132

gtgtcctgtg accttcatta ug 22

<210> 133

<211> 20

<212> DNA

<213> Artificial Sequence

<220>

<223> Designed chimeric oligonucleotide primer designated as CT-R1272-20 to amplify a portion of plasmid DNA from Chlamydia trachomatis . "Nucleotides 18 to 20 are ribonucleotides-other nucleotides are deoxyribonucleotides."

<400> 133

tgtgacggag tacaaacgcc 20

<210> 134

<211> 21

<212> DNA

<213> Artificial Sequence

<220>

<223> Designed chimeric oligonucleotide primer designated as CT-R1272-21 to amplify a portion of plasmid DNA from Chlamydia trachomatis . "Nucleotides 19 to 21 are ribonucleotides-other nucleotides are deoxyribonucleotides."

<400> 134

tgtgacggag tacaaacgcc u 21

<210> 135

<211> 22

<212> DNA

<213> Artificial Sequence

<220>

<223> Designed chimeric oligonucleotide primer designated as CT-R1272-22 to amplify a portion of plasmid DNA from Chlamydia trachomatis . "Nucleotides 20 to 22 are ribonucleotides-other nucleotides are deoxyribonucleotides."

<400> 135

tgtgacggag tacaaacgcc ua 22

<210> 136

<211> 19

<212> DNA

<213> Artificial Sequence

<220>

<223> Designed oligonucleotide probe as CT-1234 probe to detect a DNA fragment amplifing a portion of plasmid DNA from Chlamydia trachomatis.

<400> 136

tcggagtctg agcacccta 19

<210> 137

<211> 560

<212> DNA

<213> Mycoplasma pneumoniae

<400> 137

aacagtagct aacactaatc acaaaaatga aagtgataag tttgtgattt ttgaacctca 60
accaccattg agccaaacta tccccaaacc agaggctgaa ccagttatag agcccgatgc 120
tgttgctaca ccacctgtgc agaatgccga ggtgcaaatt aagcctgaca gctccaaggg 180
tgtttacagt cctggtttta agttcaacac taactttatt cctaaagtaa atactaagta 240
tcggccgggc tatgatctta gctttgcctt aaagtttggt actagttgaa aagaagctta 300
tggtacaggt tggttaattg actggaagga tgttaagcag gacaacaaat ttactgctta 360
cttagctacc aacctccatg tagctgatag cttacgaaat aaagacgatt acaagcctta 420
caacaaggat ggtaatcaga aggagttttt acctggcgat atcaccactg aattttcttt 480
gggtaaatac attgatgccc aaactgtgca aaagttaact ccagagtacc aaaatcttaa 540
gcacctcaat aaccggaata 560

<210> 138

<211> 560

<212> DNA

<213> Mycoplasma pneumoniae

<400> 138

gcacctcaat aaccggaata gtgatgcatt agtttcaatt caaacatcga agttaccaaa 60

aactgcttac actgccactg actttatcaa aactgctcag tacaaataca atcacatagt 120

gagtaacaca gtttatgagt tggacttatt ccaaaatgcc gtaagttacg ctgactttgc 180

ggtgttggaa ttagagttaa atttagccaa caatcgtgac cagcaaatct ttgacagttt 240

tataaatcca gcggtaactg cttacgaaaa gctaggtaac agtttgggtc tcttttccaa 300

cctgcaatta gatcagtatg ttgatgatac ccattatcta ttgggttatc cgttacttaa 360

acgtgaaaag acgtcttact gaaacttacc acagaagggt tatagttcac cactctatga 420

aaatagtaat aaggaagttt cgcgcataac gcgtaacatc cggaaagatg atgaaattcc 480

aggtagtcgt ttggtacaaa atcaaattaa ctatttaccc tttgcacaaa atgaccctaa 540

aggcgtaatg gactttagca 560

<210> 139

<211> 21

<212> DNA

<213> Artificial Sequence

<220>

<223> Designed chimeric oligonucleotide primer designated as Myco-140 to amplify a portion of ATPase operon from Mycoplasma pneumoniae. "Nucleotides 19 to 21 are ribonucleotides-other nucleotides are deoxyribonucleotides."

<400> 139

attggagccaa actatccccca a 21

<210> 140

<211> 22

<212> DNA

<213> Artificial Sequence

<220>

<223> Designed chimeric oligonucleotide primer designated as Myco-140-22 to amplify a portion of ATPase operon from Mycoplasma pneumoniae. "Nucleotides 20 to 22 are ribonucleotides-other nucleotides are deoxyribonucleotides."

<400> 140

attgagccaa actatcccca aa 22

<210> 141

<211> 22

<212> DNA

<213> Artificial Sequence

<220>

<223> Designed chimeric oligonucleotide primer designated as Myco-706 to amplify a portion of ATPase operon from Mycoplasma pneumoniae. "Nucleotides 20 to 22 are ribonucleotides-other nucleotides are deoxyribonucleotides."

<400> 141

atcaaaactg ctcagtacaa au 22

<210> 142

<211> 22

<212> DNA

<213> Artificial Sequence

<220>

<223> Designed chimeric oligonucleotide primer designated as MPF-910 to amplify a portion of ATPase operon from Mycoplasma pneumoniae. "Nucleotides 20 to 22 are ribonucleotides-other nucleotides are deoxyribonucleotides."

<400> 142

ctcttttcca acctgcaatu ag 22

<210> 143

<211> 21

<212> DNA

<213> Artificial Sequence

<220>

<223> Designed chimeric oligonucleotide primer designated as Myco-190 to amplify a portion of ATPase operon from Mycoplasma pneumoniae. "Nucleotides 19 to 21 are ribonucleotides-other nucleotides are deoxyribonucleotides."

<400> 143

gtagcaacag catcgggcuc u 21

<210> 144

<211> 22

<212> DNA

<213> Artificial Sequence

<220>

<223> Designed chimeric oligonucleotide primer designated as Myco-190-22 to amplify a portion of ATPase operon from Mycoplasma pneumoniae. "Nucleotides 20 to 22 are ribonucleotides-other nucleotides are deoxyribonucleotides."

<400> 144

gtagcaacag catcgggcuc ua 22

<210> 145

<211> 22

<212> DNA

<213> Artificial Sequence

<220>

<223> Designed chimeric oligonucleotide primer designated as Myco-850 to amplify a portion of ATPase operon from Mycoplasma pneumoniae. "Nucleotides 20 to 22 are ribonucleotides-other nucleotides are deoxyribonucleotides."

<400> 145

cgtaacttac ggcattttgg aa 22

<210> 146
<211> 22
<212> DNA
<213> Artificial Sequence

<220>
<223> Designed chimeric oligonucleotide primer designated as MPR-1016 to amplify a portion of ATPase operon from Mycoplasma pneumoniae. "Nucleotides 20 to 22 are ribonucleotides-other nucleotides are deoxyribonucleotides."

<400> 146
ttctgtggta agtttcagta ag 22

<210> 147
<211> 19
<212> DNA
<213> Artificial Sequence

<220>
<223> Designed oligonucleotide probe as Myco170-probe to detect a DNA fragment amplifing a portion of ATPase operon from Mycoplasma pneumoniae.

<400> 147
ccagaggctg aaccagtta 19

<210> 148
<211> 20
<212> DNA

<213> Artificial Sequence

<220>

<223> Designed oligonucleotide probe as Myco730-probe to detect a DNA fragment amplifing a portion of ATPase operon from Mycoplasma pneumoniae.

<400> 148

caatcacata gtgagtaaca 20

<210> 149

<211> 20

<212> DNA

<213> Artificial Sequence

<220>

<223> Designed oligonucleotide probe as Myco952-probe to detect a DNA fragment amplifing a portion of ATPase operon from Mycoplasma pneumoniae.

<400> 149

cattatctat tgggttatcc 20

<210> 150

<211> 560

<212> DNA

<213> Staphylococcus aureus

<400> 150

caggatcgta aaataaaaaa agtatctaaa aataaaaaac gagtagatgc tcaatataaa 60

attaaaacaa actacggtaa cattgatcgc aacgttcaat ttaattttgt taaagaagat 120

ggtatgtgga agttagattg ggatcatagc gtcattattc caggaatgca gaaagaccaa 180

agcatacata ttgaaaattt aaaatcagaa cgtggtaaaa ttttagaccg aaacaatgtg 240

gaattggcca atacaggaac acatatgaga ttaggcatcg ttccaaagaa tgtatctaaa 300

aaagattata aagcaatcgc taaagaacta agtatttctg aagactatat caacaacaaa 360

tggatcaaaa ttgggtacaa gatgatacct tcgttccact ttaaaaccgt taaaaaaatg 420

gatgaatatt taagtgattt cgcaaaaaaa tttcatctta caactaatga aacagaaagt 480

cgtaactatc ctctagaaaa agcgacttca catctattag gttatgttgg tcccattaac 540

tctgaagaat taaaacaaaa 560

<210> 151

<211> 560

<212> DNA

<213> Staphylococcus aureus

<400> 151

aaaaatgatt atggctcagg tactgctatc caccctcaaa caggtgaatt attagcactt 60

gtaagcacac cttcatatga cgtctatcca tttatgtatg gcatgagtaa cgaagaatat 120

aataaattaa ccgaagataa aaaagaacct ctgctcaaca agttccagat tacaacttca 180

ccaggttcaa ctcaaaaaat attaacagca atgattgggt taaataacaa aacattagac 240

gataaaacaa gttataaaat cgatggtaaa ggttggcaaa aagataaatc ttggggtggt 300

tacaacgtta caagatatga agtggtaaat ggtaatatcg acttaaaaca agcaatagaa 360

tcatcagata acattttctt tgctagagta gcactcgaat taggcagtaa gaaatttgaa 420

aaaggcatga aaaaactagg tgttggtgaa gatataccaa gtgattatcc attttataat 480

gctcaaattt caaacaaaaa tttagataat gaaatattat tagctgattc aggttacgga 540

caaggtgaaa tactgattaa 560

<210> 152

<211> 23

<212> DNA

<213> Artificial Sequence

<220>

<223> Designed chimeric oligonucleotide primer designated as MecA-S525 to amplify a portion of MecA gene from Staphylococcus aureus. "Nucleotides 21 to 23 are ribonucleotides-other nucleotides are deoxyribonucleotides."

<400> 152

aaagaatgta tctaaaaaag auu 23

<210> 153

<211> 20

<212> DNA

<213> Artificial Sequence

<220>

<223> Designed chimeric oligonucleotide primer designated as MecA-A611 to amplify a portion of MecA gene from Staphylococcus aureus. "Nucleotides 18 to 20 are ribonucleotides-other nucleotides are deoxyribonucleotides."

<400> 153

aggtatcatc ttgtacccaa 20

<210> 154

<211> 23

<212> DNA

<213> Artificial Sequence

<220>

<223> Designed chimeric oligonucleotide primer designated as MecA-S1281 to amplify a portion of MecA gene from Staphylococcus aureus. "Nucleotides 21 to 23 are ribonucleotides-other nucleotides are deoxyribonucleotides."

<400> 154

cgatggtaaa ggttggcaaa aag 23

<210> 155

<211> 23

<212> DNA

<213> Artificial Sequence

<220>

<223> Designed chimeric oligonucleotide primer designated as MecA-A1341 to amplify a portion of MecA gene from Staphylococcus aureus. "Nucleotides 21 to 23 are ribonucleotides-other nucleotides are deoxyribonucleotides."

<400> 155

agtcgatatt accatttacc acu 23

<210> 156

<211> 20

<212> DNA

<213> Artificial Sequence

<220>

<223> Designed oligonucleotide probe as MecA-A probe to detect a DNA fragment amplifing a portion of MecA gene from Staphylococcus aureus.

<400> 156

gcaatcgcta aagaactaag 20

<210> 157

<211> 20

<212> DNA

<213> Artificial Sequence

<220>

<223> Designed oligonucleotide probe as MecA-B probe to detect a DNA fragment amplifing a portion of MecA gene from Staphylococcus aureus.

<400> 157

ttggggtggt tacaacgtta 20

<210> 158

<211> 22

<212> DNA

<213> Artificial Sequence

<220>

<223> Designed chimeric oligonucleotide primer designated as pDON-AI-1(22) to amplify a portion of pDON-AI plasmid DNA. "Nucleotides 20 to 22 are ribonucleotides-other nucleotides are deoxyribonucleotides."

<400> 158

actagctctg tatctggcgg ac 22

<210> 159

<211> 23

<212> DNA

<213> Artificial Sequence

<220>

<223> Designed chimeric oligonucleotide primer designated as pDON-AI-2(23) to amplify a portion of pDON-AI plasmid DNA. "Nucleotides 21 to 23 are ribonucleotides-other nucleotides are deoxyribonucleotides."

<400> 159

acgatcggga tttttggact cag 23

<210> 160

<211> 20

<212> DNA

<213> Artificial Sequence

<220>

<223> Designed oligonucleotide primer to amplify a portion of iNOS-encoding sequence from mouse.

<400> 160

atgccattga gttcatcaac 20

<210> 161

<211> 19
<212> DNA
<213> Artificial Sequence

<220>
<223> Designed oligonucleotide primer to amplify a portion of iNOS-encoding sequence from mouse

<400> 161
tcttggtggc aaagatgag 19

<210> 162
<211> 18
<212> DNA
<213> Artificial Sequence

<220>
<223> Designed chimeric oligonucleotide primer designated as MTIS 2F to amplify a portion of Mycobacterium tuberculosis. "Nucleotides 16 to 18 are ribonucleotides-other nucleotides are deoxyribonucleotides."

<400> 162
tctcgtccag cgccgcuu 18

<210> 163
<211> 21
<212> DNA
<213> Artificial Sequence

<220>

<223> Designed chimeric oligonucleotide primer designated as MTIS 2R to amplify a portion of Mycobacterium tuberculosis. "Nucleotides 19 to 21 are ribonucleotides-other nucleotides are deoxyribonucleotides."

<400> 163

gacaaaggcc acgtaggcga a 21

<210> 164

<211> 20

<212> DNA

<213> Artificial Sequence

<220>

<223> Designed chimeric oligonucleotide primer designated as pJDB 10F to amplify a portion of CppB gene from Neisseria gonorrhoeae. "Nucleotides 18 to 20 are ribonucleotides-other nucleotides are deoxyribonucleotides."

<400> 164

atgcctcgtt gatatttuuc 20

<210> 165

<211> 17

<212> DNA

<213> Artificial Sequence

<220>

<223> Designed chimeric oligonucleotide primer designated as pJDB 10R to

amplify a portion of CppB gene from Neisseria gonorrhoeae. "Nucleotides 15 to 17 are ribonucleotides-other nucleotides are deoxyribonucleotides."

<400> 165
cgaagccgcc agcauag 17

<210> 166
<211> 22
<212> DNA
<213> Artificial Sequence

<220>
<223> Designed chimeric oligonucleotide primer designated as CT-F1215-4R-22 to amplify a portion of plasmid DNA from Chlamydia trachomatis. "Nucleotides 19 to 22 are ribonucleotides-other nucleotides are deoxyribonucleotides."

<400> 166
tcctgtgacc ttcattatgu cg 22

<210> 167
<211> 18
<212> DNA
<213> Artificial Sequence

<220>
<223> Designed chimeric oligonucleotide primer designated as CT-R1267-3R-18 to amplify a portion of plasmid DNA from Chlamydia

trachomatis. "Nucleotides 16 to 18 are ribonucleotides-other nucleotides are deoxyribonucleotides."

<400> 167
cggagtacaa acgccuag 18

<210> 168
<211> 15
<212> DNA
<213> Artificial Sequence

<220>
<223> Designed oligonucleotide probe as CT-1236 probe to detect a DNA fragment amplifing a portion of plasmid DNA from Chlamydia trachomatis.

<400> 168
ggagtctgag caccc 15

<210> 169
<211> 169
<212> DNA
<213> Artificial Sequence

<220>
<223> Designed nucleotide as Internal Control for Mycobacterium tuberculosis assay.

<400> 169
ccacgatcgc tgatccggcc acagcccgtc ccgccgatct cgtccagcgc cgcttcggac 60

caccagcacc taaccggctg tgggtagcac ctccgatcgt tgtcagaagc tgggcagggt 120

tcgcctacgt ggcctttgtc accgacgcct acgctcgcag gatcctggg 169

<210> 170

<211> 16

<212> DNA

<213> Artificial Sequence

<220>

<223> Designed chimeric oligonucleotide primer designated as MTIS2F16 to amplify a portion of Mycobacterium tuberculosis DNA. "nucleotides 14 to 16 are ribonucleotides-other nucleotides are deoxyribonucleotides."

<400> 170

tcgtccagcg ccgcuu 16

<210> 171

<211> 20

<212> DNA

<213> Artificial Sequence

<220>

<223> Designed chimeric oligonucleotide primer designated as MTIS2RAAC to amplify a portion of Mycobacterium tuberculosis DNA. "nucleotides 18 to 20 are ribonucleotides-other nucleotides are deoxyribonucleotides."

<400> 171

caaaggccac gtaggcgaac 20

<210> 172

<211> 20

<212> DNA

<213> Artificial Sequence

<220>

<223> Designed oligonucleotide probe designated as MTIS-S-PROBE to detect a DNA fragment amplifying a portion of nucleotide sequence from Mycobacterium tuberculosis.

<400> 172

gacctcacct atgtgtcgac 20

<210> 173

<211> 20

<212> DNA

<213> Artificial Sequence

<220>

<223> Designed oligonucleotide probe designated as INTER-PROBE to detect a DNA fragment amplifying a portion of nucleotide sequence from Mycobacterium tuberculosis.

<400> 173

cctccgatcg ttgtcagaag 20

## Description

Technical Field

The present invention relates to a method for stabilizing and storing a reaction reagent for a method for amplifying and/or detecting a target nucleic acid which is useful in fields of genetic engineering and clinical medicine.

Background Art

DNA synthesis is used for various purposes in studies in a field of genetic engineering. Most of the DNA synthesis with the exception of that of a short-chain DNA (e.g., an oligonucleotide) is carried out using an enzymatic method in which a DNA polymerase is utilized. An exemplary method is the polymerase chain reaction (PCR) method as described in United States Patent Nos. 4,683,195, 4,683,202 and 4,800,159 in detail. Another example is the reverse transcription-PCR (RT-PCR) method as described in Trends in Biotechnology, 10:146-152 (1992).

Alternatively, the ligase chain reaction (LCR) method as described in EP 320,308 or the transcription-based amplification system (TAS) method as described in PCR Protocols, Academic Press Inc., 1990, pp. 245-252 may be used. The four methods as mentioned above require repeating a reaction at a high temperature and that at a low temperature several times in order to regenerate a single-stranded target molecule for the next amplification cycle. The reaction system should be conducted using discontinuous phases or cycles because the reaction is restricted by the temperatures as described above. Thus, the methods require the use of an expensive thermal cycler that can strictly adjust a wide range of temperatures over time. Furthermore, the reaction requires time for adjusting the temperature to the two or three predetermined ones. The loss of time increases in proportion to the cycle number.

Nucleic acid amplification methods that can be carried out isothermally have been developed in order to solve the problems. Examples thereof include the strand displacement amplification (SDA) method as described in JP-B 7-114718, the self-sustained sequence replication (3SR) method, the nucleic acid sequence based amplification (NASBA) method as described in Japanese Patent No. 2650159, the transcription-mediated amplification (TMA) method, the Qβ replicase method as described in Japanese Patent No. 2710159 and the various modified SDA methods as described in United States Patent No. 5,824,517, WO 99/09211, WO 95/25180 and WO 99/49081. A method of isothermal enzymatic synthesis of an oligonucleotide is described in United States Patent No. 5,916,777. Extension from a primer and/or annealing of a primer to a single-stranded extension product (or to an original target sequence) followed by extension from the primer take place in parallel in a reaction mixture incubated at a constant temperature in the reaction of such a method of isothermal nucleic acid amplification or oligonucleotide synthesis.

Among the isothermal nucleic acid amplification methods; the SDA method is an example of systems in which a DNA is finally amplified. The SDA method is a method for amplifying a target nucleic acid sequence (and a complementary strand thereof) in a sample by displacement of double strands using a DNA polymerase and a restriction endonuclease. The method requires four primers used for the amplification, two of which should be designed to contain a recognition site for the restriction endonuclease. The method requires the use of a modified deoxyribonucleotide triphosphate as a substrate for DNA synthesis in large quantities. An example of the modified deoxyribonucleotide triphosphates is an (α-S) deoxyribonucleotide triphosphate in which the oxygen atom of the phosphate group at the α-position is replaced by a sulfur atom (S). The problem of running cost associated with the use of the modified deoxyribonucleotide triphosphate becomes serious if the reaction is routinely conducted, for example, for genetic test. Furthermore, the incorporation of the modified nucleotide (e.g., the (α-S) deoxyribonucleotide) into the amplified DNA fragment in the method may abolish the cleavability of the amplified DNA fragment with a restriction enzyme, for example, when it is subjected to a restriction enzyme fragment length polymorphism (RFLP) analysis.

The modified SDA method as described in United States Patent No. 5,824,517 is a DNA amplification method that uses a chimeric primer that is composed of an RNA and a DNA and has, as an essential element, a structure in which DNA is positioned at least at the 3'-terminus. The modified SDA method as described in WO 99/09211 requires the use of a restriction enzyme that generates a 3'-protruding end. The modified SDA method as described in WO 95/25180 requires the use of at least two pairs of primers. The modified SDA method as described in WO 99/49081 requires the use of at least two pairs of primers and at least one modified deoxyribonucleotide triphosphate. On the other hand, the method for synthesizing an oligonucleotide as described in United States Patent No. 5,916,777 comprises synthesizing a DNA using a primer having a ribonucleotide at the 3'-terminus, completing a reaction using the primer, introducing a nick between the primer and an extended strand in an primer-extended strand with an endonuclease to separate them from each other, digesting a template and recovering the primer to reuse it. It is required to isolate the primer from the reaction system and then anneal it to the template again in order to reuse the primer in the method. Additionally, the Loop-mediated Isothermal Amplification (LAMP) method as described in WO 00/28082 requires four primers for amplification and the products amplified using the method are DNAs having varying size in which the target regions for the amplification are repeated.

Furthermore, an isothermal nucleic acid amplification method using a chimeric oligonucleotide primer, Isothermal and Chimeric primer-initiated Amplification of Nucleic acids (ICAN) method, as described in WO 00/56877 or WO 02/7139 is known.

Since many of reaction reagents used for the above-mentioned methods are unstable at room temperature, the reagents are used for operations while cooling on ice in most cases. Furthermore, since most of the reaction reagents should be stored 4°C or below, or -20°C or below, one needs to pay strict attention to the storage methods. Thus, a method for stabilizing and/or storing a reaction reagent which enables stable long-term storage at room temperature without a need of a refrigerator or a freezer for the storage has been desired.

Objects of Invention

The main object of the present invention is to provide a method of stabilization and long-term storage of a reaction reagent for a target nucleic acid amplification method by which a target nucleic acid in a sample is specifically amplified with a high sensitivity using a chimeric oligonucleotide primer, as well as a highly sensitive method for detecting a pathogenic microorganism or a virus.

Summary of Invention

As a result of intensive studies, the present inventors have found a highly sensitive method for detecting a virus or a pathogenic microorganism using a method in which a region of a DNA of interest is amplified in the presence of a chimeric oligonucleotide primer, an endonuclease and a DNA polymerase. The present inventors have further found a method of stabilization and long-term storage of a reagent for the method for amplifying a region of a DNA of interest. Thus, the present invention has been completed.

The first aspect of the present invention relates to a method for stabilizing a reaction reagent used for a method for amplifying and/or detecting a target nucleic acid that comprises:

(a) preparing a reaction mixture by mixing a nucleic acid as a template, a deoxyribonucleotide triphosphate, a DNA polymerase having a strand displacement activity, at least one primer and an RNase H, wherein the primer is a chimeric oligonucleotide primer that is substantially complementary to the nucleotide sequence of the nucleic acid as the template and contains a ribonucleotide as well as at least one selected from the group consisting of a deoxyribonucleotide and a nucleotide analog, the ribonucleotide being positioned at the 3'-terminus or on the 3'-terminal side of the primer; and
(b) amplifying a target nucleic acid by incubating the reaction mixture for a sufficient time to generate a reaction product,

wherein

(i) at least one reagent component selected from the group consisting of a magnesium salt, the chimeric oligonucleotide primer and the enzymes (the DNA polymerase and/or the RNase H) is separated from other reagent components prior to the reaction; and
(ii) the enzyme concentration(s) of a reagent solution containing the enzyme(s) is (are) elevated while the salt concentration of said solution is not elevated, and the salt concentration of another reagent solution is adjusted such that the optimal salt concentration for the amplification step is achieved after mixing the separated reagent solutions each other.

According to the first aspect, the reaction reagent may consist of two reagent solutions: a reagent solution containing the chimeric oligonucleotide primer; and a reagent solution containing the enzyme(s) (the DNA polymerase and/or the RNase H) and the magnesium salt may be used. The salt concentration of the reagent solution containing the enzyme(s) (the DNA polymerase and/or the RNase H) may be equal to or lower than the optimal salt concentration for the amplification step. The enzyme concentration(s) of the reagent solution containing the enzyme(s) (the DNA polymerase and/or the RNase H) may be higher than the enzyme concentration(s) for the amplification step. The enzyme concentration(s) of the reagent solution containing the enzyme(s) (the DNA polymerase and/or the RNase H) may be adjusted such that the optimal enzyme concentration(s) for the amplification step is (are) achieved after mixing the separated reagent solutions each other.

The second aspect of the present invention relates to a kit of a reaction reagent used for a method for amplifying and/or detecting a target nucleic acid that comprises:

(a) preparing a reaction mixture by mixing a nucleic acid as a template, a deoxyribonucleotide triphosphate, a DNA polymerase having a strand displacement activity, at least one primer and an RNase H, wherein the primer is a chimeric oligonucleotide primer that is substantially complementary to the nucleotide sequence of the nucleic acid as the template and contains a ribonucleotide as well as at least one selected from the group consisting of a deoxyribonucleotide and a nucleotide analog, the ribonucleotide being positioned at the 3'-terminus or on the 3'-terminal side of the primer; and

(b) amplifying a target nucleic acid by incubating the reaction mixture for a sufficient time to generate a reaction product,

wherein

(i) at least one reagent component selected from the group consisting of a magnesium salt, the chimeric oligonucleotide primer and the enzymes (the DNA polymerase and/or the RNase H) is separated from other reagent components prior to the reaction; and

(ii) the enzyme concentration(s) of a reagent solution containing the enzyme(s) is (are) elevated while the salt concentration of said solution is not elevated, and the salt concentration of another reagent solution is adjusted such that the optimal salt concentration for the amplification step is achieved after mixing the separated reagent solutions each other.

According to the second aspect, the reaction reagent may consist of two reagent solutions: a reagent solution containing the chimeric oligonucleotide primer; and a reagent solution containing the enzyme(s) (the DNA polymerase and/or the RNase H) and the magnesium salt. The salt concentration of the reagent solution containing the enzyme (s) (the DNA polymerase and/or the RNase H) may be equal to or lower than the optimal salt concentration for the amplification step. The enzyme concentration(s) of the reagent solution containing the enzyme(s) (the DNA polymerase and/or the RNase H) may be higher than the enzyme concentration(s) for the amplification step. The enzyme concentration(s).of the reagent solution containing the enzyme(s) (the DNA polymerase and/or the RNase H) may be adjusted such that the optimal enzyme concentration(s) for the amplification step is (are) achieved after mixing the separated reagent solutions each other. The kit may contain a reagent for adjusting the salt concentration of the mixture of the separated reagent solutions.

The third aspect of the present invention relates to a method for detecting a pathogenic microorganism and/or a virus in a sample, the method comprising:

(a) preparing a reaction mixture by mixing a nucleic acid as a template, a deoxyribonucleotide triphosphate, a DNA polymerase having a strand displacement activity, at least one primer and an RNase H, wherein the primer is a chimeric oligonucleotide primer that is substantially complementary to the nucleotide sequence of the nucleic acid as the template and contains a ribonucleotide as well as at least one selected from the group consisting of a deoxyribonucleotide and a nucleotide analog, the ribonucleotide being positioned at the 3'-terminus or on the 3'-terminal side of the primer; and

(b) amplifying a target nucleic acid by incubating the reaction mixture for a sufficient time to generate a reaction product; and

(c) detecting the target nucleic acid amplified in step (b),

wherein the chimeric oligonucleotide primer is a chimeric oligonucleotide primer for detecting a pathogenic microorganism represented by the following general formula, and the pathogenic microorganism is selected from the group consisting of Mycobacterium tuberculosis, HCV, a chlamydia, a Mycobacterium avium complex, a gonococcus, HBV, HIV, Staphylococcus aureus, a mycoplasma and MRSA:

General formula: 5'-dNa-Nb-dNc-3'

(a: an integer of 11 or more; b: an integer of 1 or more; c: 0 or an integer of 1 or more; dN: deoxyribonucleotide and/or nucleotide analog; N: unmodified ribonucleotide and/or modified ribonucleotide, wherein some of dNs in dNa may be replaced by Ns, and the nucleotide at the 3'-terminus may be modified such that extension from the 3'-terminus by the action of the DNA polymerase does not take place).

According to the third aspect, the reaction mixture may further contain a chimeric oligonucleotide, primer having a sequence substantially homologous to the nucleotide sequence of the nucleic acid as the template.

The fourth aspect of the present invention relates to a primer for detecting a pathogenic microorganism and/or a virus, the primer being selected from the group consisting of:

(1) a chimeric oligonucleotide primer for detecting Mycobacterium tuberculosis having a nucleotide sequence of one selected from the group consisting of SEQ ID NOS:7, 8, 21, 22, 162, 163, 170 and 171;

(2) a chimeric oligonucleotide primer for detecting HCV having a nucleotide sequence of one selected from the group consisting of SEQ ID NOS:15, 16, 81-86 and 88-91;

(3) a chimeric oligonucleotide primer for detecting a chlamydia having a nucleotide sequence of one selected from the group consisting of SEQ ID NOS:17-20, 121-127, 130-135, 167 and 167;

(4) a chimeric oligonucleotide primer for detecting a Mycobacterium avium complex having a nucleotide sequence of one selected from the group consisting of SEQ ID NOS:25-31;

(5) a chimeric oligonucleotide primer for detecting a gonococcus having a nucleotide sequence of one selected from the group consisting of SEQ ID NOS:38-63, 164 and 165;

(6) a chimeric oligonucleotide primer for detecting HBV having a nucleotide sequence of one selected from the group consisting of SEQ ID NOS:71-78;

(7) a chimeric oligonucleotide primer for detecting HIV having a nucleotide sequence of one selected from the group consisting of SEQ ID NOS:95-103;

(8) a chimeric oligonucleotide primer for detecting Staphylococcus aureus having a nucleotide sequence of one selected from the group consisting of SEQ ID NOS:108-117;

(9) a chimeric oligonucleotide primer for detecting a mycoplasma having a nucleotide sequence of one selected from the group consisting of SEQ ID NOS:139-146; and

(10) a chimeric oligonucleotide primer for detecting MRSA having a nucleotide sequence of one selected from the group consisting of SEQ ID NOS:152-155.

The fifth aspect of the present invention relates to a probe for detecting a pathogenic microorganism and/or a virus, the primer being selected from the group consisting of:

(1) a probe for detecting Mycobacterium tuberculosis selected from a region containing a nucleotide sequence of one selected from the group consisting of SEQ ID NOS:11, 12 and 172;

(2) a probe for detecting HCV selected from a region containing a nucleotide sequence of one selected from the group consisting of SEQ ID NOS:87 and 92;

(3) a probe for detecting a chlamydia selected from a region containing a nucleotide sequence of one selected from the group consisting of SEQ ID NOS:128, 136 and 168;

(4) a probe for detecting a Mycobacterium avium complex selected from a region containing a nucleotide sequence of one selected from the group consisting of SEQ ID NOS:32 and 33;

(5) a probe for detecting a gonococcus selected from a region containing a nucleotide sequence of one selected from the group consisting of SEQ ID NOS:64-68;

(6) a probe for detecting HBV selected from a region containing a nucleotide sequence of one selected from the group consisting of SEQ ID NOS:79 and 80;

(7) a probe for detecting HIV selected from a region containing a nucleotide sequence of one selected from the group consisting of SEQ ID NOS:104 and 105;

(8) a probe for detecting Staphylococcus aureus selected from a region containing a nucleotide sequence of one selected from the group consisting of SEQ ID NOS:118 and 119;

(9) a probe for detecting a mycoplasma selected from a region containing a nucleotide sequence of one selected from the group consisting of SEQ ID NOS:147-149; and

(10) a probe for detecting MRSA selected from a region containing a nucleotide sequence of one selected from the group consisting of SEQ ID NOS:156 and 157.

The sixth aspect of the present invention relates to a kit used for the method for detecting a pathogenic microorganism and/or a virus of the third aspect, which contains the chimeric oligonucleotide primer of the ninth aspect.

The kit of the sixth aspect may further contain the probe of the fifth aspect.

Brief Description of Drawings

Figure 1 is a figure showing the polyacrylamide gel electrophoresis of DNA fragments amplified according to the method of the present invention.

Figure 2 is a chart showing the real-time detection of DNA fragments amplified according to the method of the present invention.

Figure 3 is a figure showing the agarose gel electrophoresis that represents the results of storage stability tests of the reagents used for the method of the present invention.

Figure 4 is a figure showing the autoradiography that represents the results of storage stability tests of the reagents used for the method of the present invention.

Figure 5 is a figure showing the agarose gel electrophoresis that represents the results of storage stability tests of the reagents used for the method of the present invention.

Figure 6 is a figure showing the agarose gel electrophoresis that represents the results of storage stability tests of the reagents used for the method of the present invention.

Figure 7 is a figure showing the agarose gel electrophoresis of DNA fragments amplified according to the method of the present invention.

Detailed Description of the Invention

As used herein, a deoxyribonucleotide (also referred to as a dN) refers to a nucleotide of which the sugar portion is composed of D-2-deoxyribose. The deoxyribonucleotides include, for example, ones having adenine, cytosine, guanine or thymine as the base portion. Furthermore, the deoxyribonucleotides also include a deoxyribonucleotide having a modified base such as 7-deazaguanosine and a deoxyribonucleotide analog such as deoxyinosine nucleotide.

As used herein, a ribonucleotide (also referred to as an N) refers to a nucleotide of which the sugar portion is composed of D-ribose. The ribonucleotides include ones having adenine, cytosine, guanine or uracil as the base portion. The ribonucleotides also include modified ribonucleotides such as a modified ribonucleotide in which the oxygen atom of the phosphate group at the $\alpha$-position is replaced by a sulfur atom (also referred to as an ($\alpha$-S) ribonucleotide or an ($\alpha$-S) N) or other derivatives.

The chimeric oligonucleotide primers used in the present invention include any chimeric oligonucleotide primer that has a ribonucleotide being positioned at the 3'-terminus or on the 3'-terminal side of the primer, can be used to extend a nucleic acid strand in the method of the present invention, can be cleaved with an endonuclease, and can be used to effect a strand displacement reaction.

As used herein, 3'-terminal side refers to a portion from the center to the 3'-terminus of a nucleic acid such as a primer. Likewise, 5'-terminal side refers to a portion from the center to the 5' terminus of a nucleic acid.

The chimeric oligonucleotide primer is one that contains a ribonucleotide as well as at least one selected from the group consisting of a deoxyribonucleotide and a nucleotide analog. Such primers also include an oligoribonucleotide primer that contains an unmodified ribonucleotide and/or a modified ribonucleotide.

The chimeric oligonucleotide primer used in the method of the present invention is one that has a nucleotide sequence substantially complementary to a part of the nucleotide sequence of a nucleic acid as a template. It can contribute to extension of a DNA strand under conditions used. Furthermore, a ribonucleotide is positioned at the 3'-terminus or on the 3'-terminal side of the chimeric oligonucleotide primer. The primer is usually designed such that it is complementary to a portion upstream of the region to be amplified, that is, a portion 3' to the nucleotide sequence corresponding to a region to be amplified in a nucleic acid as a template. As used herein, "a substantially complementary nucleotide sequence" means a nucleotide sequence that can anneal to a DNA as a template under reaction conditions used.

The chimeric oligonucleotide primer used in the method of the present invention may contain one or more modified ribonucleotide. A ribonucleotide may be an unmodified ribonucleotide and/or a modified ribonucleotide that can be positioned at the 3'-terminus or on the 3'-terminal side of a chimeric oligonucleotide primer and that is recognized by or cleaved with an endonuclease. The ribonucleotides include both of the unmodified ribonucleotide and the modified ribonucleotide as described above. An unmodified ribonucleotide, a modified ribonucleotide or a combination thereof can be used for the chimeric oligonucleotide primer of the present invention as long as it does not abolish the function of the primer. Examples of the modified ribonucleotides include, but are not limited to, an ($\alpha$-S) ribonucleotide in which the oxygen atom bound to the phosphate group is replaced by a sulfur atom, and a ribonucleotide in which the hydroxy group at the 2-position of the ribose is replaced by a methoxy group. Furthermore, the chimeric oligonucleotide primer used in the method of the present invention may contain a nucleotide analog or other substances. That is, one or more nucleotide analog(s) can be contained in the chimeric oligonucleotide primer of the present invention as long as the function of the primer for effecting a polymerase extension reaction from the 3'-terminus by the action of a DNA polymerase is not abolished. Plural types of the nucleotide analogs can be used in combination. Examples of the nucleotide analogs that can be used include, but are not limited to, deoxyinosine nucleotide, deoxyuracil nucleotide, a deoxyribonucleotide analog having a modified base such as 7-deazaguanine, a nucleotide analog having a ribose derivative and the like. Furthermore, the chimeric oligonucleotides used in the present invention may contain deoxynucleotides, ribonucleotides or nucleotide analogs having various modifications such as addition of labeled compounds as long as they retain the functions as described above.

Incorporation of a nucleotide analog into a primer is effective for suppressing the formation of high-order structure of the primer itself and stabilization of annealing formation with the template. A ribonucleotide may be incorporated into a primer for the same purpose. Although it is not intended to limit the present invention, a modified ribonucleotide such as ($\alpha$-S) ribonucleotide can be preferably used in order to prevent the digestion of the primer by a non-specific endonuclease (RNase). Such a chimeric oligonucleotide primer containing a modified ribonucleotide can be produced by using, for example, an (α-S) ribonucleotide triphosphate, which is prepared by a method using a sulfuration reaction reagent (Glen Research) as described in United States Patent No. 5,003,097, or a 2-OMe-RNA-CE phosphoramidite reagent (Glen Research).

A chimeric oligonucleotide primer that can be used in the amplification method of the present invention may be designed to contain a modified ribonucleotide that confers resistance to the cleavage with an endonuclease. Such a primer is useful in that one can control the cleavage site with an endonuclease during amplification reaction steps.

The length of the chimeric oligonucleotide primer used in the method of the present invention is not specifically limited, but is preferably about 12 nucleotides to about 100 nucleotides, more preferably about 15 nucleotides to about 40 nucleotides. It is preferable that the nucleotide sequence of the chimeric oligonucleotide is substantially complementary to a nucleic acid as a template such that it anneals to the nucleic acid as the template under reaction conditions used. The primer contains a sequence recognized by an endonuclease, which is utilized in a step as described below, at the 3'-terminus or on the 3'-terminal side.

For example, an oligonucleotide having a structure represented by the following general formula can be used in the DNA synthesis method of the present invention as a primer, although it is not intended to limit the present invention:

General formula: 5'-dNa-Nb-dNc-3'

(a: an integer of 11 or more; b: an integer of 1 or more; c: 0 or an integer of 1 or more; dN: deoxyribonucleotide and/or nucleotide analog; N: unmodified ribonucleotide and/or modified ribonucleotide, wherein some of dNs in dNa may be replaced by Ns, and the nucleotide at the 3'-terminus may be modified such that extension from the 3'-terminus by the action of the DNA polymerase does not take place).

The chimeric oligonucleotide primer used in the present invention has a structure in which an endonuclease recognizes or cleaves a DNA strand extended from the primer using a DNA polymerase (a primer-extended strand) at a site that contains a ribonucleotide, which ribonucleotide being positioned at the 3'-terminus or on the 3'-terminal side of the chimeric oligonucleotide primer. Although it is not intended to limit the present invention, for example, when an RNase H acts on a double-stranded DNA generated by extending a DNA from a chimeric oligonucleotide primer represented by the general formula that has been annealed to a nucleic acid as a template, the chimeric oligonucleotide primer is cleaved at the ribonucleotide portion. A double-stranded DNA in which a nick is introduced between the oligonucleotide primer and the DNA strand synthesized by the extension is then generated. Then, a strand displacement reaction with a DNA polymerase proceeds from the nicked site. Thus, any chimeric oligonucleotide primer that can be used to extend a nucleic acid strand from the 3'-terminus of the primer, that can be cleaved with an endonuclease, and with which a DNA polymerase can effect a strand displacement reaction can be used in the method of the present invention. Furthermore, the chimeric oligonucleotide primers of the present invention include one whose 3'-terminus is modified such that extension by the action of the DNA polymerase cannot take place, and DNA extension takes place from a 3'-terminus generated upon cleavage by the endonuclease.

In addition, a promoter sequence for an RNA polymerase may be included on the 5'-terminal side of the chimeric oligonucleotide primer. Such RNA polymerases are exemplified by T7 RNA polymerase and SP6 RNA polymerase.

The chimeric oligonucleotide primer can be synthesized to have desired nucleotide sequence using, for example, the 394 type DNA synthesizer from Applied Biosystems Inc. (ABI) according to a phosphoramidite method. Alternatively, any methods including a phosphate triester method, an H-phosphonate method and a thiophosphonate method may be used to synthesize the chimeric oligonucleotide primer.

An enzyme that can act on a double-stranded DNA generated by DNA extension from the chimeric oligonucleotide primer as described above that has been annealed to a nucleic acid as a template and cleaves the extended strand to effect a strand displacement reaction may be used. That is, it is an enzyme that can generate a nick in the chimeric oligonucleotide primer portion of the double-stranded DNA. Examples of endonucleases that can be used in the present invention include, but are not limited to, ribonucleases. Among these, endoribonuclease H (RNase H) that acts on an RNA portion of a double-stranded nucleic acid composed of a DNA and an RNA can be preferably used. Any ribonuclease that has the above-mentioned activities can be preferably used in the present invention, including mesophilic and heat-resistant ones. For example, an RNase H from E. coli can be used for a reaction at about 50°C to about 70°C in the method of the present invention as described below in Examples. A heat-resistant ribonuclease can be preferably used in the method of the present invention. Examples of the heat-resistant ribonucleases which can be preferably used include, but are not limited to, a commercially available ribonuclease, Hybridase™ Thermostable RNase H (Epicenter Technologies) as well as an RNase H from a thermophilic bacterium of the genus Bacillus, a bacterium of the genus Thermus, a bacterium of the genus Pyrococcus, a bacterium of the genus Thermotoga, a bacterium of the genus Archaeoglobus, a bacterium of the genus Methanococcus, a bacterium of the genus Thermococcus or the like. Furthermore, both of naturally occurring ribonucleases and variants can be preferably used.

The RNase H is not limited to a specific one as long as it can be used in the method of the present invention. For example, the RNase H may be derived from various viruses, phages, prokaryotes or eukaryotes. It may be either a cellular RNase H or a viral RNase H. The cellular RNase H is exemplified by Escherichia coli RNase HI and the viral RNase H is exemplified by HIV-1 RNase H. Type I, type II or type III RNase H can be used in the method of the present invention. For example, RNase HI from Escherichia coli, or RNase HII from a bacterium of the genus Pyrococcus, a bacterium of the genus Archaeoglobus or a bacterium of the genus Thermococcus can be preferably used, without limitation.

The efficiency of the cleavage reaction with an endonuclease such as RNase H used in the method of the present invention may vary depending on the nucleotide sequence around the 3' terminus of the primer and influence the amplification efficiency of the desired DNA. Therefore, it is natural to design the optimal primer for the RNase H used.

As used herein, the term "introducing a nick" or "nicking" means internally cleaving one of the two strands of a double-stranded nucleic acid. For example, an RNase H acts on a hybrid double-stranded nucleic acid composed of a DNA and a ribonucleotide-containing DNA to selectively cleave the ribonucleotide-containing strand among the two strands at the ribonucleotide portion, thereby introducing a nick into the hybrid double-stranded nucleic acid.

It is known that some DNA polymerases have an endonuclease activity such as an RNase H activity under specific conditions. Such a DNA polymerase can be used in the method of the present invention. In one aspect, the DNA polymerase may be used under conditions that allow the RNase H activity to express, e.g., in the presence of $Mn^{2+}$. In this case, the method of the present invention can be conducted without the addition of an RNase H. Bca DNA polymerase can exhibit an RNase activity in a buffer containing $Mn^{2+}$. The above-mentioned aspect is not limited to the use of the Bca DNA polymerase. DNA polymerases that are known to have an RNase H activity such as Tth DNA polymerase from Thermus thermophilus can be used in the present invention.

Thus, a DNA polymerase having an RNase H activity can be used under conditions under which the RNase H activity is exhibited.

dNTPs used for the PCR or the like (a mixture of dATP, dCTP, dGTP and dTTP) can be preferably used as nucleotide triphosphates that serve as substrates in the extension reaction in the method. In addition, dUTP may be used as a substrate. The dNTPs may contain a dNTP (deoxyribonucleotide triphosphate) analog such as 7-deaza-dGTP, triphosphate of dITP or the like as long as it serves as a substrate for the DNA polymerase used. A derivative of a dNTP or a dNTP analog may be used. A derivative having a functional group such as a dUTP having an amino group may be contained. A chimeric oligonucleotide primer is used in the method. The primer can be prepared, for example, using a DNA synthesizer according to a conventional synthesis method. A combination of the chimeric oligonucleotide primer and a normal oligonucleotide primer can be used in the method of the present invention.

If the activity of the enzyme used may be decreased in the course of the reaction, the enzyme can be further added during the reaction in the method of the present invention. Although it is not intended to limit the present invention, for example, an RNase H from Escherichia coli may be further added during a reaction in which the RNase H is used. The added enzyme may be the same as that contained in the reaction mixture at the beginning of the reaction, or it may be a different enzyme that exhibits the same activity. Thus, the type or the property of the enzyme to be added is not limited to a specific one as long as the addition during the reaction provides effects such as increase in the detection sensitivity or increase in the amount of amplification product.

As used herein, a DNA polymerase refers to an enzyme that synthesizes a DNA strand de novo using a DNA strand as a template. The DNA polymerases include naturally occurring DNA polymerases and variant enzymes having the above-mentioned activity. For example, such enzymes include a DNA polymerase having a strand displacement activity, a DNA polymerase lacking a 5'→3' exonuclease activity and a DNA polymerase having a reverse transcriptase activity or an endonuclease activity.

As used herein, "a strand displacement activity" refers to an activity that can effect a strand displacement, that is, that can proceed DNA duplication on the basis of the sequence of the nucleic acid as the template while displacing the DNA strand to release the complementary strand that has been annealed to the template strand. In addition, a DNA strand released from a nucleic acid as a template as a result of a strand displacement is referred to as "a displaced strand" herein.

A DNA polymerase having a strand displacement activity on a DNA can be used. Particularly, a DNA polymerase substantially lacking a 5'→3' exonuclease activity can be preferably used.

Any DNA polymerases having the strand displacement activity can be used in the present invention. Examples thereof include variants of DNA polymerases lacking their 5'→3' exonuclease activities derived from thermophilic bacteria of the genus Bacillus such as Bacillus caldotenax (hereinafter referred to as B. ca) and Bacillus stearothermophilus (hereinafter referred to as B. st), as well as large fragment (Klenow fragment) of DNA polymerase I from Escherichia coli (E. coli). Both of mesophilic and heat-resistant DNA polymerases can be preferably used in the present invention.

B. ca is a thermophilic bacterium having an optimal growth temperature of about 70°C. Bca DNA polymerase from this bacterium is known to have a DNA-dependent DNA polymerase activity, an RNA-dependent DNA polymerase activity (a reverse transcription activity), a 5'→3' exonuclease activity and a 3'→5' exonuclease activity. The enzyme may be either an enzyme purified from its original source or a recombinant protein produced by using genetic engineering techniques. The enzyme may be subjected to modification such as substitution, deletion, addition or insertion by using genetic engineering techniques or other means. Examples of such enzymes include BcaBEST DNA polymerase (Takara Shuzo), which is Bca DNA polymerase lacking its 5'→3' exonuclease activity.

The nucleic acid (DNA or RNA) used as a template according to the present invention may be prepared or isolated from any sample that may contain the nucleic acid. Alternatively, the sample may be used directly in the nucleic acid amplification reaction according to the present invention. Examples of the samples that may contain the nucleic acid include, but are not limited to, samples from organisms such as a whole blood, a serum, a buffy coat, a urine, feces, a cerebrospinal fluid, a seminal fluid, a saliva, a tissue (e.g., a cancerous tissue or a lymph node) and a cell culture (e.g., a mammalian cell culture or a bacterial cell culture), samples that contain a nucleic acid such as a viroid, a virus, a bacterium, a fungi, a yeast, a plant and an animal, samples suspected to be contaminated or infected with a microorganism such as a virus or a bacterium (e.g., a food or a biological formulation), and samples that may contain an organism such as a soil and a waste water. The sample may be a preparation containing a nucleic acid obtained by processing the above-mentioned samples according to a known method. Examples of the preparations that can be used in the present invention include a cell destruction product or a sample obtained by fractionating the product, the nucleic acid in the sample, or a sample in which specific nucleic acid molecules such as mRNAs are enriched. Furthermore, a nucleic acid such as a DNA or an RNA obtained amplifying a nucleic acid contained in the sample using a known method can be preferably used.

The preparation containing a nucleic acid can be prepared from the above-mentioned materials by using, for example, lysis with a detergent, sonication, shaking/stirring using glass beads or a French press, without limitation. In some cases, it is advantageous to further process the preparation to purify the nucleic acid (e.g., in case where an endogenous nuclease exists). In such cases, the nucleic acid is purified using a know method such as phenol extraction, chromatography, ion exchange, gel electrophoresis or density-gradient centrifugation.

When it is desired to amplify a nucleic acid having a sequence derived from an RNA, the method of the present invention may be conducted using, as a template, a cDNA synthesized using a reverse transcription reaction that uses the RNA as a template. Any RNA for which one can make a primer to be used in a reverse transcription reaction can be applied to the method of the present invention, including total RNA in a sample, RNA molecules such as, mRNA, tRNA and rRNA as well as specific RNA molecular species.

A double-stranded DNA such as a genomic DNA isolated as described above or a PCR fragment, a single-stranded DNA such as a cDNA prepared using a reverse transcription reaction from a total RNA or an mRNA, and a hybrid double strand composed of DNA and RNA can be preferably used as a template nucleic acid in the present invention. The double-stranded DNA can be preferably used after denaturing it into single-stranded DNAs or without such denaturation.

Hereinafter, the present invention will be described in detail.

(1) The method for stabilization and long-term storage of a reaction reagent for a method for amplifying or detecting a target nucleic acid of the present invention

The reaction reagent according to the present invention can be used for a method for amplifying or detecting a target nucleic acid using at least one chimeric oligonucleotide primer, an endonuclease and a DNA polymerase.

The reaction reagent of the present invention can be used for a method for amplifying a target nucleic acid that can be carried out using two primers, i.e., a chimeric oligonucleotide primer that is complementary to a nucleic acid as a template and another chimeric oligonucleotide primer that is complementary to a displaced strand. One primer binds to a DNA strand as a template to cause a strand displacement reaction, whereas another primer binds to a displaced strand released as a result of the strand displacement reaction to initiate another strand displacement reaction. It is clear that a reaction product with one primer can function as a template for another primer if this aspect is used. Thus, the amount of amplification product increases in a non-linear manner as the amount of the template increases.

A reaction reagent for a method in which a double-stranded DNA as a template and two chimeric oligonucleotide primers are used exemplifies another aspect. In the method, although it varies depending on the reaction conditions, switching of templates may occur among the template-extended strand intermediates during the extension reactions from the primers to generate a double-stranded nucleic acid consisting of the synthesized primer-extended strands being annealed each other. The double-stranded nucleic acid has chimeric oligonucleotide primers at both ends. Then, reactions of extending complementary strands comprising strand displacement can be initiated from both of the ends again. As a result of the reactions, an amplification product having the primer sequence at one end is generated. Furthermore, if switching of templates occurs during the reactions, a double-stranded nucleic acid similar to one that described above is generated again.

The reaction reagent of the present invention can be used for a method for amplifying a nucleic acid that comprises a step of using a DNA polymerase having a strand displacement activity to effect a template switching reaction. In the template switching reaction in the presence of a double-stranded nucleic acid as a template, two chimeric oligonucleotide primers substantially complementary to the nucleotide sequences of the respective strands and a DNA polymerase having a strand displacement activity, two primer-extended strands complementary to the template are synthesized. Template switching of each of the primer-extended strands from the template to the other primer-extended strand takes place during the synthesis of the primer-extended strands.

As used herein, a template switching reaction refers to a reaction in which when complementary strands are synthesized by strand displacement reactions from the both sides of a double-stranded nucleic acid, a DNA polymerase switches the template and synthesizes a complementary strand thereafter using, as a template, the other complementary strand newly synthesized by another DNA polymerase. In other words, a template switching reaction refers to a reaction in which a double-stranded nucleic acid as a template is treated with primers and a DNA polymerase having a strand displacement activity to generate extended strands complementary to the template, wherein a DNA polymerase that synthesized the primer-extended strands actively switches the template from the original templates to the other primer-extended strands during the synthesis of the extended strands. The ability of the DNA polymerase to effect a template switching reaction can be determined, for example, according to the method as described in Referential Example 3 below, although it is not intended to limit the present invention.

A DNA polymerase capable of an effect the template switching reaction during strand displacement reaction can be preferably used for the present invention. For example, a variant enzyme of Bca DNA polymerase lacking a 5'→3' exonuclease activity is preferably used in particular. Such an enzyme is commercially available as BcaBEST DNA polymerase (Takara Shuzo). It can also be prepared from Escherichia coli HB101/pU1205 (FERM BP-3720) which contains the gene for the enzyme according to the method as described in Japanese Patent No. 2978001.

In the target nucleic acid amplification method using the reaction reagent of the present invention, a polymer in which the regions to be amplified are connected each other may be generated. The polymer has a structure in which plural regions to be amplified are repeated in the same direction. The polymers are observed upon electrophoretic analysis of amplification products as laddered bands. It is considered that the generation of such polymers is influenced by the region to be amplified, the size of the region, the flanking regions, the nucleotide sequence of the chimeric oligonucleotide primer to be used, the reaction conditions and the like.

The polymer as described above contains plural regions to be amplified. For example, the polymer is useful when detection of a nucleic acid containing a region to be amplified is intended because it hybridizes to a number of probes upon hybridization using an appropriate probe and generates a intensive signal. The region to be amplified or a portion thereof can be obtained from the polymer as a monomer by using digestion with a restriction enzyme or the like in combination.

One feature of the method for amplifying a nucleic acid of the present invention is that the method does not require adjusting the temperature up and down during the nucleic acid synthesis. Thus, the present invention provides a method for isothermally synthesizing a nucleic acid. Many of conventional nucleic acid amplification methods require adjusting the temperature up and down to dissociate a target from a synthesized strand. These methods require special reaction equipment such as a thermal cycler for this purpose. However, the method of the present invention can be conducted only using equipment that can keep a constant temperature. As described above, the method of the present invention can be conducted at a single temperature. Preferably, it is conducted by selecting the reaction temperature and the stringency level such that non-specific annealing of a primer is reduced and such that the primer specifically anneals to a nucleic acid as a template. Although it is not intended to limit the present invention, the method of the present invention can be conducted under high-temperature conditions by using a heat-resistant enzyme as described above. In addition, it is preferable to conduct the method of the present invention at an appropriate temperature for sufficiently retaining the activity of the enzyme used in order to maintain the reaction efficiency at high level. Thus, the reaction temperature is preferably about 20°C to about 80°C, more preferably about 30°C to about 75°C, most preferably about 50°C to about 70°C although it varies depending on the enzyme used. It is preferable to use a longer primer than that for a reaction at a normal temperature particularly if the reaction is conducted under high-temperature conditions. An example of effects brought by the elevated reaction temperature is the solution of a problem of forming secondary structure of a DNA as a template. The elevated reaction temperature enables amplification of a desired nucleic acid even if a nucleic acid having a high GC content is used as a template. Furthermore, it is similarly effective in amplifying a region of a long chain length. Such effect is observed in a range between about 60 bp and about 20 kbp, specifically between about 60 bp and about 1500 bp.

The amplification efficiency can be increased by adjusting the reaction temperature in accordance with the GC content of the nucleic acid as the template. For example, if a nucleic acid having a low GC content is used as a template, the amplification reaction of the present invention can be conducted at 50 to 55°C, although the temperature depends on the chain length to be amplified and the Tm value of the primer.

Use of a DNA polymerase having a reverse transcriptase activity (e.g., BcaBEST DNA polymerase) in the method of the present invention can make the amplification of a nucleic acid from an RNA, which comprises a step of preparing a cDNA from an RNA (a reverse transcription reaction), be conveniently conducted using only a single enzyme. Alternatively, a product obtained by independently conducting a step of preparing a cDNA from an RNA (i.e., a cDNA) can be used in the method of the present invention as the DNA as a template.

In each case, the reaction in the method of the present invention is repeated until it is terminated by appropriate means, for example, by inactivating the enzyme or by lowering the reaction temperature, or until the reaction is deprived of one of the substrates.

The method for amplifying a nucleic acid of the present invention can be used for various experimental procedures that utilize amplification of a nucleic acid including detection, labeling and sequencing of a nucleic acid.

Furthermore, the method for amplifying a nucleic acid of the present invention can be used for an in situ nucleic acid amplification method, a method for amplifying a nucleic acid on a solid substrate such as a DNA chip, or a multiplex nucleic acid amplification method in which plural regions are simultaneously amplified.

The utilization efficiency of the primer in the method for amplifying a nucleic acid of the present invention is about 100%, which may be 5- to 10-fold higher than that in a conventional method such as the PCR.

The nucleic acid amplification method of the present invention can be used to produce an amplification product with high fidelity to the nucleotide sequence of the template nucleic acid. When the frequency of error in the DNA synthesis in the method of the present invention was determined by analyzing the nucleotide sequences of resulting amplification products, the frequency of error found in amplification products observed for the method of the present invention was equivalent to that observed for the LA-PCR which is known to be able to amplify a nucleic acid with high fidelity. In other words, the method of the present invention has fidelity equivalent to that of the LA-PCR.

The method for detecting a target nucleic acid of the present invention can be conducted by amplifying the target nucleic acid directly from a sample containing the nucleic acid. In this case, the chain length of the target nucleic acid to be amplified is not limited to a specific one. For example, a region of 200 bp or shorter, preferably 150 bp or shorter is effective for sensitive detection of the target nucleic acid. The target nucleic acid in the sample can be detected with high sensitivity by designing the chimeric oligonucleotide primers of the present invention to result in the chain length to be amplified as described above.

In addition, a target nucleic acid can be detected with more sensitivity even from a trace amount of a nucleic acid sample in the detection method of the present invention by using a reaction buffer containing Bicine, Tricine, HEPES, phosphate or tris as a buffering component and an annealing solution containing spermidine or propylenediamine. In this case, the endonuclease and the DNA polymerase to be used are not limited to specific ones. For example, a combination of an RNase H from Escherichia coli, a bacterium of the genus Pyrococcus or a bacterium of the genus Archaeoglobus and BcaBEST DNA polymerase is preferable. It is considered that the preferable units of the endonuclease and the DNA polymerase may vary depending on the types the enzymes. In such a case, the composition of the buffer and the amount of the enzymes added may be adjusted using the increase in detection sensitivity or the amount of amplification product as an index.

In the detection method of the present invention, dUTP may be incorporated as a substrate during amplification of a target nucleic acid. Thus, if dUTP is used as a substrate, it is possible to prevent false positives due to amplification product carry-over contamination by degrading amplification products utilizing uracil N-glycosidase (UNG).

Known methods for detecting a nucleic acid can be used for detection of a target nucleic acid. Examples of such methods include detection of a reaction product having a specific size by electrophoresis, and detection by hybridization with a probe. Furthermore, a detection method in which magnetic beads are used in combination can be preferably used. Pyrophosphoric acid generated during the step of amplification of a target nucleic acid may be converted into a insoluble substance such as a magnesium salt, and then the turbidity may be measured. A fluorescent substance such as ethidium bromide is usually used in the detection by electrophoresis. The hybridization with a probe may be used in combination with the detection by electrophoresis. The probe may be labeled with a radioisotope or with a non-radioactive substance such as biotin or a fluorescent substance. Additionally, use of a labeled nucleotide in the detection step may facilitate the detection of an amplification product into which the labeled nucleotide is incorporated, or may enhance the signal for detection utilizing the label. A fluorescence polarization method, fluorescence resonance energy transfer (FRET) or the like can also be utilized for the detection. The target nucleic acid can be detected automatically or quantified by constructing a suitable detection system. In addition, detection with naked eyes by a hybrid chromatography method can be preferably used.

A ribonucleotide (RNA) probe, or a chimeric oligonucleotide probe composed of a ribonucleotide and a deoxyribonucleotide, labeled with two or more fluorescent substances positioned at a distance that results in a quenching state can be used in the detection method of the present invention. The probe does not emit fluorescence. When it is annealed to a DNA amplified from a target nucleic acid that is complementary to the probe, RNase H digests the probe. The distance between the fluorescent substances on the probe then increases, resulting in the emission of fluorescence. Thus, the emission reveals the presence of the target nucleic acid. If an RNase H is used in the method for amplifying a nucleic acid of the present invention, a target nucleic acid can be detected only by adding the probe to the reaction mixture. For example, a combination of 6-carboxyfluorescein (6-FAM) and N,N,N',N'-tetramethyl-6-carboxyrhodamine (TAMRA), which is a pair of labels for FRET, or a combination of 6-carboxyfluorescein (6-FAM) and 4-(4'-dimethylaminophenylazo)benzoic acid (DABCYL), which is a pair of labels for non-FRET, can be preferably used as fluorescent substances for labeling the probe.

The present invention further provides a probe used in the above-mentioned method for detecting a target nucleic acid. The probe of the present invention is not limited to specific one as long as it can hybridize to a target nucleic acid amplified using the nucleic acid amplification method of the present invention under normal hybridization conditions. In view of specific detection of an amplification product, a probe that hybridizes under conditions, for example, known to those skilled in the art as being stringent is preferable. The stringent hybridization conditions are described in, for example, T. Maniatis et al. (eds.), Molecular Cloning: A Laboratory Manual 2nd ed., 1989, Cold Spring Harbor Laboratory. Specifically, the stringent conditions are exemplified by the following: incubation at a temperature lower by about 25°C than the Tm of the probe to be used for 4 hours to overnight in 6 x SSC (1 x SSC: 0.15 M NaCl, 0.015 M sodium citrate, pH 7.0) containing 0.5% SDS, 5 x Denhardt's (0.1% bovine serum albumin (BSA), 0.1% polyvinylpyrrolidone, 0.1% Ficoll 400) and 100 µg/ml salmon sperm DNA. A probe having a label as described above may be used as the probe for facilitating the detection of the target nucleic acid.

The method for amplifying a nucleic acid under isothermal conditions of the present invention does not require the use of equipment such as a thermal cycler. The number of primers used in the amplification method of the present invention can be one or two, which is less than that used in a conventional method. Since reagents such as dNTPs used for PCR and the like can be applied to the method of the present invention, the running cost can be reduced as compared with a conventional method. Therefore, the method of the present invention can be preferably used, for example, in a field of genetic test in which the detection is routinely conducted. The method of the present invention provides a greater amount of an amplification product in a shorter time than the PCR. Therefore, the method of the present invention can be utilized as a convenient, rapid and sensitive method for detecting a gene.

As for the reaction reagent for preparing a reaction mixture used for the method of the present invention, the respective components contained in the reaction mixture can be stored separately. Alternatively, a premixed solution in which plural components are mixed together beforehand may be prepared for convenient operation. In case of such a reaction reagent, it is preferable to prepare a premixed solution to have such a composition that suppresses a reaction that may abolish the fundamental function as a primer of the chimeric oligonucleotide primer in the solution in view of stability of the reagent. Although it is not intended to limit the present invention, the premixed solution preferably has such a composition that does not result in polymerization and/or degradation of the chimeric oligonucleotide primer. In one embodiment of the method of the present invention, for example, it is preferable to exclude or reversibly inactivate at least one of the components of the premixed solution which are required for and involved in the nucleic acid synthesis reaction used in the method of the present invention. Although it is not intended to limit the present invention, for example, at least one selected from the group consisting of a DNA polymerase, a chimeric oligonucleotide primer, a magnesium salt and dNTPs may be excluded or inactivated. By the above-mentioned method, the reaction reagent used for the method of the present invention can be stabilized at any temperature. In addition, the enzyme(s) (the DNA polymerase and/or the RNase H) may be separated in order to suppress the inactivation of the enzyme(s).

Thus, the present invention encompasses a reaction reagent consisting of two premixed solutions, i.e., one containing a separated component (separated components) and another containing other components. Optionally, the reaction reagent may consists of three or more premixed solutions.

The above-mentioned stabilization method can be utilized as a method of long-term storage of the reaction reagent used for the method of the present invention. Although there is no specific limitation concerning the forms for long-term storage, for example, it is preferable to prepare the reaction reagent in a form consisting of two parts, i.e., a solution of the chimeric oligonucleotide primer and a reaction buffer containing the enzyme(s), the magnesium salt and the dNTPs. In one embodiment of the long-term storage method of the present invention, the reaction buffer preferably contains a salt. As used herein, a salt concentration means a concentration of salts including a buffering component, a magnesium salt and a salt for adjusting ion strength (e.g., potassium acetate). The concentration of the contained salt can be appropriately adjusted depending on the types of the DNA polymerase and the RNase H to be used. Preferably, the salt concentration is adjusted such that it results in ion strength that stabilizes the enzyme(s). In this case, the salt concentration can be optimized using the method as described in Example 4. Although it is not intended to limit the present invention, for example, the concentration of the salt contained in the reaction buffer containing the enzyme(s) is around the final salt concentration upon reaction, for example, 5 times or less, preferably 3 times or less, more preferably 1.5 times or less the final salt concentration upon reaction. The concentration may be equal to or lower than the final salt concentration upon reaction.

Although it is not intended to limit the present invention, for example, in case of a reaction system in which Bca DNA polymerase and an RNase H from a bacterium of the genus Archaeoglobus are used, the concentration of HEPES-potassium hydroxide buffer may be more than 0 mM and up to 160 mM, preferably within a range from 30 mM to 120 mM, more preferably within a range from 32 mM to 102 mM. If magnesium acetate is to be used as a magnesium salt, the concentration may be more than 0 mM and up to 20 mM, preferably within a range from 3 mM to 15 mM, more preferably within a range from 4 mM to 13 mM.

If potassium acetate is to be used for adjusting ion strength, the concentration may be more than 0 mM and up to 500 mM, preferably within a range from 90 mM to 360 mM, more preferably within a range from 100 mM to 317 mM. Optionally, a salt concentration adjustment reagent which is used for adjusting the salt concentration may be included separately.

A chimeric oligonucleotide primer solution may contain a salt for adjusting the salt concentration of a reaction mixture. In this case, a reaction mixture can be prepared by using two premixed solutions.

If a reagent for a gene amplification reaction is to be prepared, a primer is usually dissolved in sterile water of a low-salt solution (e.g., TE buffer), and a necessary amount thereof is subjected to preparation of a reaction mixture (see Molecular Cloning, 2nd ed., 14.18).

According to the present invention, the salt concentration of a premixed solution containing the enzyme(s) can be made suitable for stabilization of the enzyme(s) by including, in the primer solution, a salt which is a component of the reaction mixture.

In another embodiment of the long-term storage method of the present invention, it the reaction reagent is in a form consisting of two separate parts (i.e., a solution of the chimeric oligonucleotide primer and a reaction buffer containing the enzymes, the magnesium salt and the dNTPs), it is preferable to elevate the enzyme concentrations of the reaction buffer. The concentrations of the contained enzymes can be appropriately adjusted depending on the types of the DNA polymerase and the RNase H to be used. In this case, the concentrations can be optimized using the method as described in Example 4. Although it is not intended to limit the present invention, for example, in case of a reaction system in which Bca DNA polymerase and an RNase H from a bacterium of the genus Archaeoglobus or the genus Thermococcus are used, the concentrations of the enzymes contained in a reaction buffer are preferably elevated so long as the contained enzymes are not inactivated.

In an embodiment of a reaction reagent with which a primer can be readily changed depending on the nucleic acid as the template, a solution for dissolving a primer which does not contain a primer may be used in place of the primer solution.

In another embodiment, the dNTPs rather than the chimeric oligonucleotide primer may be separated from other components, and salt concentrations of two premixed solutions may be determined taking the stabilities of the enzymes into consideration.

As for the relationship between the enzyme concentration(s) and the salt concentration of the enzyme-containing premixed solution according to the present invention, it is preferable to set the concentration rate of the enzyme at above 1 while maintaining the concentration rate of the salt at about 1, defining the final concentration of the enzyme or the salt upon reaction as 1.

Preferably, the ratio of the concentration rate of the enzyme (E) / the concentration rate of the salt (S) is above 1. The ratio is preferably $100 \geq E/S > 1$, more preferably $10 \geq E/S > 1$, most preferably $5 \geq E/S > 1$.

An antiseptic agent or an antifungal agent may be added to a reaction reagent according to the method of the present invention. A commercially available antiseptic or antifungal agent can be utilized. Examples thereof that can be preferably used include, but are not limited to, sodium azide, paraoxybenzoic acid as well as derivatives and salts thereof, Thimerosal and ProClin. Naturally, the concentration of the antiseptic agent or the antifungal agent is determined such that the enzyme contained in the reaction reagent is not inactivated during storage or reaction.

A reaction reagent can be stored for about one month or longer according to the long-term storage method of the present invention. In this case, although there is no specific limitation concerning the storage temperature, for example, the temperature is 50°C or below, preferably 30°C or below.

(2) Primer used for the method for detecting a pathogenic microorganism and/or a virus of the present invention

A target nucleic acid in a sample can be detected using a nucleic acid amplification method of the present invention. The method comprises:

(a) preparing a reaction mixture by mixing a nucleic acid as a template, a deoxyribonucleotide triphosphate, a DNA polymerase having a strand displacement activity, at least one primer and an RNase H, wherein the primer is a chimeric oligonucleotide primer that is substantially complementary to the nucleotide sequence of the nucleic acid as the template and contains a ribonucleotide as well as at least one selected from the group consisting of a deoxyribonucleotide and a nucleotide analog, the ribonucleotide being positioned at the 3'-terminus or on the 3'-terminal side of the primer;
(b) amplifying a target nucleic acid by incubating the reaction mixture for a sufficient time to generate a reaction product; and
(c) detecting the target nucleic acid amplified in step (b).

If an RNA is used as a template in step (a) above, the reverse transcription reaction and the nucleic acid amplification reaction may be conducted in one step. Although it is not intended to limit the present invention, for example, a combination of AMV RTase, MMLV RTase or RAV-2 RTase and Bca DNA polymerase can be preferably used as a combination of a reverse transcriptase and a strand displacement-type DNA polymerase.

The method for detecting a target nucleic acid of the present invention can be used to distinguish difference in a nucleotide sequence of the target nucleic acid. In this aspect, the chimeric oligonucleotide primer to be used is designed such that the 3'-terminal portion of the primer is positioned close to the specific base of the target nucleotide sequence to be distinguished. For example, it is designed such that a hydrogen bond is formed between the base and the 3'-terminal base of the primer. If a mismatch exists between the nucleotide sequence of the 3'-terminal portion of the primer and the nucleotide sequence of the template, amplification from the target nucleic acid does not take place and no amplification product is generated using the above-mentioned chimeric oligonucleotide primer for an amplification reaction. Information concerning a specific base in a gene such as a point mutation or a single nucleotide polymorphism (SNP) can be obtained using the method.

Although it is not intended to limit the present invention, for example, a primer for detecting a pathogenic microorganism and/or a virus selected from the group consisting of the following can be preferably used for the method for detecting a target nucleic acid of the present invention:

(1) a chimeric oligonucleotide primer for detecting Mycobacterium tuberculosis having a nucleotide sequence of one selected from the group consisting of SEQ ID NOS:7, 8, 21, 22, 162, 163, 170 and 171;
(2) a chimeric oligonucleotide primer for detecting HCV having a nucleotide sequence of one selected from the group consisting of SEQ ID NOS:15, 16, 81-86 and 88-91;
(3) a chimeric oligonucleotide primer for detecting a chlamydia having a nucleotide sequence of one selected from the group consisting of SEQ ID NOS:17-20, 121-127, 130-135, 166 and 167;
(4) a chimeric oligonucleotide primer for detecting a Mycobacterium avium complex having a nucleotide sequence of one selected from the group consisting of SEQ ID NOS:25-31;
(5) a chimeric oligonucleotide primer for detecting a gonococcus having a nucleotide sequence of one selected from the group consisting of SEQ ID NOS:38-63, 164 and 165;
(6) a chimeric oligonucleotide primer for detecting HBV having a nucleotide sequence of one selected from the group consisting of SEQ ID NOS:71-78;
(7) a chimeric oligonucleotide primer for detecting HIV having a nucleotide sequence of one selected from the group consisting of SEQ ID NOS:95-103;
(8) a chimeric oligonucleotide primer for detecting Staphylococcus aureus having a nucleotide sequence of one selected from the group consisting of SEQ ID NOS:108-117;
(9) a chimeric oligonucleotide primer for detecting a mycoplasma having a nucleotide sequence of one selected from the group consisting of SEQ ID NOS:139-146; and
(10) a chimeric oligonucleotide primer for detecting MRSA having a nucleotide sequence of one selected from the group consisting of SEQ ID NOS:152-155.

A probe for detecting a pathogenic microorganism and/or a virus selected from the group consisting of the following can be preferably used for the detection method of the present invention:

(1) a probe for detecting Mycobacterium tuberculosis selected from a region containing a nucleotide sequence of one selected from the group consisting of SEQ ID NOS:11, 12 and 172;
(2) a probe for detecting HCV selected from a region containing a nucleotide sequence of one selected from the group consisting of SEQ ID NOS:87 and 92;
(3) a probe for detecting a chlamydia selected from a region containing a nucleotide sequence of one selected from the group consisting of SEQ ID NOS:128, 136 and 168;
(4) a probe for detecting a Mycobacterium avium complex selected from a region containing a nucleotide sequence of one selected from the group consisting of SEQ ID NOS:32 and 33;
(5) a probe for detecting a gonococcus selected from a region containing a nucleotide sequence of one selected from the group consisting of SEQ ID NOS:64-68;
(6) a probe for detecting HBV selected from a region containing a nucleotide sequence of one selected from the group consisting of SEQ ID NOS:79 and 80;
(7) a probe for detecting HIV selected from a region containing a nucleotide sequence of one selected from the group consisting of SEQ ID NOS:104 and 105;
(8) a probe for detecting Staphylococcus aureus selected from a region containing a nucleotide sequence of one selected from the group consisting of SEQ ID NOS:118 and 119;
(9) a probe for detecting a mycoplasma selected from a region containing a nucleotide sequence of one selected from the group consisting of SEQ ID NOS:147-149; and
(10) a probe for detecting MRSA selected from a region containing a nucleotide sequence of one selected from the group consisting of SEQ ID NOS:156 and 157.

(3) Kit of the present invention

The kit of the present invention contains the chimeric oligonucleotide primer and/or the probe as described in (2) above. The present invention provides a kit used for the method for amplifying a nucleic acid or the method for detecting a nucleic acid of the present invention. In one embodiment, the kit is in a packaged form and contains instructions regarding the use of a DNA polymerase and an endonuclease in a strand displacement reaction. A kit that contains a DNA polymerase having a strand displacement activity, an endonuclease, and a buffer for a strand displacement reaction is preferably used for the method of the present invention. Alternatively, a commercially available DNA polymerase having a strand displacement activity and/or endonuclease may be selected and used according to the instructions. Additionally, the kit may contain a reagent for a reverse transcription reaction that is used when an RNA is used as a template. The DNA polymerase can be selected from the DNA polymerases to be used in the present invention as described above. The endonuclease can be selected from the endonucleases as described above. The buffer for strand displacement reaction may contain a reaction buffer containing Bicine, Tricine, HEPES, phosphate or tris as a buffering component and an annealing solution. Furthermore, the kit may contain a modified deoxyribonucleotide or a deoxynucleotide triphosphate analog.

"Instructions" are printed matters describing a method of using the kit, e.g., a method for preparing a reagent solution for a strand displacement reaction, recommended reaction conditions and the like. The instructions include an instruction manual in a form of a pamphlet or a leaflet, a label stuck to the kit, and description on the surface of the package containing the kit. The instructions also include information disclosed or provided through electronic media such as the Internet.

The kit used for the method for detecting a target nucleic acid may contain, in addition to the instructions and the reagent for amplification reaction, a chimeric oligonucleotide primer suitable for amplification of the target nucleic acid or a reagent for the detection of the amplified target nucleic acid (e.g., a probe). A kit containing, as a component, a reaction reagent constructed according to the method for stabilization or long-term storage of a reaction reagent of the present invention can be preferably used. Furthermore, the kit of the present invention may contain a nucleic acid that serves as an internal control (I. C.) for judging false negative. Although it is not intended to limit the present invention, an exemplary kit contains chimeric oligonucleotide primers having nucleotide sequences of SEQ ID NOS: 170 and 171, a probe for detecting Mycobacterium tuberculosis having a nucleotide sequence of SEQ ID.NO:172, a plasmid containing the nucleotide sequence of SEQ ID NO:169 as an internal control, and a probe for detecting the internal control having a nucleotide sequence of SEQ ID NO:173. Another exemplary kit for detecting a pathogenic microorganism and/or a virus may contain an internal control similarly.

## Examples

The following Examples illustrate the present invention in more detail, but are not to be construed to limit the scope thereof.

### Referential Example 1

(1) A unit value of a heat-resistant RNase H used for the method of the present invention was calculated as follows.

1 mg of poly(rA) or poly(dT) (both from Amersham Pharmacia Biotech) was dissolved in 1 ml of 40 mM tris-HCl (pH 7.7) containing 1 mM EDTA to prepare a poly(rA) solution and a poly(dT) solution.

The poly(rA) solution (to a final concentration of 20 μg/ml) and the poly(dT) solution (to a final concentration of 30 μg/ml) were then added to 40 mM tris-HCl (pH 7.7) containing 4 mM $MgCl_2$, 1 mM DTT, 0.003% BSA and 4% glycerol. The mixture was reacted at 37°C for 10 minutes and then cooled to 4°C at prepare a poly(rA)-poly(dT) solution. 1 μl of an appropriately diluted enzyme solution was added to 100 μl of the poly(rA)-poly(dT) solution. The mixture was reacted at 40°C for 10 minutes. 10 μl of 0.5 M EDTA was added thereto to terminate the reaction. Absorbance at 260 nm was then measured. As a control, 10 μl of 0.5 M EDTA was added to the reaction mixture, the resulting mixture was reacted at 40°C for 10 minutes, and the absorbance was then measured. A value (difference in absorbance) was obtained by subtracting the absorbance for the control from the absorbance for the reaction in the absence of EDTA. Thus, the concentration of nucleotide released from the poly(rA)-poly(dT) hybrid by the enzymatic reaction was determined on the basis of the difference in absorbance.

Unit = [Difference in Absorbance x Reaction

Volume (ml)] / 0.0152 x (110 / 100) x Dilution Rate

Referential Example 2: Preparation of RNase H

An RNase H used according to the present invention was prepared according to the method as described in WO 02/22831. Specifically, Escherichia coli recombinant cells were cultured and an RNase H of interest was prepared from the cells as follows.

(1) Polypeptide having RNase H activity derived from Pyrococcus furiosus

Escherichia coli JM109 transformed with a plasmid pPFU220 which contains a DNA encoding a polypeptide having an RNase H activity derived from Pyrococcus furiosus is designated and indicated as Escherichia coli JM109/pPFU220, and deposited on September 5, 2000 (date of original deposit) at International Patent Organism Depositary, National Institute of Advanced Industrial Science and Technology, AIST Tsukuba Central 6, 1-1, Higashi 1-chome, Tsukuba-shi, Ibaraki 305-8566, Japan under accession number FERM BP-7654. Escherichia coli JM109 transformed with pPFU220 was inoculated into 2 L of LB medium containing 100 μg/ml of ampicillin and cultured with shaking at 37°C for 16 hours. After cultivation, cells collected by centrifugation were suspended in 66.0 ml of a sonication buffer [50 mM tris-HCl (pH 8.0), 1 mM EDTA, 2 mM phenylmethanesulfonyl fluoride] and sonicated. A supernatant obtained by centrifuging the sonicated suspension at 12000 rpm for 10 minutes was heated at 60°C for 15 minutes. It was then centrifuged at 12000 rpm for 10 minutes again to collect a supernatant. Thus, 61.5 ml of a heated supernatant was obtained.

The heated supernatant was subjected to RESOURSE Q column (Amersham Pharmacia Biotech) equilibrated with Buffer A [50 mM tris-HCl (pH 8.0), 1 mM EDTA] and chromatographed using FPLC system (Amersham Pharmacia Biotech). As a result, RNase HII flowed through the RESOURSE Q column.

60.0 ml of the flow-through RNase HII fraction was subjected to RESOURSE S column (Amersham Pharmacia Biotech) equilibrated with Buffer A and eluted with a linear gradient of 0 to 500 mM NaCl using FPLC system. A fraction containing RNase HII eluted with about 150 mM NaCl was obtained. 2.0 ml of the RNase HII fraction was concentrated by ultrafiltration using Centricon-10 (Amicon). 250 μl of the concentrate was subjected to Superdex 200 gel filtration column (Amersham Pharmacia Biotech) equilibrated with 50 mM tris-HCl (pH 8.0) containing 100 mM NaCl and 0.1 mM EDTA and eluted with the same buffer. As a result, RNase HII was eluted at a position corresponding to a molecular weight of 17 kilodalton. The enzymatic activity of the thus obtained preparation was measured as described in Referential Example 1. As a result, an RNase H activity was observed for the preparation.

(2) Polypeptide having RNase H activity derived from Pyrococcus horikoshii

Escherichia coli JM109 transformed with a plasmid pPHO238 which contains a DNA encoding a polypeptide having an RNase H activity derived from Pyrococcus horikoshii is designated and indicated as Escherichia coli JM109/pPHO238, and deposited on February 22, 2001 (date of original deposit) at International Patent Organism Depositary, National Institute of Advanced Industrial Science and Technology, AIST Tsukuba Central 6, 1-1, Higashi 1-chome, Tsukuba-shi, Ibaraki 305-8566, Japan under accession number FERM BP-7692. Escherichia coli JM109 transformed with pPHO238 was inoculated into 1 L of LB medium containing 100 μg/ml of ampicillin and cultured with shaking at 37°C for 16 hours. After cultivation, cells collected by centrifugation were suspended in 34.3 ml of a sonication buffer [50 mM tris-HCl (pH 8.0), 1 mM EDTA, 2 mM phenylmethanesulfonyl fluoride] and sonicated. A supernatant obtained by centrifuging the sonicated suspension at 12000 rpm for 10 minutes was heated at 80°C for 15 minutes. It was then centrifuged at 12000 rpm for 10 minutes again to collect a supernatant. Thus, 33.5 ml of a heated supernatant was obtained.

The heated supernatant was subjected to RESOURSE Q column (Amersham Pharmacia Biotech) equilibrated with Buffer A [50 mM tris-HCl (pH 8.0), 1 mM EDTA] and chromatographed using FPLC system (Amersham Pharmacia Biotech). As a result, RNase HII flowed through the RESOURSE Q column.

35.0 ml of the flow-through RNase HII fraction was dialyzed against 2 L of Buffer B (50 mM tris-HCl (pH 7.0), 1 mM EDTA) for 2 hours. The dialysis was repeated two more times. 34.5 ml of the dialyzed enzyme solution was subjected to RESOURSE S column (Amersham Pharmacia Biotech) equilibrated with Buffer B and eluted with a linear gradient of 0 to 500 mM NaCl using FPLC system. A fraction containing RNase HII eluted with about 155 mM NaCl was obtained.

Buffer B was added to 4.0 ml of the fraction to make the NaCl concentration to 50 mM. The mixture was subjected to HiTrap-heparin column (Amersham Pharmacia Biotech) equilibrated with Buffer B containing 50 mM NaCl and eluted with a linear gradient of 50 to 550 mM NaCl using FPLC system. As a result, a fraction containing RNase HII eluted with about 160 mM NaCl was obtained.

6.9 ml of the RNase HII fraction was concentrated by ultrafiltration using Centricon-10 (Amicon). 250 µl of the concentrate was divided into two portions and subjected to Superose 6 gel filtration column (Amersham Pharmacia Biotech) equilibrated with 50 mM tris-HCl (pH 7.0) containing 100 mM NaCl and 0.1 mM EDTA and eluted with the same buffer. As a result, RNase HII was eluted at a position corresponding to a molecular weight of 24.5 kilodalton. This molecular weight corresponds to that of the RNase HII in the monomeric form. The enzymatic activity of the thus obtained preparation was measured as described in Referential Example 1. As a result, an RNase H activity was observed for the preparation.

(3) Polypeptide having RNase H activity derived from Archaeoglobus fulgidus

Escherichia coli JM109 transformed with a plasmid pAFU204 which contains a DNA encoding a polypeptide having an RNase H activity derived from Archaeoglobus fulgidus is designated and indicated as Escherichia coli JM109/pAFU204, and deposited on February 22, 2001 (date of original deposit) at International Patent Organism Depositary, National Institute of Advanced Industrial Science and Technology, AIST Tsukuba Central 6, 1-1, Higashi 1-chome, Tsukuba-shi, Ibaraki 305-8566, Japan under accession number FERM BP-7691. Escherichia coli JM109 transformed with pAFU204 was inoculated into 2 L of LB medium containing 100 µg/ml of ampicillin and cultured with shaking at 37°C for 16 hours. After cultivation, cells collected by centrifugation were suspended in 37.1 ml of a sonication buffer [50 mM tris-HCl (pH 8.0), 1 mM EDTA, 2 mM phenylmethanesulfonyl fluoride] and sonicated. A supernatant obtained by centrifuging the sonicated suspension at 12000 rpm for 10 minutes was heated at 70 °C for 15 minutes. It was then centrifuged at 12000 rpm for 10 minutes again to collect a supernatant. Thus, 40.3 ml of a heated supernatant was obtained.

The heated supernatant was subjected to RESOURSE Q column (Amersham Pharmacia Biotech) equilibrated with Buffer A [50 mM tris-HCl (pH 8.0), 1 mM EDTA] and chromatographed using FPLC system (Amersham Pharmacia Biotech). As a result, RNase HII flowed through the RESOURSE Q column.

The flow-through RNase HII fraction was subjected to RESOURSE S column (Amersham Pharmacia Biotech) equilibrated with Buffer A and chromatographed using FPLC system (Amersham Pharmacia Biotech). As a result, RNase HII flowed through the RESOURSE S column.

40.0 ml of the flow-through RNase HII fraction was dialyzed against 2 L of Buffer B (50 mM tris-HCl (pH 7.0), 1 mM EDTA) containing 50 mM NaCl for 2 hours: The dialysis was repeated two more times. 40.2 ml of the dialyzed enzyme solution was subjected to HiTrap-heparin column (Amersham Pharmacia Bi-otech) equilibrated with Buffer B containing 50 mM NaCl and eluted with a linear gradient of 50 to 550 mM NaCl using FPLC system. As a result, a fraction containing RNase HII eluted with about 240 mM NaCl was obtained.

7.8 ml of the RNase HII fraction was concentrated by ultrafiltration using Centricon-10 (Amicon). About 600 µl of the concentrate was divided into four portions and subjected to Superose 6 gel filtration column (Amersham Pharmacia Biotech) equilibrated with 50 mM tris-HCl (pH 7.0) containing 100 mM NaCl and 0.1 mM EDTA and eluted with the same buffer. As a result, RNase HII was eluted at a position corresponding to a molecular weight of 30.0 kilodalton. This molecular weight corresponds to that of the RNase HII in the monomeric form. The enzymatic activity of the thus obtained preparation was measured as described in Referential Example 1. As a result, an RNase H activity was observed for the preparation.

(4) Escherichia coli HMS174(DE3) transformed with a plasmid pTLI204 which contains a DNA encoding a polypeptide having an RNase H activity derived from Thermococcus litoralis is designated and indicated as Escherichia coli HMS174(DE3)/pTLI204, and deposited on February 22, 2001 (date of original deposit) at International Patent Organism Depositary, National Institute of Advanced Industrial Science and Technology, AIST Tsukuba Central 6, 1-1, Higashi 1-chome, Tsukuba-shi, Ibaraki 305-8566, Japan under accession number FERM BP-7693. Escherichia coli HMS174(DE3) transformed with pTLI204 was inoculated into 10 ml of LB medium containing 100 µg/ml of ampicillin and cultured with shaking at 37°C overnight. After cultivation, cells collected by centrifugation were processed as described above to obtain a heated supernatant. The enzymatic activity of the thus obtained heated supernatant was measured as described in Referential Example 1. As a result, an RNase H activity was observed for the heated supernatant.

(5) Polypeptide having RNase H activity derived from Thermococcus celer

Escherichia coli HMS174(DE3) transformed with a plasmid pTCE207 which contains a DNA encoding a polypeptide having an RNase H activity derived from Thermococcus celer is designated and indicated as Escherichia coli HMS174(DE3)/pTCE207, and deposited on February 22, 2001 (date of original deposit) at International Patent Organism Depositary, National Institute of Advanced Industrial Science and Technology, AIST Tsukuba Central 6, 1-1, Higashi 1-chome, Tsukuba-shi, Ibaraki 305-8566, Japan under accession number FERM BP-7694. Escherichia coli HMS174(DE3) transformed with pTCE207 was cultured, and the resulting cells were subjected to purification to obtain a heated supernatant as described in (4). The enzymatic activity of the thus obtained heated supernatant was measured as described in (4) above. As a result, an RNase H activity was observed for the heated supernatant.

Referential Example 3

The amplification method of the present invention was examined.

(1) A PCR was carried out using pUC19 upper 150 PCR primer (SEQ ID NO:1) and pUC19 lower PCR primer (SEQ ID NO:2), as well as 100 pg of pUC19 plasmid DNA as a template. The resulting amplified fragment was purified using Microcon-100, blunt-ended using DNA blunting kit (Takara Shuzo) and subcloned into a HincII site of the plasmid pUC19. The plasmid with the amplified fragment being inserted was used to transform Escherichia coli JM109. The transformant was cultured. The plasmid having the inserted DNA, pUC19-150, was purified from the cells using QIAGEN plasmid mini kit (Qiagen). A PCR was carried out using the plasmid having the inserted DNA as a template as well as primers MCS-F (SEQ ID NO:3) and MCS-R (SEQ ID NO:4). A 534-bp PCR amplified fragment was obtained by purifying the reaction mixture using Microcon-100 (Millipore). A reaction mixture containing 15 ng of the PCR fragment, 30 pmol of a primer MR2 (SEQ ID NO:5) labeled with [$\gamma$-$^{32}$P]ATP by phosphorylation at the 5' end and sterile distilled water to 5 µl, and a reaction mixture further containing 30 pmol of a primer MR1 (SEQ ID NO:6) were prepared. The reaction mixtures were heat-denatured at 98°C for 2 minutes and then cooled to 55°C. 20 µl of a reaction mixture (42.5 mM Tricine buffer (pH 8.7), 12.5 mM potassium chloride, 12.5 mM ammonium sulfate, 0.0125% BSA, 1.25% DMSO, 5 mM magnesium acetate, 0.625 mM each of dNTPs) containing 1 U of BcaBEST DNA polymerase was added to each reaction mixture. The resulting mixtures were reacted at 55°C for 15 minutes. After reaction, 2.5 µl of a reaction termination solution (95% formamide, 20 mM EDTA, 0.05% Bromophenol Blue, 0.5% xylene cyanol) was added to 5 µl of each reaction mixture. The mixtures were heat-denatured at 94°C for 3 minutes. 1.6 µl each of the reaction mixtures was subjected to electrophoresis on 6% polyacrylamide gel containing 8 M urea and the signals were read using BAS2000 (Fujix) to detect products extended from the primer MR1. The results are shown in Figure 1A. The sequence ladder in Figure 1A was prepared by sequencing M13mp18 single strand DNA (Takara Shuzo) using the primer MF2 labeled with [$\gamma$-$^{32}$P]ATP by phosphorylation and used for the determination of the length of the extension product. Lane 1: a combination of the primers MF2 and MR1; and lane 2: MR1.

As shown in Figure 1A, a 448-bp band extended from the primer MR1 to the end of the template was detected when the extension reaction was carried out by adding only the primer MR1 to the template. On the other hand, in addition to the above-mentioned band, a 373-bp band bounded by the primers MR1 and MF2 was detected by further adding the primer MF2. Thus, it was confirmed that the extension from the MR1 primer using the PCR amplified fragment as a template by the action of BcaBEST DNA polymerase was switched due to template switching to the extension using a strand extended from the primer MF2 as a template. Furthermore, template switching was observed when Klenow DNA polymerase was used as a mesophilic DNA polymerase having a strand displacement activity under similar conditions. On the other hand, the template switching was not observed using TaKaRa Taq DNA polymerase (Takara Shuzo) or PyroBEST DNA polymerase (Takara Shuzo) which does not have a strand displacement activity.

(2) The template switching reaction was examined using a template DNA strand with a primer being annealed thereto. DNA fragments to which the primers MF2 and MR1 could be annealed were prepared as follows. PCRs were carried out using the plasmid pUC19 as a template and primers MCSF and RV (Takara Shuzo) or primers M4 (Takara Shuzo) and MCSR. The reaction mixtures were purified using Microcon-100 to obtain PCR amplified fragments MSCF-RV (236 bp) and M4-MCSR (271 bp). A region bounded by the primers M4 and RV was commonly present in the two PCR amplified fragments.

Next, a template-primer (2)-1 in which template DNA strands with primers being annealed thereto were not annealed each other, and a template-primer (2)-2 in which template DNA strands with primers being annealed thereto were annealed each other were prepared as follows.

(2)-1

A reaction mixture containing 30 ng of the fragment MCSF-RV, 40 pmol of the primer MF2 labeled with [$\gamma$-$^{32}$P]ATP by phosphorylation at the 5' end, propylenediamine at a final concentration of 0.01% and sterile distilled water to 5 µl, and a reaction mixture containing 30 ng of the fragment M4-MCSR, 40 pmol of the primer MR1, propylenediamine at a final concentration of 0.01% and sterile distilled water to 5 µl were separately heat-denatured at 98°C for 2 minutes and then cooled to 55°C. 2.5 µl each of the reaction mixtures were mixed together to prepare a template-primer.

(2)-2

A reaction mixture containing 15 ng of the fragment MCSF-RV, 15 ng of the fragment M4-MCSR, 20 pmol of the primer MF2 labeled with [$\gamma$-$^{32}$P]ATP by phosphorylation at the 5' end, 20 pmol of the primer MR1, propylenediamine at a final concentration of 0.01% and sterile distilled water to 5 µl was heat-denatured at 98°C for 2 minutes and then cooled to 55°C to prepare a template-primer.

20 µl of a reaction mixture (42.5 mM Tricine buffer (pH 8.7), 12.5 mM potassium chloride, 12.5 mM ammonium sulfate, 0.0125% BSA, 1.25% DMSO, 5 mM magnesium acetate, 0.625 mM each of dNTPs) containing 1 U of BcaBEST DNA polymerase was added to 5 µl of each template-primer reaction mixture. The resulting mixtures were reacted at 55°C for 15 minutes. After reaction, 2.5 µl of a reaction termination solution (95% formamide, 20 mM EDTA, 0.05% Bromophenol Blue, 0.5% xylene cyanol) was added to 5 µl of each reaction mixture. The mixtures were heat-denatured at 94°C for 3 minutes. 1.6 µl each of the reaction mixtures was subjected to electrophoresis on 6% polyacrylamide gel containing 8 M urea and the signals were read using BAS2000 (Fujix) to detect products extended from the primer MF2. The results are shown in Figure 1B. The sequence ladder in Figure 1B was prepared by sequencing M13mp18 single strand DNA using the primer MR1 labeled with [$\gamma$-$^{32}$P]ATP by phosphorylation and used for the determination of the length of the extension product. Lane 1: template DNA strands not being annealed each other; and lane 2: template DNA strands being annealed each other.

As shown in Figure 1B, only a 161-bp band extended from the primer MF2 to the end of the template was detected for the template-primer in which template DNA strands with primers being annealed thereto were not annealed each other. On the other hand, in addition to the above-mentioned band, a 223-bp band bounded by the primers MF2 and MR1 was detected for the template-primer in which template DNA strands with primers being annealed thereto were annealed each other. Thus, it was confirmed that a template switching reaction took place if template DNA strands with primers being annealed thereto were annealed each other.

Example 1

The detection method of the present invention was examined using Mycobacterium tuberculosis as a subject. Primers K-F-1033-2 (SEQ ID NO:7) and K-F-1133-2 (SEQ ID NO:8) for amplifying a region with relatively low GC content in the Mycobacterium tuberculosis genome were synthesized on the basis of the nucleotide sequence of the Mycobacterium tuberculosis genome registered in GenBank under accession number AL123456. The length of the region bordered by the primer pair including the primer portions is 105 bp. A Mycobacterium tuberculosis genomic DNA as a template was extracted from dried BCG vaccine (Nippon BCG Seizo) according to a conventional method. Serial dilutions containing 100 fg to 10 pg of the genomic DNA in 1 µl of sterile water were prepared. Reactions were carried out as follows. Briefly, reaction mixtures of final volumes of 25 µl containing the following at final concentrations were prepared: 32 mM HEPES-potassium hydroxide buffer (pH 7.8); 100 mM potassium acetate; 1% DMSO; 0.01% BSA; 4 mM magnesium acetate; 500 µM each of dNTPs; 50 pmol each of the primers K-F-1033-2 and K-F-1133-2; 9.375 U of Pfu RNase HII, 4.375 U of Afu RNase HII or 4 U of Tli RNase H; 2.75 U of BcaBEST DNA polymerase; 1 µl of one of the templates; and sterile water. The reaction mixtures were placed in Thermal Cycler Personal which had been set at 62°C and incubated for 60 minutes. After reaction, 3 µl each of the reaction mixtures was subjected to electrophoresis on 3.0% agarose gel. As a result, it was confirmed that an amplification product was observed using each RNase HII and each amount (100 fg to 10 pg) of the genomic DNA as a template.

Example 2

(1) A method for detecting a target nucleic acid using an RNA probe was examined. Mycobacterium tuberculosis was selected as a subject. 1 ng or 100 pg of the BCG genomic DNA as a template prepared in Example 1 was added to 50 µl of a reaction mixture. Primers MTIS2F (SEQ ID NO:162) and MTIS2R (SEQ ID NO:163) as well as RNA probes for detection MTIS (SEQ ID NO:11) and MTIS-2 (SEQ ID NO:12) were synthesized using a DNA synthesizer. Each probe had fluorescence labels 6-FAM (Glen Research) and TAMRA (Glen Research) at the 5' and 3' ends, respectively. The reactions were carried out as described in Example 1 except that 4 U of BcaBEST DNA polymerase and 18.75 U of Pfu RNase HII were used. 5 pmol of the RNA probe was added to each reaction mixture (a final volume of 50 µl). 25 µl out of 50 µl each of the reaction mixtures was used for an ICAN reaction at 58°C. Smart Cycler (Takara Shuzo) was used for the ICAN reactions and detection of the amplification products. The results are shown in Figure 2A. In Figure 2A, B and C, the longitudinal axes represent the fluorescence intensity and the horizontal axes represent the time. As shown in Figure 2A, only the amplified fragments of interest could be monitored using 1 ng or 100 pg of the template DNA by analysis using Smart Cycler. Furthermore, it was confirmed that both RNA probes could be preferably used for real-time detection.

(2) A one-step RT-ICAN detection system using an intercalator was examined. An HCV genome was selected as a subject. An RNA as a template was prepared as follows. An RNA sample was prepared from 300 µl of a serum from a patient with hepatitis C after obtaining informed consent using TRIzol reagent (Life Technologies) according to the instructions attached to the reagent and finally dissolved in 20 µl of injectable water (Otsuka Pharmaceutical). The RNA sample was used as a template for an RT-PCR. The reaction was carried out as follows. 50 μl of a reaction mixture was prepared using 2 μl of the RNA sample, 20 pmol each of primers SP6-HCV-F (SEQ ID NO:13) and T7-HCV-R (SEQ ID NO:14) and One-Step RNA PCR kit (Takara Shuzo) according to the manual attached to the kit. The reaction mixture was placed in Thermal Cycler Personal, reacted at 50°C for 15 minutes and at 94°C for 2 minutes, and subjected to 40 cycles of reactions as follows: 94°C for 30 seconds, 60°C for 30 seconds and 72°C for 30 seconds. After reaction, the reaction mixture was subjected to electrophoresis on 2% SeaPlaque GTG agarose gel, and a 350-bp amplification product of interest was excised from the gel. The DNA was recovered using EASYTRAP Ver. 2 (Takara Shuzo) according to the instructions attached to the kit. A transcript RNA was synthesized using the recovered DNA as a template and Competitive RNA Transcription Kit (Takara Shuzo) according to the instructions attached to the kit. The RNA was used as a template for examination of the one-step RT-ICAN. The template RNA corresponding to 0, 1 x $10^5$, 1 x $10^6$ or 1 x $10^7$ copies was added. Reaction mixtures of final volumes of 50 μl containing the following at final concentrations were prepared: 32 mM HEPES-potassium hydroxide buffer (pH 7.8), 100 mM potassium acetate, 1% DMSO, 0.01% BSA, 4 mM magnesium acetate, 500 μM each of dNTPs, 50 pmol each of primers represented by SEQ ID NOS:15 and 16, 5 U of Afu RNase HII, 4 U of BcaBEST DNA polymerase, 20 U of RNase inhibitor, 2.5 U of AMV RTase XL (Takara Shuzo), 1 μl of the transcript RNA corresponding to the given copy number, and 5 μl of 3000-fold dilution of the stock solution of SYBR Green I (SYBR Green I nucleic acid Gel Stain, BioWhittaker Molecular Applications) with sterile water as an intercalator. 25 μl of each reaction mixture was used for an ICAN reaction at 53°C. ABI PRISM™ 7700 System (Applied Biosystems) was used for the ICAN reactions and detection. The results are shown in Figure 2B. As shown in Figure 2B, it was confirmed that a target nucleic acid could be detected in a real-time manner using the one-step RT-ICAN method.

(3) An ICAN detection system using an intercalator was examined. A chlamydia genome was selected as a subject. Primers CT2F (SEQ ID NO:17) and CT2R (SEQ ID NO:18) were synthesized on the basis of the nucleotide sequence of the Chlamydia trachomatis plasmid (GenBank accession number X06707). The length of the region bordered by the primer pair including the primer portions is 109 bp. In addition, primers CT-FB19-3 (SEQ ID NO:19) and CT-RB23-2 (SEQ ID NO:20) were also used as primers for amplifying a chlamydia. The length of the region bordered by the primer pair including the primer portions is 107 bp. Reaction mixtures of final volumes of 50 μl containing the following at final concentrations were prepared: 32 mM HEPES-potassium hydroxide buffer (pH 7.8), 100 mM potassium acetate, 1% DMSO, 0.01% BSA, 4 mM magnesium acetate, 500 μM each of dNTPs, 50 pmol each of the primers CT2F and CT2R or the primers CT-FB19-3 and CT-RB23-2, 35 U of Afu RNase HII, 8 U of BcaBEST DNA polymerase, 1 μl of a sample and sterile water. 2.5 μl of a 3000-fold dilution of the SYBR Green I stock solution as an intercalator was added to 22.5 μl of each reaction mixture and the reaction mixtures were subjected to ICAN reactions at 55°C. Smart Cycler was used for the ICAN reactions and detection. The results are shown in Figure 2C. As shown in Figure 2C, it was confirmed that a target nucleic acid could be detected in a real-time manner using an intercalator in the system for detecting a chlamydia.

As described above, it was confirmed that the method of the present invention can be used to specifically detect a target nucleic acid in a real-time manner.

Example 3

Storage stability of a reagent used for the method of the present invention was examined as follows.

(1) A premixed solution for ICAN reaction containing the following was prepared and stored at 4°C or 30°C for about one month: 1.6 mM each of dNTPs, 101 mM HEPES-potassium hydroxide buffer (pH 7.8), 317 mM potassium acetate, 12.7 mM magnesium acetate, 0.03% bovine serum albumin, 3.2% dimethyl sulfoxide, 0.56 U/μl of Afu RNase HII and 0.35 U/μl of BcaBEST DNA polymerase.

16.125 μl of an aqueous solution containing 50 pmol each of primers for detecting Mycobacterium tuberculosis, K-F-1033(68) (SEQ ID NO:21) and K-R-1133(68) (SEQ ID NO:22) was added to 7.875 μl of the premixed solution for ICAN reaction. In addition, 1 μl of an aqueous solution containing the BCG genomic DNA prepared in Example 1 at a concentration of 100 pg/μl or 10 pg/μl was added to the mixture. The resulting mixtures were subjected to ICAN reactions at 64°C for 1 hour. After reaction, 5 μl each of the reaction mixtures was subjected to electrophoresis on 3.0% agarose gel for confirming the amplification. The procedure was repeated after appropriate storage periods (in days), and the stability of the premixed solution for ICAN reaction was assessed based on the ICAN amplification products observed upon electrophoresis. The results are shown in Figure 3. Figure 3 is a figure showing the electrophoresis that represents the storage stability of the premixed solution of the present invention. Figure 3A shows results of storage at 4°C. Lane 1: immediately after preparation of the premixed solution, 100 pg of the BCG genomic DNA; lane 2: immediately after preparation, 10 pg; lane 3: 9 days after preparation, 100 pg; lane 4: 9 days after preparation, 10 pg; lane 5: 18 days after preparation, 100 pg; lane 6: 18 days after preparation, 10 pg; lane 7: 28 days after preparation, 100 pg; and lane 8: 28 days after preparation, 10 pg.

Figure 3B shows results of storage at 30°C. Lane 1: immediately after preparation of the premixed solution, 100 pg of the BCG genomic DNA; lane 2: immediately after preparation, 10 pg; lane 3: 6 days after preparation, 100 pg; lane 4: 6 days after preparation, 10 pg; lane 5: 10 days after preparation, 100 pg; and lane 6: 10 days after preparation, 10 pg.

As shown in Figure 3, it was confirmed that the premixed solution could be used to stably carry out an ICAN reaction after storage at 4°C for 28 days or longer from the preparation. It was also confirmed that the premixed solution could be used to stably carry out an ICAN reaction after storage at 30°C for 10 days or longer from the preparation. Thus, it was confirmed that a reagent for amplification using the ICAN method can be stored for a long period of time by preparing and storing a premixed solution for reaction under the conditions as described above.

In addition, when premixed solutions for ICAN containing a 3-, 10- or 30-fold amount of Afu RNase HII were prepared and subjected to storage tests under the conditions as described above, similar results were obtained.

Example 4

Stability and long-term storability of a reagent used for the method of the present invention were examined as follows.

(1) The composition of a premixed solution for storage was examined. Specifically, a solution (I) containing 142 mM HEPES-potassium hydroxide buffer (pH 7.8), 444 mM potassium acetate, 0.044% bovine serum albumin, 4.44% dimethyl sulfoxide, 0.8 U/μl of Afu RNase HII and 0.5 U/μl of BcaBEST DNA polymerase was prepared. A premix [A], a premix [B], a premix [C] and a premix [D] were prepared by adding the following to 5.625 μl of the solution (I): the premix [A] (no primer): 1 μl of 100 mM magnesium acetate and 1.25 μl of dNTPs; the premix [B] (no dNTPs): 1 μl each of the primers for detecting Mycobacterium tuberculosis, K-F-1033(68) and K-R-1133(68) (50 pmol/μl), and 1 μl of 100 mM magnesium acetate; the premix [C] (no magnesium acetate): 1 μl each of K-F-1033(68) and K-R-1133(68) (50 pmol/μl), and 1.25 μl of 10 mM dNTPs; and the premix [D]: 1 μl each of K-F-1033(68) and K-R-1133(68) (50 pmol/μl), 1 μl of 100 mM magnesium acetate and 1.25 μl of 10 mM dNTPs. The premixed solutions were stored at 30°C for 2 hours. 1 μl each of K-F-1033(68) and K-R-1133(68) (50 pmol/μl) was added to 7.875 μl of the premix [A]; 1.25 μl of 10 mM dNTPs was added to 8.625 μl of the premix [B]; and 1 μl of 100 mM magnesium acetate was added to 8.875 μl of the premix [C]. Then, injectable water was added to the premix [A], the premix [B], the premix [C] or 9.875 μl of the premix [D] to a volume of 24 μl. 1 μl of the BCG genomic DNA at a concentration of 100 pg/μl was added to each mixture. The resulting mixtures were incubated at 64°C for 1 hour. After reaction, 5 μl each of the reaction mixtures was subjected to electrophoresis on 3.0% agarose gel for confirming the amplification. As a result, it was confirmed that compositions of the premix [A], the premix [B] and the premix [C] were preferable for stabilization of the reagent.

(2) Changes during storage were examined using a premixed solution for storage which contained all the components necessary for an ICAN reaction and premixed solutions for storage from which a primer, $Mg^{2+}$ or the like was eliminated. Specifically, a premix (I) containing 69 mM HEPES-potassium hydroxide buffer (pH 7.8), 215 mM potassium acetate, 0.022% bovine serum albumin, 2.2% dimethyl sulfoxide, 8.6 mM magnesium acetate, 1.1 mM each of dNTPs, 0.38 U/μl of Afu RNase HII, 0.24 U/μl of BcaBEST DNA polymerase, 80 kBq/μl of [$\alpha$-$^{33}$P]-dATP (Amersham Pharmacia Biotech) and 5.2 μM each of the primers for detecting Mycobacterium tuberculosis, K-F-1033(68) and K-R-1133(68), was prepared. In addition, a premix (II) in which the primers were eliminated from the premix (I), and a premix (III) in which magnesium acetate was eliminated from the premix (I) were prepared. 3 μl of a sample was taken from each premix after storage at 30°C for 2, 5 or 20 hours and frozen at -20°C. The samples were subjected to electrophoresis on 15% acrylamide gel. The gel was run for 1.5 hours, dried and then subjected to autoradiography. The results are shown in Figure 4. Figure 4 is a figure showing the autoradiography. Lane 1: the premix (III) stored for 2 hours; lane 2: the premix (III) stored for 5 hours; lane 3: the premix (III) stored for 20 hours; lane 4: the premix (II) stored for 2 hours; lane 5: the premix (II) stored for 5 hours; lane 6: the premix (II) stored for 20 hours; lane 7: the premix (I) stored for 2 hours; lane 8: the premix (I) stored for 5 hours; and lane 9: the premix (I) stored for 20 hours.

As shown in Figure 4, it was confirmed that generation of a macromolecular DNA during storage as detected for the premix (I) was suppressed by changing the composition to that of the premix (II) or the premix (III) in which the primers or magnesium acetate were (was) eliminated from the premix (I). Thus, it was confirmed that a side reaction of a chimeric oligonucleotide primer can be suppressed, and a reagent can be stabilized by eliminating the chimeric oligonucleotide primer, a magnesium salt or dNTPs from a reaction mixture.

(3) The concentration rate (salt concentration) of a premixed solution for storage containing all the components necessary for an ICAN reaction except primers was examined. Specifically, a premixed solution A for ICAN reaction, a premixed solution B for ICAN reaction and a premixed solution C for ICAN reaction containing the following were prepared and stored at 30°C: the premixed solution A: 1.6 mM each of dNTPs, 102 mM HEPES-potassium hydroxide buffer (pH 7.8), 317 mM potassium acetate, 13 mM magnesium acetate, 3.2% dimethyl sulfoxide, 0.03% bovine serum albumin, 0.556 U/μl of Afu RNase HII and 0.349 U/μl of BcaBEST DNA polymerase; the premixed solution B: 1 mM each of dNTPs, 64 mM HEPES-potassium hydroxide buffer (pH 7.8), 200 mM potassium acetate, 8 mM magnesium acetate, 2% dimethyl sulfoxide, 0.02% bovine serum albumin, 0.35 U/μl of Afu RNase HII and 0.22 U/μl of BcaBEST

DNA polymerase; and the premixed solution C: 0.57 mM each of dNTPs, 36 mM HEPES-potassium hydroxide buffer (pH 7.8), 114 mM potassium acetate, 4.5 mM magnesium acetate, 1.1% dimethyl sulfoxide, 0.01% bovine serum albumin, 0.2 U/μl of Afu RNase HII and 0.125 U/μl of BcaBEST DNA polymerase.

50 pmol each of the primers for detecting Mycobacterium tuberculosis, K-F-1033(68) and K-R-1133(68) as well as injectable water to 24 μl were added to 7.875 μl of the premixed solution A, 12.5 μl of the premixed solution B or 22 μl of the premixed solution C. Then, 1 μl of an aqueous solution containing the BCG genomic DNA at a concentration of 100 pg/μl or 10 pg/μl was added thereto. The resulting mixtures were subjected to ICAN reactions at 64°C for 1 hour. After reaction, 5 μl each of the reaction mixtures was subjected to electrophoresis on 3.0% agarose gel for confirming the amplification. The procedure was carried out at appropriate intervals, and the stabilities of the premixed solutions were assessed based on the ICAN amplification products observed upon electrophoresis. The results are shown in Figure 5. Figure 5 is a figure showing the electrophoresis that represents the storage stabilities of the premixed solutions A, B and C of the present invention. Lane 1: the premixed solution A, 13 days after preparation, 100 pg of the BCG genomic DNA; lane 2: the premixed solution A, 13 days after preparation, 10 pg; lane 3: the premixed solution B, 13 days after preparation, 100 pg; lane 4: the premixed solution B, 13 days after preparation, 10 pg; lane 5: the premixed solution C, 13 days after preparation, 100 pg; lane 6: the premixed solution C, 13 days after preparation, 10 pg; lane 7: the premixed solution B, 18 days after preparation, 100 pg; lane 8: the premixed solution B, 18 days after preparation, 10 pg; lane 9: the premixed solution C, 18 days after preparation, 100 pg; and lane 10: the premixed solution C, 18 days after preparation, 10 pg.

As shown in Figure 5, it was confirmed that the premixed solutions A and B could be stored at 30°C for about 2 and 3 weeks, respectively. The premixed solution C could be used to stably carry out an ICAN reaction 18 days or longer after preparation.

Thus, it was confirmed that the concentration of salt contained in the reaction buffer containing enzymes was preferably around the final salt concentration upon reaction. Specifically, the preferable concentration was 1.5 times or less the final salt concentration upon reaction.

(4) If a solution containing an enzyme is to be stored, the stability of the enzyme is generally increased by elevating the enzyme concentration. Stability of a premix for storage was examined. The premix for storage was designed such that the enzyme concentration was kept high by decreasing the salt concentration and adding a salt immediately before the reaction to compensate for the insufficiency for an ICAN reaction. Specifically, a premixed solution for ICAN reaction containing the following was prepared and stored at 30°C: 2.5 mM each of dNTPs, 32 mM HEPES-potassium hydroxide buffer (pH 7.8), 100 mM potassium acetate, 4 mM magnesium acetate, 0.05% bovine serum albumin, 0.875 U/μl of Afu RNase HII and 0.55 U/μl of BcaBEST DNA polymerase.

19 μl of an aqueous solution containing 50 pmol each of the primers for detecting Mycobacterium tuberculosis, K-F-1033(68) and K-R-1133(68), 33.64 mM HEPES-potassium hydroxide buffer (pH 7.8), 105.22 mM potassium acetate, 4.21 mM magnesium acetate and 1.32% dimethyl sulfoxide was added to 5 μl of the premixed solution for ICAN reaction. 1 μl of an aqueous solution containing the BCG genomic DNA at a concentration of 100 pg/μl or 10 pg/μl was added to the mixture. The resulting mixtures were subjected to ICAN reactions at 64°C for 1 hour. After reaction, 5 μl each of the reaction mixtures was subjected to electrophoresis on 3.0% agarose gel for confirming the amplification.

The procedure was carried out at appropriate intervals, and the stabilities of the premixed solutions of the present invention were assessed based on the ICAN amplification product observed upon electrophoresis. The results are shown in Figure 6. Figure 6 is a figure showing the electrophoresis that represents the storage stabilities of the premixed solutions of the present invention. Lane 1: immediately after preparation of the premixed solution, 100 pg of the BCG genomic DNA; lane 2: immediately after preparation, 10 pg; lane 3: 21 days after preparation, 100 pg; lane 4: 21 days after preparation, 10 pg; lane 5: 25 days after preparation, 100 pg; lane 6: 25 days after preparation, 10 pg; lane 7: 32 days after preparation, 100 pg; and lane 8: 32 days after preparation, 10 pg.

As shown in Figure 6, it was confirmed that the premixed solution could be used to stably carry out an ICAN reaction after storage at 30°C for 32 days or longer. Thus, it was confirmed that a reagent for amplification using the ICAN method can be stored for a long period of time by preparing and storing a premixed solution for reaction under the conditions as described above.

Similar results were obtained when examination was carried out as described in (1) to (4) above using a combination of BcaBEST DNA polymerase and Tli RNase H.

Thus, it was confirmed that the ratio of concentration rates (the ratio of the enzyme concentration rate / the salt concentration rate) is preferably above 1, defining the final concentration of the enzyme (DNA polymerase and/or RNase H) or the salt upon reaction as 1. Furthermore, it was confirmed that the ratio is preferably 5 or less.

Similar results were obtained when examination was carried out as described in (1) to (4) above increasing the amount of RNase H to be used by 3-, 10- or 30-fold.

Example 5

Detection of a Mycobacterium avium complex using the method of the present invention was examined. First, positive controls for Mycobacterium avium and Mycobacterium intracellulare were prepared. 560-bp amplification products corresponding to portions of sequences of the 16S RNA genes from Mycobacterium avium and Mycobacterium intracellulare having nucleotide sequences of SEQ ID NOS:23 and 24 were obtained using Ex Taq DNA polymerase for extension reactions followed by PCRs using ten 74-mer synthetic primers according to the method as described in BioTechniques, 9(3):298-300 (1990). The amplification products were inserted into pT7 Blue T vector using DNA Ligation Kit Ver. 2 (Takara Shuzo). The ligation mixtures were used to transform E. coli JM109 to prepare plasmids as positive controls for M. avium and M. intracellulare. Primers Myco-F-1, Myco-F-2, Myco-F-3, Myco-R-1, Myco-R-1-2, Myco-R-2I and Myco-R-2A having nucleotide sequences of SEQ ID NOS:25 to 31 were synthesized. The reactions were carried out as follows. Briefly, reaction mixtures of final volumes of 25 μl containing the following at final concentrations were prepared: 32 mM HEPES-potassium hydroxide buffer (pH 7.8), 100 mM potassium acetate, 1% DMSO, 0.01% BSA, 4 mM magnesium acetate, 500 μM each of dNTPs, 4.4 U of Afu RNase HII or 4 U of Tli RNase H, 4 U of BcaBEST DNA polymerase, $10^5$ copies of the positive control for M. avium or M. intracellulare as a template, and 25 pmol each of an upstream primer and a downstream primer. The reaction mixtures were placed in a thermal cycler which had been set at 55°C and incubated for 60 minutes. When the positive control for M. avium was used as a template, 101-bp, 96-bp, 96-bp, 101-bp, 96-bp, 96-bp, 106-bp, 101-bp and 101-bp amplification products of interest were observed upon agarose gel electrophoresis using either RNase H and a pair of the primers: Myco-F-1 and Myco-R-1; Myco-F-1 and Myco-R-1-2; Myco-F-1 and Myco-R-2A; Myco-F-2 and Myco-R-1; Myco-F-2 and Myco-R-1-2; Myco-F-2 and Myco-R-2A; Myco-F-3 and Myco-R-1; Myco-F-3 and Myco-R-1-2; or Myco-F-3 and Myco-R-2A. No amplification product was observed using a pair of the primers Myco-F-1 and Myco-R-2I; Myco-F-2 and Myco-R-2I; or Myco-F-3 and Myco-R-2I.

When the positive control for M. intracellulare was used as a template, 101-bp, 96-bp, 96-bp, 101-bp, 96-bp, 96-bp, 106-bp, 101-bp and 101-bp amplification products of interest were observed upon agarose gel electrophoresis using either RNase H and a pair of the primers: Myco-F-1 and Myco-R-1; Myco-F-1 and Myco-R-1-2; Myco-F-1 and Myco-R-2I; Myco-F-2 and Myco-R-1; Myco-F-2 and Myco-R-1-2; Myco-F-2 and Myco-R-2I; Myco-F-3 and Myco-R-1; Myco-F-3 and Myco-R-1-2; or Myco-F-3 and Myco-R-2I. No amplification product was observed using a pair of the primers Myco-F-1 and Myco-R-2A; Myco-F-2 and Myco-R-2A; or Myco-F-3 and Myco-R-2A.

Based on the above-mentioned results, it was confirmed that M. avium could be distinguished from M. intracellulare using the primer Myco-R-2I or Myco-R-2A which resulted in template-specific reactions. Dot blot hybridization with the amplified fragments was carried out as follows using avium-probe (SEQ ID NO:32) or intracellulare-probe (SEQ ID NO:33) each labeled at the 5' end with biotin. Briefly, 1 μl each of the reaction mixtures was subjected to denaturation at 98°C for 5 minutes, rapidly cooled on ice, and spotted onto Hybond-N™ (Amersham Pharmacia Biotech). After UV irradiation, the membrane was placed in a hybridization bag. 10 ml of a hybridization solution (0.5 M disodium hydrogenphosphate (pH 7.2), 1 mM ethylenediaminetetraacetic acid and 7% sodium lauryl sulfate) was added thereto. Prehybridization was carried out at 42°C for 30 minutes. 10 μl of a solution containing the avium-probe or the intracellulare-probe at a concentration of 100 ng/μl was heat-denatured and added to the prehybridization reaction system. After hybridization at 42°C for 60 minutes, the membrane was washed twice at room temperature for 5 minutes in a solution containing 66.6 mM sodium chloride, 66.6 mM trisodium citrate hydrate and 0.1% sodium lauryl sulfate. The membrane was incubated at 42°C for 12 minutes in a mixture prepared by adding 2 μl of a solution containing Horseradish peroxidase streptoavidin conjugate (Pierce) at a concentration of 5 mg/ml to 6 ml of a washing buffer (0.3 M sodium chloride, 17.3 mM sodium dihydrogenphosphate dihydrate, 2.5 mM EDTA and 0.1% sodium lauryl sulfate). The membrane was washed twice in the washing buffer at room temperature followed by 10 ml of 0.1 M citrate buffer (pH 5.0) at room temperature, and reacted in the dark for about 10 minutes in a mixture of 5 ml of 0.1 M citrate buffer, 5 μl of 3% hydrogen peroxide and 250 μl of a solution containing tetramethylbenzidine (TMB, Nacalai Tesque) at a concentration of 2 mg/ml in ethanol. After color development, the reaction was terminated with deionized water.

As a result, a signal for the amplification product from the positive control for M. avium could be observed only using the avium-probe labeled at the 5' end with biotin and no signal could be observed using the intracellulare-probe labeled at the 5' end with biotin. On the other hand, a signal for the amplification product from the positive control for M. intracellulare could be observed only using the intracellulare-probe labeled at the 5' end with biotin and no signal could be observed using the avium-probe labeled at the 5' end with biotin. Based on these results, it was confirmed that M. avium could be distinguished from M. intracellulare using these probes.

Example 6

Detection of a gonococcus using the method of the present invention was examined. 560-bp fragments amplified from the Neisseria gonorrhoeae CppB gene, the Neisseria gonorrhoeae plasmid pJD4 gene and the Neisseria gonorrhoeae DNA cytosine methyltranferase (M.NgoMIII) gene having nucleotide sequences of SEQ ID NOS:34 to 37 were obtained according to the method as described in Example 5. A 490-bp fragment was amplified from the Neisseria gonorrhoeae N-4 cytosine-specific methyltransferase gene in a similar manner using ten 67-mer synthetic primers. The amplification products were inserted into pT7 Blue T vector to construct positive controls A, B, C and D.

Primers NEI-5103, NEI-5150, CppB-F1, CppB-F2, CppB-F3, CppB-R1, CppB-R2, CppB-R3, pJDB F-1, pJDB F-2, pJDB R-1, pJDB R-2, pJDB R-3, M.Ngo F-1, M.Ngo F-2, M.Ngo F-3, M.Ngo R-1, M.Ngo R-2, M.Ngo R-3, M.Ngo R-4, Cytosine F-1, Cytosine F-2, Cytosine F-3, Cytosine R-1, Cytosine R-2, Cytosine R-3, pJDB10F and pJDB10R having nucleotide sequences of SEQ ID NOS:38-63 and 164-165 were synthesized. ICAN reactions were carried out under the conditions as described in Example 5 using $10^6$ copies of the positive control B as a template and a pair of the primers NEI-5103 and NEI-5150. As a result, 69-bp amplified fragments were be observed upon agarose gel electrophoresis using either RNase H. The amplified fragments were spotted onto a nylon membrane, and dot blot hybridization was carried out according to the method as described in Example 5 using NEI-5130 probe (SEQ ID NO:64) labeled at the 5' end with biotin. As a result, signals of interest were observed.

Similarly, ICAN reactions were carried out under the conditions as described in Example 5 using $10^6$ copies of the positive control A as a template as well as a pair of the primers :CppB-F1 and CppB-R1; CppB-F1 and CppB-R2; CppB-F1 and CppB-R3; CppB-F2 and CppB-R1; CppB-F2 and CppB-R2; CppB-F2 and CppB-R3; CppB-F3 and CppB-R1; CppB-F3 and CppB-R2; or CppB-F3 and CppB-R3. 60-bp amplified fragments of interest were observed upon agarose gel electrophoresis in all cases. Dot blot hybridization with the amplified fragments was carried out according to the method as described in Example 5 using NEI-CppB probe-1 (SEQ ID NO:65) labeled at the 5' end with biotin. As a result, signals of interest were observed.

ICAN reactions were carried out under the conditions as described in Example 5 using $10^6$ copies of the positive control A as a template as well as a pair of the primers :pJDB F-1 and pJDB R-1; pJDB F-1 and pJDB R-2; pJDB F-1 and pJDB R-3; pJDB F-2 and pJDB R-1; pJDB F-2 and pJDB R-2; or pJDB F-2 and pJDB R-3. 91-bp amplified fragments of interest were observed upon agarose gel electrophoresis in all cases. ICAN reactions were carried out under the conditions as described in Example 5 using a pair of primers pJDB10F and pJDB10R. 71-bp amplified fragments of interest were observed upon agarose gel electrophoresis using either RNase H. The amplified fragments were spotted onto a riylon membrane, and dot blot hybridization was carried out according to the method as described in Example 5 using NEI-CppB probe-2 (SEQ ID NO:66) labeled at the 5' end with biotin. As a result, signals of interest were observed.

Similarly, ICAN reactions were carried out under the conditions as described in Example 5 using $10^6$ copies of the positive control C as a template as well as a pair of the primers: M.Ngo F-1 and M.Ngo R-1; M.Ngo F-1 and M. Ngo R-2; M.Ngo F-1 and M.Ngo R-3; M.Ngo F-1 and M.Ngo R-4; M.Ngo F-2 and M.Ngo R-1; M.Ngo F-2 and M.Ngo R-2; M.Ngo F-2 and M.Ngo R-3; M.Ngo F-2 and M.Ngo R-4; M.Ngo F-3 and M.Ngo R-1; M.Ngo F-3 and M.Ngo R-2; M.Ngo F-3 and M.Ngo R-3; or M.Ngo F-3 and M.Ngo R-4. 60-bp amplified fragments of interest were observed upon agarose gel electrophoresis in all cases. Dot blot hybridization was carried out using M.Ngo-probe (SEQ ID NO:67) labeled at the 5' end with biotin. As a result, signals of interest were observed. Furthermore, ICAN reactions were carried out under the conditions as described in Example 5 using $10^6$ copies of the positive control D as a template as well as a pair of the primers: Cytosine F-1 and Cytosine R-1; Cytosine F-1 and Cytosine R-2; Cytosine F-1 and Cytosine R-3; Cytosine F-2 and Cytosine R-1; Cytosine F-2 and Cytosine R-2; Cytosine F-2 and Cytosine R-3; Cytosine F-3 and Cytosine R-1; Cytosine F-3 and Cytosine R-2; or Cytosine F-3 and Cytosine R-3. 70-bp amplified fragments of interest were observed upon agarose gel electrophoresis in all cases. Dot blot hybridization was carried out using Cytosine-probe (SEQ ID NO:68) labeled at the 5' end with biotin. As a result, signals of interest were observed.

Example 7

Detection of human hepatitis B virus (HBV) using the method of the present invention was examined. Specifically, 560-bp portions of the HBV X protein gene represented by SEQ ID NOS:69 and 70 were amplified according to the method as described in Example 5, inserted into pT7 Blue T vector, and used as HBV positive control 1-T and HBV positive control 1-G. Primers HBV-F-1, HBV-F-2, HBV-F-3, HBV-F-4, HBV-R-1, HBV-R-2, HBV-R-3 and HBV-R-4 having nucleotide sequences of SEQ ID NOS:7.1 to 78 were synthesized. ICAN reactions were carried out under the conditions as described in Example 5 using $10^6$ copies of the HBV positive control 1-G or the HBV positive control 1-T as a template as well as a pair of the primers: HBV-F-1. and HBV-R-1; HBV-F-1 and HBV-R-2; HBV-F-2 and HBV-R-1; or HBV-F-2 and HBV-R-2. 81-bp, 76-bp, 76-bp and 71-bp amplified fragments of interest were observed upon agarose gel electrophoresis.

ICAN reactions were carried out under the conditions as described in Example 5 using $10^6$ copies of the HBV positive control 1-T or 1-G as a template as well as a pair of the primers: HBV-F-3 and HBV-R-3; HBV-F-3 and HBV-R-4; HBV-F-4 and HBV-R-3; or HBV-F-4 and HBV-R-4. 84-bp, 79-bp, 78-bp and 73-bp amplified fragments of interest were observed upon agarose gel electrophoresis. The amplified fragments obtained using the pairs of primers HBV-F-1 and HBV-R-1; HBV-F-1 and HBV-R-2; HBV-F-2 and HBV-R-1; and HBV-F-2 and HBV-R-2 were spotted onto a nylon membrane, and dot blot hybridization was carried out using HBV-probe 1 (SEQ ID NO:79) labeled at the 5' end with biotin. As a result, signals of interest were observed. Similarly, the amplified fragments obtained using the pairs of primers HBV-F-3 and HBV-R-3; HBV-F-3 and HBV-R-4; HBV-F-4 and HBV-R-3; and HBV-F-4 and HBV-R-4 were subjected to dot blot hybridisation using HBV-probe 2 (SEQ ID NO:80) labeled at the 5' end with biotin. As a result, signals of interest were observed.

Example 8

Detection of HCV using the method of the present invention was examined. Reaction mixtures of final volumes of 50 μl containing the following at final concentrations were prepared: 32 mM HEPES-potassium hydroxide buffer (pH 7.8), 100 mM magnesium acetate, 1% dimethyl sulfoxide, 0.01% BSA, 4 mM magnesium acetate, 500 μM each of dNTPs, 50 pmol each of primers HCV-A2-S and HCV-A2-A; HCV-A4-S and HCV-A4-A; HCV-A4-S19 and HCV-A4-A19; HCV-F1 and HCV-R1; or HCV-F2 and HCV-R2 (SEQ ID NOS:81-86 and 88-89), 20 U of Afu RNase HII or 4 U of Tli RNase H, 4 U of BcaBEST DNA polymerase, 20 U of RNase inhibitor, 1.25 U of AMV RTase XL, and $10^6$ copies of the RNA transcript as described in Example 2-(2) as a template. The reaction mixtures were placed in Thermal Cycler Personal which had been set at 53°C and incubated for 60 minutes. After reaction, 3 μl each of the reaction mixtures was subjected to electrophoresis on 3% agarose gel. As a result, 74-bp, 76-bp, 78-bp, 61-bp and 61-bp amplified fragments of interest were observed using either RNase H. Dot blot hybridization with the amplified fragments obtained using the pairs of primers HCV-A2-S and HCV-A2-A; HCV-A4-S and HCV-A4-A; and HCV-A4-S19 and HCV-A4-A19 was carried out using HCV-C probe (SEQ ID NO:87) labeled at the 5' end with biotin. As a result, signals of interest were observed. Similarly, dot blot hybridization with the amplified fragments obtained using the pairs of primers HCV-F1 and HCV-R1; and HCV-F2 and HCV-R2 was carried out using HCV-D probe (SEQ ID NO:92) labeled at the 5' end with biotin. As a result, signals of interest were observed.

Example 9

Application of the method of the present invention to a one-step RT-ICAN amplification method was examined. HIV was selected as a subject.

(1) Preparation of transcript RNA

A transcript RNA as a template was prepared. A plasmid into which the HIV gag region is inserted (ATCC 40829) was purchased. A PCR amplification product of about 1.4 kbp corresponding to a portion of the HIV gag region was' obtained using the plasmid as a template, a pair of primers SP6-HIV-F (SEQ ID NO:93) and HIV-R (SEQ ID NO: 94) and Ex Taq DNA polymerase. A transcript RNA was synthesized using the PCR amplification product as a template and Competitive RNA Transcription Kit (Takara Shuzo) according to the instructions attached to the kit. The RNA was used as an RNA template for examination of the one-step RT-ICAN.

(2) Examination of primer for detection of HIV using one-step RT-ICAN

Primers HIV-F1, HIV-F2, HLV-F3, HIV-F4, HIV-R1, HIV-R2, HIV-R3, HIV-R4 and HIV-R4M having nucleotide sequences of SEQ ID NOS:95 to 103 were synthesized. The reactions were carried out as follows. Briefly, reaction. mixtures of final volumes of 25 μl containing the following at final concentrations were prepared: 32 mM HEPES-potassium hydroxide buffer (pH 7.8), 100 mM potassium acetate, 1% DMSO, 0.01% BSA, 7 mM magnesium acetate, 500 μM each of dNTPs, 2.2 U of Afu RNase HII or 4 U of Tli RNase H, 5.5 U of BcaBEST DNA polymerase, 0.625 U of AMV RTase XL, $10^6$ copies of the transcript RNA as well as 25 pmol each of an upstream primer and a downstream primer. The reaction mixtures were placed in a thermal cycler which had been set at 55°C and incubated for 60 minutes. 100-bp, 74-bp, 76-bp, 72-bp, 72-bp, 72-bp, 74-bp, 70-bp, 70-bp, 76-bp, 78-bp, 74-bp and 74-bp amplified fragments of interest were observed using upon agarose gel electrophoresis either RNase H and the pairs of primers: HIV-F1 and HIV-R1; HIV-F2 and HIV-R2; HIV-F2 and HIV-R3; HIV-F2 and HIV-R4; HIV-F2 and HIV-R4M; HIV-F3 and HIV-R2; HIV-F3 and HIV-R3; HIV-F3 and HIV-R4; HIV-F3 and HIV-R4M; HIV-F4 and HIV-R2; HIV-F4 and HIV-R3; HIV-F4 and HIV-R4; and HIV-F4 and HIV-R4M. Dot blot hybridization with the amplified fragments was carried out using HIV-A probe (SEQ ID NO:104) or HIV-B probe (SEQ ID NO:105) each labeled at the 5' end with biotin. As a result, a signal of interest was observed for the amplification product obtained using the pair of primers HIV-F1 and HIV-R1 in case of the HIV-A probe labeled at the 5' end with biotin, and signals of interest were observed for the amplification products obtained using the pairs of primers: HIV-F2 and HIV-R2; HIV-F2 and HIV-R3; HIV-F2 and HIV-R4; HIV-F2 and HIV-R4M; HIV-F3 and HIV-R2; HIV-F3 and HIV-R3; HIV-F3 and HIV-R4; HIV-F3 and HIV-R4M; HIV-F4 and HIV-R2;

HIV-F4 and HIV-R3; HIV-F4 and HIV-R4; and HIV-F4 and HIV-R4M in case of the HIV-B probe labeled at the 5' end with biotin.

Example 10

Detection of Staphylococcus aureus using the method of the present invention was examined. A region of interest to be amplified was selected from the Staphylococcus aureus coagulase gene. First, a 221-bp amplification product was obtained by carrying out a PCR using primers coa-PCR-F (SEQ ID NO:106) and coa-PCR-R (SEQ ID NO:107), a Staphylococcus aureus genome as a template and Ex Taq DNA polymerase. A positive control for the coa gene was prepared by inserting the amplified fragment into pT7 Blue T vector.

Primers coa-F1, coa-F2, coa-F3, coa-F4, coa-F5, coa-R1, coa-R2, coa-R3, coa-R4 and coa-R5 having nucleotide sequences of SEQ ID NOS:108-117 were synthesized. ICAN reactions were carried out under the conditions as described in Example 5 using $10^6$ copies of the positive control for the coa gene as a template and a pair of the primers: coa-F1 and coa-R1; coa-F1 and coa-R2; coa-F2 and coa-R1; coa-F2 and coa-R2; coa-F3 and coa-R3; coa-F3 and coa-R4; coa-F3 and coa-R5; coa-F4 and coa-R3; coa-F4 and coa-R4; coa-F4 and coa-R5; coa-F5 and coa-R3; coa-F5 and coa-R4; or coa-F5 and coa-R5. As a result, 59-bp, 69-bp, 75-bp, 85-bp, 95-bp, 101-bp, 98-bp, 89-bp, 95-bp, 92-bp, 107-bp, 113-bp and 110-bp amplified fragments of interest were observed upon agarose gel electrophoresis using either RNase H. Dot blot hybridization with the amplification products obtained using the pairs of primers coa-F1 and coa-R1; coa-F1 and coa-R2; coa-F2 and coa-R1; and coa-F2 and coa-R2 was carried out according to the method as described in Example 5 using coa-A probe (SEQ ID NO:118) labeled at the 5' end with biotin. As a result, signals of interest were observed for all spots. Similarly, dot blot hybridization with the amplification products obtained using the pairs of primers coa-F3 and coa-R3; coa-F3 and coa-R4; coa-F3 and coa-R5; coa-F4 and coa-R3; coa-F4 and coa-R4; coa-F4 and coa-R5; coa-F5 and coa-R3; coa-F5 and coa-R4; and coa-F5 and coa-R5 was carried out according to the method as described in Example 5 using coa-B probe (SEQ ID NO:119) labeled at the 5' end with biotin. As a result, signals of interest were observed for all spots.

Example 11

(1) Detection of a chlamydia using the method of the present invention was examined. A chlamydia positive control was prepared by inserting a 560-bp amplified fragment corresponding to a portion of a chlamydia gene (SEQ ID NO:120) into pT7 Blue T vector according to the method as described in Example 5. Primers CT- FB19, CT-FB19-3, CT-FB-19-3-21, CT-FB19-3-23, CT-RB21, CT-RB23-2 and CT-RB23-2-24 having nucleotide sequences of SEQ ID NOS:121-127 were synthesized. ICAN reactions were carried out under the conditions as described in Example 5 using $10^6$ copies of the chlamydia positive control as a template and a pair of the primers: CT-FB19 and CT-RB21; CT-FB19 and CT-RB23-2; CT-FB19-3 and CT-RB21; CT-FB19-3 and CT-RB23-2; CT-FB19-3 and CT-RB23-2-24; CT-FB-19-3-21 and CT-RB23-2; CT-FB-19-3-21 and CT-RB23-2-24; CT-FB19-3-23 and CT-RB23-2; or CT-FB19-3-23 and CT-RB23-2-24. As a result, 125-bp, 116-bp, 116-bp, 107-bp, 107-bp, 107-bp, 107-bp, 107-bp and 107-bp amplified fragments of interest were observed upon agarose gel electrophoresis using either RNase H. Dot blot hybridization with the amplified fragments was carried out according to the method as described in Example 5 using CT-probe (SEQ ID NO:128) labeled at the 5' end with biotin. As a result, signals of interest were observed for all spots.

(2) Detection of a chlamydia using another region as a target was examined. A chlamydia positive control 2 was prepared by inserting a 560-bp amplified fragment corresponding to a portion of the chlamydia cryptic gene (SEQ ID NO:129) into pT7 Blue T vector according to the method as described in (1) above. Primers CT-F1212-20, CT-F1212-21, CT-F1212-22, CT-R1272-20, CT-R1272-21, CT-R1272-22, CT-F1215-4R-22 and CT-R1267-3R-18 having nucleotide sequences of SEQ ID NOS:130-135 and 166-167 were synthesized. ICAN reactions were carried out under the conditions as described in (1) above using $10^6$ copies of the chlamydia positive control 2 as a template and a pair of the primers: CT-F1212-20 and CT-R1272-20; CT-F1212-20 and CT-R1272-21; CT-F1212-20 and CT-R1272-22; CT-F1212-21 and CT-R1272-20; CT-F1212-21 and CT-R1272-21; CT-F1212-21 and CT-R1272-22; CT-F1212-22 and CT-R1272-20; CT-F1212-22 and CT-R1272-21; or CT-F1212-22 and CT-R1272-22. As a result, 61-bp amplification products of interest were observed upon agarose gel electrophoresis using either RNase H in all cases. Dot blot hybridization with the amplified fragments was carried out according to the method as described in Example 5 using CT-1234 probe (SEQ ID NO:136) labeled at the 5' end with biotin. As a result, signals of interest were observed for all spots. In addition, ICAN reactions were carried out using a pair of the primers CT-F1215-4R-22 and CT-R1267-3R-18. As a result, 53-bp amplification products of interest were observed upon agarose gel electrophoresis using either RNase H. Dot blot hybridization with the amplified fragments was carried out according to the method as described in Example 5 using CT-1236 probe (SEQ ID NO: 168) labeled at the 5' end with biotin. As a result, signals of interest were observed for all spots.

Example 12

Detection of Mycoplasma pneumoniae using the method of the present invention was examined. A Mycoplasma pneumoniae positive control A and a Mycoplasma pneumoniae positive control B were prepared by inserting 560-bp amplified fragments corresponding to portions of the Mycoplasma pneumoniae ATPase operon gene (SEQ ID NOS:137 and 138) into pT7 Blue T vector according to the method as described in Example 5. Primers Myco-140, Myco140-22, Myco-706, MPF-910, Myco-190, Myco-190-22, Myco-850 and MPR-1016 having nucleotide sequences of SEQ ID NOS:139-146 were synthesized. ICAN reactions were carried out under the conditions as described in Example 5 using $10^6$ copies of the Mycoplasma pneumoniae positive control A as a template and a pair of the primers: Myco-140 and Myco-190; Myco140-22 and Myco-190; Myco140-22 and Myco-190-22; or Myco-140 and Myco-190-22. As a result, 63-bp amplified fragments of interest were observed upon agarose gel electrophoresis using either RNase H in all cases. ICAN reactions were carried out under the conditions as described in Example 5 using $10^6$ copies of the Mycoplasma pneumoniae positive control B as a template and.a pair of the primers: Myco-706 and Myco-850; or MPF-910 and MPR-1016. As a result, 85-bp and 107-bp amplified fragments of interest were observed upon agarose gel electrophoresis using either RNase H. Dot blot hybridization with the amplified fragments obtained using the pairs of primers Myco-140 and Myco-190; Myco140-22 and Myco-190; Myco140-22 and Myco-190-22; and Myco-140 and Myco-190-22 was carried out according to the method as described in Example 5 using Myco-170-probe (SEQ ID NO: 147) labeled at the 5' end with biotin. As a result, signals of interest were observed for all spots. Similarly, dot blot hybridization with the amplified fragment obtained using the pair of primers Myco-706 and Myco-850 was carried out using Myco-730-probe (SEQ ID NO:148) labeled at the 5' end with biotin. As a result, a signal of interest was observed. Furthermore, dot blot hybridization with the amplified fragment obtained using the pair of primers MPF-910 and MPR-1016 was carried out using Myco-952-probe (SEQ ID NO:149) labeled at the 5' end with biotin. As a result, a signal of interest was observed.

Example 13

Detection of methicillin-resistant Staphylococcus aureus (MRSA) using the method of the present invention was examined. A- region of interest to be amplified was selected from the MecA gene. MecA positive controls A and B were prepared by inserting 560-bp amplified fragments corresponding to portions of the MecA gene, MecA-A and MecA-B (SEQ ID NOS:150 and 151) into pT7 Blue T vector according to the method as described in Example 5. Primers MecA-S525, MecA-A611, MecA-S1281 and MecA-A1341 having nucleotide sequences of SEQ ID NOS:152-155 were synthesized. ICAN reactions were carried out under the conditions as described in Example 5 using $10^6$ copies of the MecA positive control A as a template and a pair of the primers MecA-S525 and MecA-A611, or $10^6$ copies of the MecA positive control B as a template and a pair of the primers MecA-S1281 and MecA-A1341. As a result, 106-bp and 83-bp amplified fragments were observed upon agarose gel electrophoresis using either RNase H.

1 μl of the ICAN reaction mixture for which an amplified fragment was obtained using the pair of primers MecA-S525 and MecA-A611 was spotted onto a nylon membrane Hybond-N, and dot blot hybridization was carried out according to the method as described in Example 5 using MecA-A probe (SEQ ID NO:156) labeled at the 5' end with biotin. Furthermore, the amplified fragment obtained using the pair of primers MecA-S1281 and MecA-A1341 was spotted onto a nylon membrane in a similar manner, and dot blot hybridization was carried out using MecA-B probe (SEQ ID NO:157) labeled at the 5' end with biotin. As a result, signals were observed using both probes, confirming amplification of the regions of interest.

Example 14

RNase H that can be used for the method of the present invention was examined.

(1) Primers pDON-AI-1(22) and pDON-AI-2(23) having nucleotide sequences of SEQ ID NOS:158 and 159 were synthesized based the nucleotide sequence of the packaging region in a vector plasmid pDON-AI (Takara Shuzo). Reaction mixtures of total volumes of 25 μl each containing 1 μl of a solution containing 10 fg of pDON-AI DNA or water for a negative control, 100 pmol each of the primers, 0.5 mM each of dNTPs, 32 mM HEPES-potassium hydroxide buffer (pH 7.8), 100 mM potassium acetate, 4.0 mM magnesium acetate, 0.01% bovine serum albumin, 1.0% dimethyl sulfoxide, 2.64 U of Bca DNA polymerase and 8.75, 4.38, 2.19, 1.09, 0.55 or 0.27 U of Afu RNase HII, 4.69, 2.34, 1.17, 0.59, 0.29, 0.15 or 0.07 U of Pfu RNase HII, or 14, 7, 3.5, 1.75, 0.875 or 0.438 U of Pho RNase HII were incubated at 64°C for 1 hour in a thermal cycler. After reaction, 5 μl each of the reaction mixtures was analyzed using electrophoresis on 3.0% agarose gel. As a result, amplification products of interest were observed using Afu RNase HII regardless of the unit value. Furthermore, amplification products of interest were observed using 0.59 to 4.69 U of Pfu RNase HII or 1.75 to 14 U of Pho RNase HII. Based on these results, it was confirmed that all the heat-resistant RNase HIIs could be preferably used for the method of the present invention.

(2) A reaction mixture of a total volume of 50 µl containing 50 pmol each of primers having nucleotide sequences of SEQ ID NOS:160 and 161 synthesized on the basis of the nucleotide sequence of the mRNA for mouse inducible NO synthase (iNOS), 1 µl of a cDNA prepared according to a conventional method from a commercially available mouse cell (corresponding to 50 ng of RNA), 5 µl of 10 x Ex Taq buffer (Takara Shuzo), 1.25 U of TaKaRa Ex Taq DNA polymerase (Takara Shuzo) and 0.2 mM each of dNTPs was reacted using a thermal cycler. The program was as follows: 1 cycle of 94°C for 2 minutes; 30 cycles of 94°C for 30 seconds, 55°C for 30 seconds and 72°C for 30 seconds; and 1 cycle of 72°C for 5 minutes. The PCR amplified fragment from mouse iNOS cDNA was cloned into a plasmid vector pT7 Blue T-vector (Novagen) which was used as a template for the method of the present invention. Reaction mixtures of total volumes of 25 µl each containing 1 µl of a solution containing 10 fg of the plasmid DNA as a template or 1 ml of water for a negative control, 100 pmol each of primers NS5 (SEQ ID NO:9) and NS6 (SEQ ID NO:10), 0.5 mM each of dNTPs, 32 mM HEPES-potassium hydroxide buffer (pH 7.8), 100 mM potassium acetate, 4.0 mM magnesium acetate, 0.01% bovine serum albumin, 1.0% dimethyl sulfoxide, 2.64 U of Bca DNA polymerase and 10, 5, 2.5, 1.25, 0.25, 0.125 U of Methanococcus jannashi (Mja) RNase HII, Tce RNase HII or Tli RNase HII were incubated at 62°C for 1 hour in a thermal cycler. Mja RNase HII was prepared according to the method as described in Structure, 8:897-904. After reaction, 5 µl each of the reaction mixtures was analyzed using electrophoresis on 3.0% agarose gel. The results are shown in Figure 7. Figure 7 is a figure showing the agarose gel electrophoresis of amplification products obtained using the three types of RNase Hs. Figure 7A, 7B and 7C represent results obtained using Methanococcus jannashi RNase HII, Thermococcus celer RNase HII and Thermococcus litoralis RNase HII, respectively. Lane 1: 10 U of RNase HII; lane 2: 10 U of RNase HII, negative control; lane 3: 5 U of RNase HII; lane 4: 5 U of RNase HII, negative control; lane 5: 2.5 U of RNase HII; lane 6: 2.5 U of RNase HII, negative control; lane 7: 1.25 U of RNase HII; lane 8: 1.25 U of RNase HII, negative control; lane 9: 0.25 U of RNase HII; lane 10: 0.25 U of RNase HII, negative control; lane 11: 0.125 U of RNase HII; and lane 12: .0125 U of RNase HII negative control.

As shown in Figure 7, amplification products of interest were observed using 0.125 to 10 U of Mja RNase HII, 1 to 5 U of Tce RNase HII, or 0.125 to 5 U of Tli RNase HII. Based on these results, it was confirmed that all the heat-resistant RNase HIIs could be preferably used for the method of the present invention.

(3) The method of the present invention was further examined under the reaction conditions as described in Example 5 except that different type and amount of RNase H were used. 1 U of Thermococcus litoralis RNase HII or 8.75 U of Thermococcus celer RNase HII was used as an RNase H. Amplification reactions were carried out using $10^6$ copies of the HBV positive control 1-T as a template as well as the pair of primers HBV-F-2 and HBV-R-1 prepared in Example 7. As a result, 76-bp amplified fragments of interest were observed upon agarose gel electrophoresis using either RNase H. Based on these results, it was confirmed that both RNase HIIs could be preferably used according to the present invention

Example 15

The detection method of the present invention was examined using a reaction system containing an internal control. First, an internal control for Mycobacterium tuberculosis was prepared according to the method as described in Example 5. A 169-bp amplification product having a nucleotide sequence of SEQ ID NO:169 was obtained using Ex Taq DNA polymerase for an extension reaction followed by a PCR using four 60-mer synthetic primers according to the method as described in BioTechniques, 9(3):298-300 (1990). The amplification product was inserted into pT7 Blue T vector using DNA Ligation Kit Ver. 2 (Takara Bio). The ligation mixture was used to transform E. coli JM109. The resulting plasmid was used as an internal control for Mycobacterium tuberculosis. MTIS2F16 and MTIS2RAAC having nucleotide sequences of SEQ ID NOS:170 and 171 were synthesized. The length of the region amplified using the primer pair including the primer portions is 98 bp.

The reactions were carried out as follows. Reaction mixtures of final volumes of 25 µl containing the following at final concentrations were prepared: 32 mM HEPES-potassium hydroxide buffer (pH 7.8), 100 mM potassium acetate, 1% DMSO, 0.01% BSA, 0.1% propylenediamine, 4 mM magnesium acetate, 500 µM each of dNTPs, 25 pmol each of the primers MTIS2F16 and MTIS2RAAC, $10^3$ copies of the internal control for Mycobacterium tuberculosis, 2.2 U of Afu RNase HII or 8 U of Tli RNase HII, 11 U of BcaBEST DNA polymerase, 1 µl of a template and sterile water. 0, 1 or 10 pg of the BCG genomic DNA which was used in Example 1 was used as the template. The reaction mixtures were placed in Thermal Cycler Personal which had been set at 60°C and incubated for 60 minutes. The samples obtained after the ICAN reactions were spotted onto a nylon membrane, and dot blot hybridization was carried out according to the method as described in Example 5 using a probe for detecting Mycobacterium tuberculosis MTIS-S-PROBE (SEQ ID NO:172) labeled at the 5' end with biotin, or a probe for detecting the internal control INTER-PROBE (SEQ ID NO:173) labeled at the 5' end with biotin. As a result, when the samples obtained after the ICAN reactions with no addition of the BCG genomic DNA were spotted, no signal was observed using the MTIS-S-PROBE while signals were observed using the INTER-PROBE. When the samples obtained after the ICAN reactions with the addition of 1 pg of the BCG genomic DNA were spotted, signals were observed using both of the MTIS-S-PROBE and the INTER-PROBE. When the samples obtained after the ICAN reactions with the addition of 10 pg of the BCG genomic DNA were spotted, signals were observed using the MTIS-S-PROBE while no signal was observed using the INTER-PROBE. Based on these results, it was confirmed that a signal was observed using the MTIS-S-PROBE if the BCG genome as the target was amplified, whereas a signal was observed using the INTER-PROBE if the internal control was amplified. Thus, it was confirmed that a target nucleic acid can be specifically detected even in the presence of an internal control.according to the method of the present invention.

Industrial Applicability

The present invention provides a method for stabilization and long-term storage of a reaction reagent for a method for amplifying a target nucleic acid in which a region suitable for specific amplification in the nucleotide sequence of the target nucleic acid is amplified by a DNA synthesis reaction using a chimeric oligonucleotide primer, or a method for detecting a target nucleic acid which comprises a step of detecting a fragment amplified from the target nucleic acid obtained by the amplification method. The present invention also provides a method for detecting a target nucleic acid which is used for highly sensitive and specific detection or quantification of a microorganism (in particular, a pathogenic microorganism) such as a virus, a bacterium, a fungus or a yeast, as well as a chimeric oligonucleotide primer and a probe for the method.

Sequence Listing Free Text

SEQ ID NO:1: Designed oligonucleotide primer designated as pUC19 upper 150 to amplify a portion of plasmid pUC19.

SEQ ID NO:2: Designed oligonucleotide primer designated as pUC19 lower NN to amplify a portion of plasmid pUC19.

SEQ ID NO:3: Designed oligonucleotide primer designated as MCS-F to amplify a long DNA fragment

SEQ ID NO:4: Designed oligonucleotide primer designated as MCS-R to amplify a long DNA fragment

SEQ ID NO:5: Designed oligonucleotide primer designated as MF2 to amplify a portion of pUC19 plasmid DNA.

SEQ ID NO:6: Designed oligonucleotide primer designated as MR1 to amplify a portion of pUC19 plasmid DNA.

SEQ ID NO:7: Designed chimeric oligonucleotide primer designated as K-F-1033-2 to amplify a portion of Mycobacterium tuberculosis DNA. "nucleotides 19 to 21 are ribonucleotides-other nucleotides are deoxyribonucleotides."

SEQ ID NO:8: Designed chimeric oligonucleotide primer designated as K-R-1133-2 to amplify a portion of Mycobacterium tuberculosis DNA. "nucleotides 20 to 22 are ribonucleotides-other nucleotides are deoxyribonucleotides."

SEQ ID NO:9: Designed chimeric oligonucleotide primer to amplify a portion of INOS-encoding sequence from mouse. "nucleotides 21 to 23 are ribonucleotides-other nucleotides are deoxyribonucleotides"

SEQ ID NO:10: Designed chimeric oligonucleotide primer to amplify a portion of INOS-encoding sequence from mouse. "nucleotides 20 to 22 are ribonucleotides-other nucleotides are deoxyribonucleotides"

SEQ ID NO:11: Designed oligonucleotide probe designated as MTIS to detect a DNA fragment amplifying a portion of Mycobacterium tuberculosis sequence.

SEQ ID NO:12: Designed oligonucleotide probe designated as MTIS-2 to detect a DNA fragment amplifying a portion of Mycobacterium tuberculosis sequence.

SEQ ID NO:13: Designed oligonucleotide primer designated as SP6-HCV-F to amplify a portion of HCV

SEQ ID NO:14: Designed oligonucleotide primer designated as T7-HCV-R to amplify a portion of HCV

SEQ ID NO:15: Designed chimeric oligonucleotide primer designated as HCV-A2-S to amplify a portion of HCV. "nucleotides 16 to 18 are ribonucleotides-other nucleotides are deoxyribonucleotides."

SEQ ID NO:16: Designed chimeric oligonucleotide primer designated as HCV-A2-A to amplify a portion of HCV. "nucleotides 18 to 20 are ribonucleotides-other nucleotides are deoxyribonucleotides."

SEQ ID NO:17: Designed chimeric oligonucleotide primer designated as CT2F to amplify a portion of Chlamydia trachomatis cryptic plasmid DNA. "Nucleotides 18 to 20 are ribonucleotides-other nucleotides are deoxyribonucleotides."

SEQ ID NO:18: Designed chimeric oligonucleotide primer designated as CT2R to amplify a portion of Chlamydia trachomatis cryptic plasmid DNA. "Nucleotides 16 to 18 are ribonucleotides-other nucleotides are deoxyribonucleotides."

SEQ ID NO:19: Designed chimeric oligonucleotide primer designated as CT-FB19-3 to amplify a portion of Chlamydia trachomatics cryptic plasmid DNA. "Nucleotides 20 to 22 are ribonucleotides-other nucleotides are deoxyribonucleotides."

SEQ ID NO:20: Designed chimeric oligonucleotide primer designated as CT-RB23-2 to amplify a portion of Chlamydia trachomatics cryptic plasmid DNA. "Nucleotides 21 to 23 are ribonucleotides-other nucleotides are deoxyribonucleotides."

SEQ ID NO:21: Designed chimeric oligonucleotide primer designated as K-F-1033(68) to amplify a portion of Mycobacterium tuberculosis DNA."nucleotides 20 to 22 are ribonucleotides-other nucleotides are deoxyribonucleotides."

SEQ ID NO:22: Designed chimeric oligonucleotide primer designated as K-R-1133(68) to amplify a portion of Mycobacterium tuberculosis DNA. "nucleotides 20 to 22 are ribonucleotides-other nucleotides are deoxyribonucleotides."

SEQ ID NO:23: Nucleotide sequence of 16S rRNA from Mycobacterium avium

SEQ ID NO:24: Nucleotide sequence of 16S rRNA from Mycobacterium intracellulare

SEQ ID NO:25: Designed chimeric oligonucleotide primer designated as Myco-F-1 to amplify a portion of Mycobacterium 16S rRNA. "Nucleotides 18 to 20 are ribonucleotides-other nucleotides are deoxyribonucleotides."

SEQ ID NO:26: Designed chimeric oligonucleotide primer designated as Myco-F-2 to amplify a portion of Mycobacterium 16S rRNA. "Nucleotides 16 to 18 are ribonucleotides-other nucleotides are deoxyribonucleotides."

SEQ ID NO:27: Designed chimeric oligonucleotide primer designated as Myco-F-3 to amplify a portion of Mycobacterium 16S rRNA. "Nucleotides 16 to 18 are ribonucleotides-other nucleotides are deoxyribonucleotides."

SEQ ID NO:28: Designed chimeric oligonucleotide primer designated as Myco-R-1 to amplify a portion of Mycobacterium 16S rRNA. "Nucleotides 17 to 19 are ribonucleotides-other nucleotides are deoxyribonucleotides."

SEQ ID NO:29: Designed chimeric oligonucleotide primer designated as Myco-R-1-2 to amplify a portion of Mycobacterium 16S rRNA. "Nucleotides 16 to 18 are ribonucleotides-other nucleotides are deoxyribonucleotides."

SEQ ID NO:30: Designed chimeric oligonucleotide primer designated as Myco-R-2I to amplify a portion of 16S rRNA-encoding sequence from Mycobacterium intracellulare. "Nucleotides 18 to 20 are ribonucleotides-other nucleotides are deoxyribonucleotides."

SEQ ID NO:31: Designed chimeric oligonucleotide primer designated as Myco-R-2A to amplify a portion of 16S rRNA-encoding sequence from Mycobacterium avium. "Nucleotides 18 to 20 are ribonucleotides-other nucleotides are deoxyribonucleotides."

SEQ ID NO:32: Designed oligonucleotide probe as avium-probe to detect a DNA fragment amplifing a portion of 16S rRNA-encoding sequence from Mycobacterium avium.

SEQ ID NO:33: Designed oligonucleotide probe as intracellulare-probe to detect a DNA fragment amplifing a portion of 16S rRNA-encoding sequence from Mycobacterium intracellurare.

SEQ ID NO:34: Nucleotide sequence of CppB gene from Neisseria gonorrhorae

SEQ ID NO:35: Nucleotide sequence of pJD4 plasmid DNA from Neisseria gonorrhorae

SEQ ID NO:36: Nucleotide sequence of M.NgoMIII gene from Neisseria gonorrhorae

SEQ ID NO:37: Nucleotide sequence of Cytosine methyltransferase from Neisseria gonorrhorae

SEQ ID NO:38: Designed chimeric oligonucleotide primer designated as NEI-5103 to amplify a portion of pJDB4 plasmid DNA from Neisseria gonorrhoeae. "Nucleotides 20 to 22 are ribonucleotides-other nucleotides are deoxyribonucleotides."

SEQ ID NO:39: Designed chimeric oligonucleotide primer designated as NEI-5150 to amplify a portion of pJDB4 plasmid DNA from Neisseria gonorrhoeae. "Nucleotides 20 to 22 are ribonucleotides-other nucleotides are deoxyribonucleotides."

SEQ ID NO:40: Designed chimeric oligonucleotide primer designated as NEI-CppB-F1 to amplify a portion of CppB gene from Neisseria gonorrhoeae. "Nucleotides 17 to 19 are ribonucleotides-other nucleotides are deoxyribonucleotides."

SEQ ID NO:41: Designed chimeric oligonucleotide primer designated as NEI-CppB-F2 to amplify a portion of CppB gene from Neisseria gonorrhoeae. "Nucleotides 18 to 20 are ribonucleotides-other nucleotides are deoxyribonucleotides."

SEQ ID NO:42: Designed chimeric 'oligonucleotide primer designated as NEI-CppB-F3 to amplify a portion of CppB gene from Neisseria gonorrhoeae. "Nucleotides 16 to 18 are ribonucleotides-other nucleotides are deoxyribonucleotides."

SEQ ID NO:43: Designed chimeric oligonucleotide primer designated as NEI-CppB-R1 to amplify a portion of CppB gene from Neisseria gonorrhoeae. "Nucleotides 18 to 20 are ribonucleotides-other nucleotides are deoxyribonucleotides."

SEQ ID NO:44: Designed chimeric oligonucleotide primer designated as NEI-CppB-R2 to amplify a portion of CppB gene from Neisseria gonorrhoeae. "Nucleotides 17 to 19 are ribonucleotides-other nucleotides are deoxyribonucleotides."

SEQ ID NO:45: Designed chimeric oligonucleotide primer designated as NEI-CppB-R3 to amplify a portion of CppB gene from Neisseria gonorrhoeae. "Nucleotides 19 to 21 are ribonucleotides-other nucleotides are deoxyribonucleotides."

SEQ ID NO:46: Designed chimeric oligonucleotide primer designated as pJDB F-1 to amplify a portion of CppB gene from Neisseria gonorrhoeae. "Nucleotides 18 to 20 are ribonucleotides-other nucleotides are deoxyribonucleotides."

SEQ ID NO:47: Designed chimeric oligonucleotide primer designated as pJDB F-2 to amplify a portion of CppB gene from Neisseria gonorrhoeae. "Nucleotides 19 to 21 are ribonucleotides-other nucleotides are deoxyribonucleotides."

SEQ ID NO:48: Designed chimeric oligonucleotide primer designated as pJDB R-1 to amplify a portion of CppB gene from Neisseria gonorrhoeae. "Nucleotides 13 to 15 are ribonucleotides-other nucleotides are deoxyribonucleotides."

SEQ ID NO:49: Designed chimeric oligonucleotide primer designated as pJDB R-2 to amplify a portion of CppB gene from Neisseria gonorrhoeae. "Nucleotides 14 to 16 are ribonucleotides-other nucleotides are deoxyribonucleotides."

SEQ ID NO:50: Designed chimeric oligonucleotide primer designated as pJDB R-3 to amplify a portion of CppB gene from Neisseria gonorrhoeae. "Nucleotides 15 to 17 are ribonucleotides-other nucleotides are deoxyribonucleotides."

SEQ ID NO:51: Designed chimeric oligonucleotide primer designated as M.Ngo F-1 to amplify a portion of M:NgoMIII gene from Neisseria gonorrhoeae. "Nucleotides 19 to 21 are ribonucleotides-other nucleotides are deoxyribonucleotides."

SEQ ID NO:52: Designed chimeric oligonucleotide primer designated as M.Ngo F-2 to amplify a portion of M.NgoMIII gene from Neisseria gonorrhoeae. "Nucleotides 18 to 20 are ribonucleotides-other nucleotides are deoxyribonucleotides."

SEQ ID NO:53: Designed chimeric oligonucleotide primer designated as M.Ngo F-3 to amplify a portion of M.NgoMIII gene from Neisseria gonorrhoeae. "Nucleotides 17 to 19 are ribonucleotides-other nucleotides are deoxyribonucleotides."

SEQ ID NO:54: Designed chimeric oligonucleotide primer designated as M.Ngo R-1 to amplify a portion of M.NgoMIII gene from Neisseria gonorrhoeae. "Nucleotides 18 to 20 are ribonucleotides-other nucleotides are deoxyribonucleotides."

SEQ ID NO:55: Designed chimeric oligonucleotide primer designated as M.Ngo R-2 to amplify a portion of M.NgoMIII gene from Neisseria gonorrhoeae. "Nucleotides 17 to 19 are ribonucleotides-other nucleotides are deoxyribonucleotides."

SEQ ID NO:56: Designed chimeric oligonucleotide primer designated as M.Ngo R-3 to amplify a portion of M.NgoMIII gene from Neisseria gonorrhoeae. "Nucleotides 16 to 18 are ribonucleotides-other nucleotides are deoxyribonucleotides."

SEQ ID NO:57: Designed chimeric oligonucleotide primer designated as M.Ngo R-4 to amplify a portion of M.NgoMZII gene from Neisseria gonorrhoeae. "Nucleotides 19 to 21 are ribonucleotides-other nucleotides are deoxyribonucleotides."

SEQ ID NO:58: Designed chimeric oligonucleotide primer designated as Cytosine F-1 to amplify a portion of methyltransferase gene from Neisseria gonorrhoeae. "Nucleotides 18 to 20 are ribonucleotides-other nucleotides are deoxyribonucleotides."

SEQ ID NO:59: Designed chimeric oligonucleotide primer designated as Cytosine F-2 to amplify a portion of methyltransferase gene from Neisseria gonorrhoeae. "Nucleotides 19 to 21 are ribonucleotides-other nucleotides are deoxyribonucleotides."

SEQ ID NO:60: Designed chimeric oligonucleotide primer designated as Cytosine F-3 to amplify a portion of methyltransferase gene from Neisseria gonorrhoeae. "Nucleotides 17 to 19 are ribonucleotides-other nucleotides are deoxyribonucleotides."

SEQ ID NO:61: Designed chimeric oligonucleotide primer designated as Cytosine R-1 to amplify a portion of methyltransferase gene from Neisseria gonorrhoeae. "Nucleotides 18 to 20 are ribonucleotides-other nucleotides are deoxyribonucleotides."

SEQ ID NO:62: Designed chimeric oligonucleotide primer designated as Cytosine R-2 to amplify a portion of methyltransferase gene from Neisseria gonorrhoeae. "Nucleotides 19 to 21 are ribonucleotides-other nucleotides are deoxyribonucleotides."

SEQ ID NO:63: Designed chimeric oligonucleotide primer designated as Cytosine R-3 to amplify a portion of methyltransferase gene from Neisseria gonorrhoeae. "Nucleotides 20 to 22 are ribonucleotides-other nucleotides are deoxyribonucleotides."

SEQ ID NO:64: Designed oligonucleotide probe as NEI-5130 probe to detect a DNA fragment amplifing a portion of pJDB4 plasmid DNA from Neisseria gonorrhoeae.

SEQ ID NO:65: Designed oligonucleotide probe as NEI-CppB probe-1 to detect a DNA fragment amplifing a portion of CppB-encoding sequence from Neisseria gonorrhoeae.

SEQ ID NO:66: Designed oligonucleotide probe as NEI-CppB probe-2 to detect a DNA fragment amplifing a portion of CppB-encoding sequence from Neisseria gonorrhoeae.

SEQ ID NO:67: Designed oligonucleotide probe as M.Ngo-probe to detect a DNA fragment amplifing a portion of M.NgoMIII gene-encoding sequence from Neisseria gonorrhoeae.

SEQ ID NO:68: Designed oligonucleotide probe as Cytosine-probe to detect a DNA fragment amplifing a portion of methyltransferase-encoding sequence from Neisseria gonorrhoeae.

SEQ ID NO:69: Nucleotide sequence of X-protein from Hepatitis B virus

SEQ ID NO:70: Nucleotide sequence of X-protein from Hepatitis B virus

SEQ ID NO:71: Designed chimeric oligonucleotide primer designated as HBV-F-1 to amplify a portion of X-protein gene from Hepatitis B virus. "Nucleotides 22 to 24 are ribonucleotides-other nucleotides are deoxyribonucleotides."

SEQ ID NO:72: Designed chimeric oligonucleotide primer designated as HBV-F-2 to amplify a portion of X-protein gene from Hepatitis B virus. "Nucleotides 18 to 20 are ribonucleotides-other nucleotides are deoxyribonucleotides."

SEQ ID NO:73: Designed chimeric oligonucleotide primer designated as HBV-F-3 to amplify a portion of X-protein gene from Hepatitis B virus. "Nucleotides 19 to 21 are ribonucleotides-other nucleotides are deoxyribonucleotides."

SEQ ID NO:74: Designed chimeric oligonucleotide primer designated as HBV-F-5 to amplify a portion of X-protein gene from Hepatitis B virus. "Nucleotides 22 to 24 are ribonucleotides-other nucleotides are deoxyribonucleotides."

SEQ ID NO:75: Designed chimeric oligonucleotide primer designated as HBV-R-1 to amplify a portion of X-protein gene from Hepatitis B virus. "Nucleotides 20 to 22 are ribonucleotides-other nucleotides are deoxyribonucleotides."

SEQ ID NO:76: Designed chimeric oligonucleotide primer designated as HBV-R-2 to amplify a portion of X-protein gene from Hepatitis B virus. "Nucleotides 21 to 23 are ribonucleotides-other nucleotides are deoxyribonucleotides."

SEQ ID NO:77: Designed chimeric oligonucleotide primer designated as HBV-R-3 to amplify a portion of X-protein gene from Hepatitis B virus. "Nucleotides 19 to 21 are ribonucleotides-other nucleotides are deoxyribonucleotides."

SEQ ID NO:78: Designed chimeric oligonucleotide primer designated as HBV-R-4 to amplify a portion of X-protein gene from Hepatitis B virus. "Nucleotides 22 to 24 are ribonucleotides-other nucleotides are deoxyribonucleotides."

SEQ ID NO:79: Designed oligonucleotide probe as HBV-probel to detect a DNA fragment amplifing a portion of X-protein-encoding sequence from Hepatitis B virus.

SEQ ID NO:80: Designed oligonucleotide probe as HBV-probe2 to detect a DNA fragment amplifing a portion of X-protein-encoding sequence from Hepatitis B virus.

SEQ ID NO:81: Designed chimeric oligonucleotide primer designated as HCV-A2-S to amplify a portion of Hepatitis C virus gene. "Nucleotides 16 to 18 are ribonucleotides-other nucleotides are deoxyribonucleotides."

SEQ ID NO:82: Designed chimeric oligonucleotide primer designated as HCV-A2-A to amplify a portion of Hepatitis C virus. "Nucleotides 18 to 20 are ribonucleotides-other nucleotides are deoxyribonucleotides."

SEQ ID NO:83: Designed chimeric oligonucleotide primer designated as HCV-A4-S to amplify a portion of Hepatitis C virus. "Nucleotides 15 to 17 are ribonucleotides-other nucleotides are deoxyribonucleotides."

SEQ ID NO:84: Designed chimeric oligonucleotide primer designated as HCV-A4-A to amplify a portion of Hepatitis C virus. "Nucleotides 16 to 18 are ribonucleotides-other nucleotides are deoxyribonucleotides."

SEQ ID NO:85: Designed chimeric oligonucleotide primer designated as HCV-A4-S19 to amplify a portion of Hepatitis C virus. "Nucleotides 17 to 19 are ribonucleotides-other nucleotides are deoxyribonucleotides."

SEQ ID NO:86: Designed chimeric oligonucleotide primer designated as HCV-A4-A19 to amplify a portion of Hepatitis C virus. "Nucleotides 17 to 19 are ribonucleotides-other nucleotides are deoxyribonucleotides."

SEQ ID NO:87: Designed oligonucleotide probe as HCV-C probe to detect a DNA fragment amplifing a portion of Hepatitis C virus.

SEQ ID NO:88: Designed chimeric oligonucleotide primer designated as HCV-F1 to amplify a portion of Hepatitis C virus. "Nucleotides 17 to 19 are ribonucleotides-other nucleotides are deoxyribonucleotides."

SEQ ID NO:89: Designed chimeric oligonucleotide primer designated as HCV-R1 to amplify a portion of Hepatitis C virus. "Nucleotides 16 to 18 are ribonucleotides-other nucleotides are deoxyribonucleotides."

SEQ ID NO:90: Designed chimeric oligonucleotide primer designated as HCV-F2 to amplify a portion of Hepatitis C virus. "Nucleotides 16 to 18 are ribonucleotides-other nucleotides are deoxyribonucleotides."

SEQ ID NO:91: Designed chimeric oligonucleotide primer designated as HCV-R2 to amplify a portion of Hepatitis C virus. "Nucleotides 17 to 19 are ribonucleotides-other nucleotides are deoxyribonucleotides."

SEQ ID NO:92: Designed oligonucleotide probe as HCV-D probe to detect a DNA fragment amplifing a Hepatitis C virus.

SEQ ID NO:93: Designed oligonucleotide primer designated as SP6-HIV-F to amplify a portion of gag sequence from HIV.

SEQ ID NO:94: Designed oligonucleotide primer designated as HIV-R to amplify a portion of gag sequence from HIV.

SEQ ID NO:95: Designed chimeric oligonucleotide primer designated as HIV-F1 to amplify a portion of gag sequence from HIV. "Nucleotides 18 to 20 are ribonucleotides-other nucleotides are deoxyribonucleotides."

SEQ ID NO:96: Designed chimeric oligonucleotide primer designated as HIV-F2 to amplify a portion of gag sequence from HIV. "Nucleotides 20 to 22 are ribonucleotides-other nucleotides are deoxyribonucleotides."

SEQ ID NO:97: Designed chimeric oligonucleotide primer designated as HIV-F3 to amplify a portion of gag sequence from HIV. "Nucleotides 20 to 22 are ribonucleotides-other nucleotides are deoxyribonucleotides."

SEQ ID NO:98: Designed chimeric oligonucleotide primer designated as HIV-F4 to amplify a portion of gag sequence from HIV. "Nucleotides 20 to 22 are ribonucleotides-other nucleotides are deoxyribonucleotides."

SEQ ID NO:99: Designed chimeric oligonucleotide primer designated as HIV-R1 to amplify a portion of gag sequence from HIV. "Nucleotides 18 to 20 are ribonucleotides-other nucleotides are deoxyribonucleotides."

SEQ ID NO:100: Designed chimeric oligonucleotide primer designated as HIV-R2 to amplify a portion of gag sequence from HIV. "Nucleotides 20 to 22 are ribonucleotides-other nucleotides are deoxyribonucleotides."

SEQ ID NO:101: Designed chimeric oligonucleotide primer designated as HIV-R3 to amplify a portion of gag sequence from HIV. "Nucleotides 20 to 22 are ribonucleotides-other nucleotides are deoxyribonucleotides."

SEQ ID NO:102: Designed chimeric oligonucleotide primer designated as HIV-R4 to amplify a portion of gag sequence from HIV. "Nucleotides 20 to 22 are ribonucleotides-other nucleotides are deoxyribonucleotides."

SEQ ID NO:103: Designed chimeric oligonucleotide primer designated as HIV-R4M to amplify a portion of gag sequence from HIV. "Nucleotides 20 to 22 are ribonucleotides-other nucleotides are deoxyribonucleotides."

SEQ ID NO:104: Designed oligonucleotide probe as HIV-A probe to detect a DNA fragment amplifing a portion of gag sequence from HIV.

SEQ ID NO:105: Designed oligonucleotide probe as HIV-B probe to detect a DNA fragment amplifing a portion of gag sequence from HIV.

SEQ ID NO:106: Designed oligonucleotide primer designated as coa-PCR-F to amplify a portion of coagulase gene from Staphylococcus aureus.

SEQ ID NO:107: Designed oligonucleotide primer designated as coa-PCR-R to amplify a portion of coagulase gene from Staphylococcus aureus.

SEQ ID NO:108: Designed chimeric oligonucleotide primer designated as coa-F1 to amplify a portion of coagulase gene from Staphylococcus aureus. "Nucleotides 16 to 18 are ribonucleotides-other nucleotides are deoxyribonucleotides."

SEQ ID NO:109: Designed chimeric oligonucleotide primer designated as coa-F2 to amplify a portion of coagulase gene from Staphylococcus aureus. "Nucleotides 17 to 19 are ribonucleotides-other nucleotides are deoxyribonucleotides."

SEQ ID NO:110: Designed chimeric oligonucleotide primer designated as coa-F3 to amplify a portion of coagulase gene from Staphylococcus aureus. "Nucleotides 18 to 20 are ribonucleotides-other nucleotides are deoxyribonucleotides."

SEQ ID NO:111: Designed chimeric oligonucleotide primer designated as coa-F4 to amplify a portion of coagulase gene from Staphylococcus aureus. "Nucleotides 18 to 20 are ribonucleotides-other nucleotides are deoxyribonucleotides."

SEQ ID NO:112: Designed chimeric oligonucleotide primer designated as coa-F5 to amplify a portion of coagulas genee from Staphylococcus aureus. "Nucleotides 16 to 18 are ribonucleotides-other nucleotides are deoxyribonucleotides."

SEQ ID NO:113: Designed chimeric oligonucleotide primer designated as coa-R1 to amplify a portion of coagulase gene from Staphylococcus aureus. "Nucleotides 16 to 18 are ribonucleotides-other nucleotides are deoxyribonucleotides."

SEQ ID NO:114: Designed chimeric oligonucleotide primer designated as coa-R2 to amplify a portion of coagulase gene from Staphylococcus aureus. "Nucleotides 16 to 18 are ribonucleotides-other nucleotides are deoxyribonucleotides."

SEQ ID NO:115: Designed chimeric oligonucleotide primer designated as coa-R3 to amplify a portion of coagulase gene from Staphylococcus aureus. "Nucleotides 18 to 20 are ribonucleotides-other nucleotides are deoxyribonucleotides."

SEQ ID NO:116: Designed chimeric oligonucleotide primer designated as coa-R4 to amplify a portion of coagulase gene from Staphylococcus aureus. "Nucleotides 18 to 20 are ribonucleotides-other nucleotides are deoxyribonucleotides."

SEQ ID NO:117: Designed chimeric oligonucleotide primer designated as coa-R5 to amplify a portion of coagulase gene from Staphylococcus aureus. "Nucleotides 16 to 18 are ribonucleotides-other nucleotides are deoxyribonucleotides."

SEQ ID NO:118: Designed oligonucleotide probe as coa-A probe to detect a DNA fragment amplifing a coagulase gene from Staphylococcus aureus.

SEQ ID NO:119: Designed oligonucleotide probe as coa-B probe to detect a DNA fragment amplifing a coagulase gene from Staphylococcus aureus.

SEQ ID NO:120: Nucleotide sequence of criptic plasmid from Chlamydia trachomatis

SEQ ID NO:121 Designed chimeric oligonucleotide primer designated as CT-FB19 to amplify a portion of plasmid DNA from Chlamydia trachomatis . "Nucleotides 17 to 19 are ribonucleotides-other nucleotides are deoxyribonucleotides."

SEQ ID NO:122: Designed chimeric oligonucleotide primer designated as CT-FB19-3 to amplify a portion of plasmid DNA from Chlamydia trachomatis . "Nucleotides 20 to 22 are ribonucleotides-other nucleotides are deoxyribonucleotides."

SEQ ID NO:123: Designed chimeric oligonucleotide primer designated as CT-FB19-3-21 to amplify a portion of plasmid DNA from Chlamydia trachomatis . "Nucleotides 19 to 21 are ribonucleotides-other nucleotides are deoxyribonucleotides."

SEQ ID NO:124: Designed chimeric oligonucleotide primer designated as CT-FB19-3-23 to amplify a portion of plasmid DNA from Chlamydia trachomatis . "Nucleotides 21 to 23 are ribonucleotides-other nucleotides are deoxyribonucleotides."

SEQ ID NO:125: Designed chimeric oligonucleotide primer designated as CT-RB21 to amplify a portion of plasmid DNA from Chlamydia trachomatis . "Nucleotides 19 to 21 are ribonucleotides-other nucleotides are deoxyribonucleotides."

SEQ ID NO:126: Designed chimeric oligonucleotide primer designated as CT-RB23-2 to amplify a portion of plasmid DNA from Chlamydia trachomatis . "Nucleotides 21 to 23 are ribonucleotides-other nucleotides are deoxyribonucleotides."

SEQ ID NO:127: Designed chimeric oligonucleotide primer designated as CT-RB23-2-24 to amplify a portion of plasmid DNA from Chlamydia trachomatis . "Nucleotides 22 to 24 are ribonucleotides-other nucleotides are deoxyribonucleotides."

SEQ ID NO:128: Designed oligonucleotide probe as CT-probe to detect a DNA fragment amplifing a portion of plasmid DNA from Chlamydia trachomatis.

SEQ ID NO:129: Nucleotide sequence of criptic plasmid from Chlamydia trachomatis

SEQ ID NO:130: Designed chimeric oligonucleotide primer designated as CT-F1212-20 to amplify a portion of plasmid DNA from Chlamydia trachomatis . "Nucleotides 18 to 20 are ribonucleotides-other nucleotides are deoxyribonucleotides."

SEQ ID NO:131: Designed chimeric oligonucleotide primer designated as CT-F1212-21 to amplify a portion of plasmid DNA from Chlamydia trachomatis . "Nucleotides 19 to 21 are ribonucleotides-other nucleotides are deoxyribonucleotides."

SEQ ID NO:132: Designed chimeric oligonucleotide primer designated as CT-F1212-22 to amplify a portion of plasmid DNA from Chlamydia trachomatis . "Nucleotides 20 to 22 are ribonucleotides-other nucleotides are deoxyribonucleotides."

SEQ ID NO:133: Designed chimeric oligonucleotide primer designated as CT-R1272-20 to amplify a portion of plasmid DNA from Chlamydia trachomatis . "Nucleotides 18 to 20 are ribonucleotides-other nucleotides are deoxyribonucleotides."

SEQ ID NO:134: Designed chimeric oligonucleotide primer designated as CT-R1272-21 to amplify a portion of plasmid DNA from Chlamydia trachomatis . "Nucleotides 19 to 21 are ribonucleotides-other nucleotides are deoxyribonucleotides."

SEQ ID NO:135: Designed chimeric oligonucleotide primer designated as CT-R1272-22 to amplify a portion of plasmid DNA from Chlamydia trachomatis . "Nucleotides 20 to 22 are ribonucleotides-other nucleotides are deoxyribonucleotides."

SEQ ID NO:136: Designed oligonucleotide probe as CT-1234 probe to detect a DNA fragment amplifing a portion of plasmid DNA from Chlamydia trachomatis.

SEQ ID NO:137: Nucleotide sequence of ATPase operon from Mycoplasma pneumoniae

SEQ ID NO:138: Nucleotide sequence of ATPase operon from Mycoplasma pneumoniae

SEQ ID NO:139: Designed chimeric oligonucleotide primer designated as Myco-140 to amplify a portion of ATPase operon from Mycoplasma pneumoniae. "Nucleotides 19 to 21 are ribonucleotides-other nucleotides are deoxyribonucleotides."

SEQ ID NO:140: Designed chimeric oligonucleotide primer designated as Myco-140-22 to amplify a portion of ATPase operon from Mycoplasma pneumoniae. "Nucleotides 20 to 22 are ribonucleotides-other nucleotides are deoxyribonucleotides."

SEQ ID NO:141: Designed chimeric oligonucleotide primer designated as Myco-706 to amplify a portion of ATPase operon from Mycoplasma pneumoniae. "Nucleotides 20 to 22 are ribonucleotides-other nucleotides are deoxyribonucleotides."

SEQ ID NO:142: Designed chimeric oligonucleotide primer designated as MPF-910 to amplify a portion of ATPase operon from Mycoplasma pneumoniae. "Nucleotides 20 to 22 are ribonucleotides-other nucleotides are deoxyribonucleotides."

SEQ ID NO:143: Designed chimeric oligonucleotide primer designated as Myco-190 to amplify a portion of ATPase operon from Mycoplasma pneumoniae. "Nucleotides 19 to 21 are ribonucleotides-other nucleotides are deoxyribonucleotides."

SEQ ID NO:144: Designed chimeric oligonucleotide primer designated as Myco-190-22 to amplify a portion of ATPase operon from Mycoplasma pneumoniae. "Nucleotides 20 to 22 are ribonucleotides-other nucleotides are deoxyribonucleotides."

SEQ ID NO:145: Designed chimeric oligonucleotide. primer designated as Myco-850 to amplify a portion of ATPase operon from Mycoplasma pneumoniae. "Nucleotides 20 to 22 are ribonucleotides-other nucleotides are deoxyribonucleotides."

SEQ ID NO:146: Designed chimeric oligonucleotide primer designated as MPR-1016 to amplify a portion of ATPase operon from Mycoplasma pneumoniae. "Nucleotides 20 to 22 are ribonucleotides-other nucleotides are deoxyribonucleotides."

SEQ ID NO:147: Designed oligonucleotide probe as Myco170-probe to detect a DNA fragment amplifing a portion of ATPase operon from Mycoplasma pneumoniae.

SEQ ID NO:148: Designed oligonucleotide probe as Myco730-probe to detect a DNA fragment amplifing a portion of ATPase operon from Mycoplasma pneumoniae.

SEQ ID NO:149: Designed oligonucleotide probe as Myco952-probe to detect a DNA fragment amplifing a portion of ATPase operon from Mycoplasma pneumoniae.

SEQ ID NO:150: Nucleotide sequence of MecA gene from Staphylococcus aureus

SEQ ID NO:151: Nucleotide sequence of MecA gene from Staphylococcus aureus

SEQ ID NO:152: Designed chimeric oligonucleotide primer designated as MecA-S525 to amplify a portion of MecA gene from Staphylococcus aureus. "Nucleotides 21 to 23 are ribonucleotides-other nucleotides are deoxyribonucleotides."

SEQ ID NO:153: Designed chimeric oligonucleotide primer designated as MecA-A611 to amplify a portion of MecA gene from Staphylococcus aureus. "Nucleotides 18 to 20 are ribonucleotides-other nucleotides are deoxyribonucleotides."

SEQ ID NO:154: Designed chimeric oligonucleotide primer designated as MecA-S1281 to amplify a portion of MecA gene from Staphylococcus aureus. "Nucleotides 21 to 23 are ribonucleotides-other nucleotides are deoxyribonucleotides."

SEQ ID NO:155: Designed chimeric oligonucleotide primer designated as MecA-A1341 to amplify a portion of MecA gene from Staphylococcus aureus. "Nucleotides 21 to 23 are ribonucleotides-other nucleotides are deoxyribonucleotides."

SEQ ID NO:156: Designed oligonucleotide probe as MecA-A probe to detect a DNA fragment amplifing a portion of MecA gene from Staphylococcus aureus.

SEQ ID NO:157: Designed oligonucleotide probe as MecA-B probe to detect a DNA fragment amplifing a portion of MecA gene from Staphylococcus aureus.

SEQ ID NO:158: Designed chimeric oligonucleotide primer designated as pDON-AI-1(22) to amplify a portion of pDON-AI plasmid DNA. "Nucleotides 20 to 22 are ribonucleotides-other nucleotides are deoxyribonucleotides."

SEQ ID NO:159: Designed chimeric oligonucleotide primer designated as pDON-AI-2(23) to amplify a portion of pDON-AI plasmid DNA. "Nucleotides 21 to 23 are ribonucleotides-other nucleotides are deoxyribonucleotides."

SEQ ID NO:160: Designed oligonucleotide primer to amplify a portion of iNOS-encoding sequence from mouse.

SEQ ID NO:161: Designed oligonucleotide primer to amplify a portion of iNOS-encoding sequence from mouse.

SEQ ID NO:162: Designed chimeric oligonucleotide primer designated as MTIS 2F to amplify a portion of Mycobacterium tuberculosis sequence."Nucleotides 16 to 18 are ribonucleotides-other nucleotides are deoxyribonucleotides."

SEQ ID NO:163: Designed chimeric oligonucleotide primer designated as MTIS2R to amplify a portion of Mycobacterium tuberculosis sequence. "Nucleotides 19 to 21 are ribonucleotides-other nucleotides are deoxyribonucleotides."

SEQ ID NO:164: Designed chimeric oligonucleotide primer designated as pJDB10F to amplify a portion of CppB gene from Neisseria gonorrhoeae. "Nucleotides 18 to 20 are ribonucleotides-other nucleotides are deoxyribonucleotides."

SEQ ID NO:165: Designed chimeric oligonucleotide primer designated as pJDB10R to amplify a portion of CppB gene from Neisseria gonorrhoeae. "Nucleotides 15 to 17 are ribonucleotides-other nucleotides are deoxyribonucleotides."

SEQ ID NO:166: Designed chimeric oligonucleotide primer designated as CT-F1215-4R-22 to amplify a portion of Chlamidia trachomatis cryptic plasmid DNA. "Nucleotides 19 to 22 are ribonucleotides-other nucleotides are deoxyribonucleotides."

SEQ ID NO:167: Designed chimeric oligonucleotide primer designated as CT-R1267-3R-18 to amplify a portion of Chlamidia trachomatis cryptic plasmid DNA. "Nucleotides 16 to 18 are ribonucleotides-other nucleotides are deoxyribonucleotides."

SEQ ID NO:168: Designed oligonucleotide probe as CT-1236 probe to detect a DNA fragment amplifing a portion of plasmid DNA from Chlamydia trachomatis.

SEQ ID NO:169: Designed nucleotide as Internal Control for Mycobacterium tuberculosis assay.

SEQ ID NO:170: Designed chimeric oligonucleotide primer designated as MTIS2F16 to amplify a portion of Mycobacterium tuberculosis DNA. "nucleotides 14 to 16 are ribonucleotides-other nucleotides are deoxyribonucleotides."

SEQ ID NO:171: Designed chimeric oligonucleotide primer designated as MTIS2RAAC to amplify a portion of Mycobacterium tuberculosis DNA. "nucleotides 18 to 20 are ribonucleotides-other nucleotides are deoxyribonucleotides."

SEQ ID NO:172: Designed oligonucleotide probe designated as MTIS-S-PROBE to detect a DNA fragment amplifying a portion of nucleotide sequence from Mycobacterium tuberculosis.

SEQ ID NO:173: Designed oligonucleotide probe designated as INTER-PROBE to detect a DNA fragment amplifying a portion of nucleotide sequence from Mycobacterium tuberculosis.

**Claims**

1. A method for stabilizing a reaction reagent used for a method for amplifying and/or detecting a target nucleic acid that comprises:

(a) preparing a reaction mixture by mixing a nucleic acid as a template, a deoxyribonucleotide triphosphate, a DNA polymerase having a strand displacement activity, at least one primer and an RNase H, wherein the primer is a chimeric oligonucleotide primer that is substantially complementary to the nucleotide sequence of the nucleic acid as the template and contains a ribonucleotide as well as at least one selected from the group consisting of a deoxyribonucleotide and a nucleotide analog, the ribonucleotide being positioned at the 3'-terminus or on the 3'-terminal side of the primer; and
(b) amplifying a target nucleic acid by incubating the reaction mixture for a sufficient time to generate a reaction product,

wherein

(i) at least one reagent component selected from the group consisting of a magnesium salt, the chimeric oligonucleotide primer and the enzymes (the DNA polymerase and/or the RNase H) is separated from other reagent components prior to the reaction; and
(ii) the enzyme concentration(s) of a reagent solution containing the enzyme (s) is (are) elevated while the salt concentration of said solution is not elevated, and the salt concentration of another reagent solution is adjusted such that the optimal salt concentration for the amplification step is achieved after mixing the separated reagent solutions each other.

2. The method according to claim 1, wherein the reaction reagent consists of two reagent solutions: a reagent solution containing the chimeric oligonucleotide primer; and a reagent solution containing the enzyme(s) (the DNA polymerase and/or the RNase H) and the magnesium salt.

3. The method according to claim 1, wherein the salt concentration of the reagent solution containing the enzyme(s) (the DNA polymerase and/or the RNase H) is equal to or lower than the optimal salt concentration for the amplification step.

4. The method according to claim 1, wherein the enzyme concentration(s) of the reagent solution containing the enzyme(s) (the DNA polymerase and/or the RNase H) is (are) higher than the enzyme concentration(s) for the amplification step.

5. The method according to claim 4, wherein the enzyme concentration(s) of the reagent solution containing the enzyme(s) (the DNA polymerase and/or the RNase H) is (are) adjusted such that the optimal enzyme concentration (s) for the amplification step is (are) achieved after mixing the separated reagent solutions each other.

6. A kit of a reaction reagent used for a method for amplifying and/or detecting a target nucleic acid that comprises:

(a) preparing a reaction mixture by mixing a nucleic acid as a template, a deoxyribonucleotide triphosphate, a DNA polymerase having a strand displacement activity, at least one primer and an RNase H, wherein the primer is a chimeric oligonucleotide primer that is substantially complementary to the nucleotide sequence of the nucleic acid as the template and contains a ribonucleotide as well as at least one selected from the group consisting of a deoxyribonucleotide and a nucleotide analog, the ribonucleotide being positioned at the 3'-terminus or on the 3'-terminal side of the primer; and
(b) amplifying a target nucleic acid by incubating the reaction mixture for a sufficient time to generate a reaction product,

wherein

(i) at least one reagent component selected from the group consisting of a magnesium salt, the chimeric oligonucleotide primer and the enzymes (the DNA polymerase and/or the RNase H) is separated from other reagent components prior to the reaction; and
(ii) the enzyme concentration(s) of a reagent solution containing the enzyme(s) is (are) elevated while the salt concentration of said solution is not elevated, and the salt concentration of another reagent solution is adjusted such that the optimal salt concentration for the amplification step is achieved after mixing the separated reagent solutions each other.

**7.** The kit according to claim 6, wherein the reaction reagent consists of two reagent solutions: a reagent solution containing the chimeric oligonucleotide primer; and a reagent solution containing the enzyme(s) (the DNA polymerase and/or the RNase H) and the magnesium salt are used.

**8.** The kit according to claim 6, wherein the salt concentration of the reagent solution containing the enzyme(s) (the DNA polymerase and/or the RNase H) is equal to or lower than the optimal salt concentration for the amplification step.

**9.** The kit according to claim 6, wherein the enzyme concentration(s) of the reagent solution containing the enzyme (s) (the DNA polymerase and/or the RNase H) is (are) higher than the enzyme concentration(s) for the amplification step.

**10.** The kit according to claim 9, wherein the enzyme concentration(s) of the reagent solution containing the enzyme (s) (the DNA polymerase and/or the RNase H) is (are) adjusted such that the optimal enzyme concentration(s) for the amplification step is (are) achieved after mixing the separated reagent solutions each other.

**11.** The kit according to claim 6, which contains a reagent for adjusting the salt concentration of the mixture of the separated reagent solutions.

Figure 1

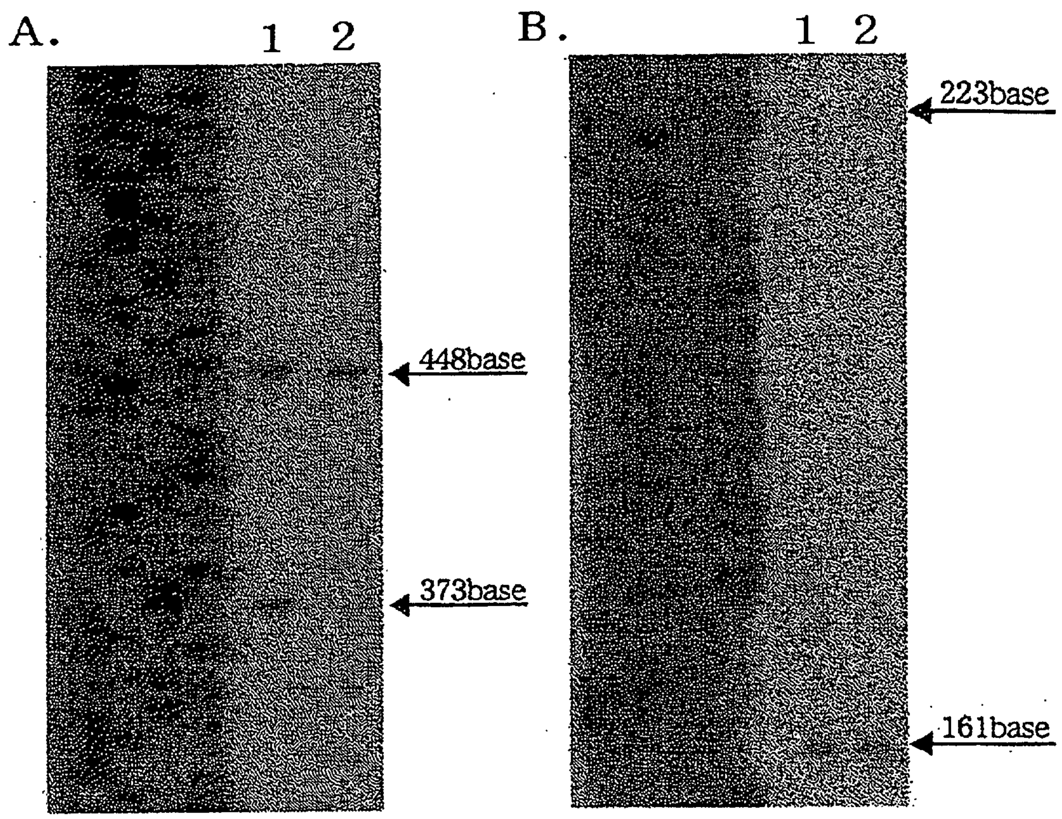

Figure 2

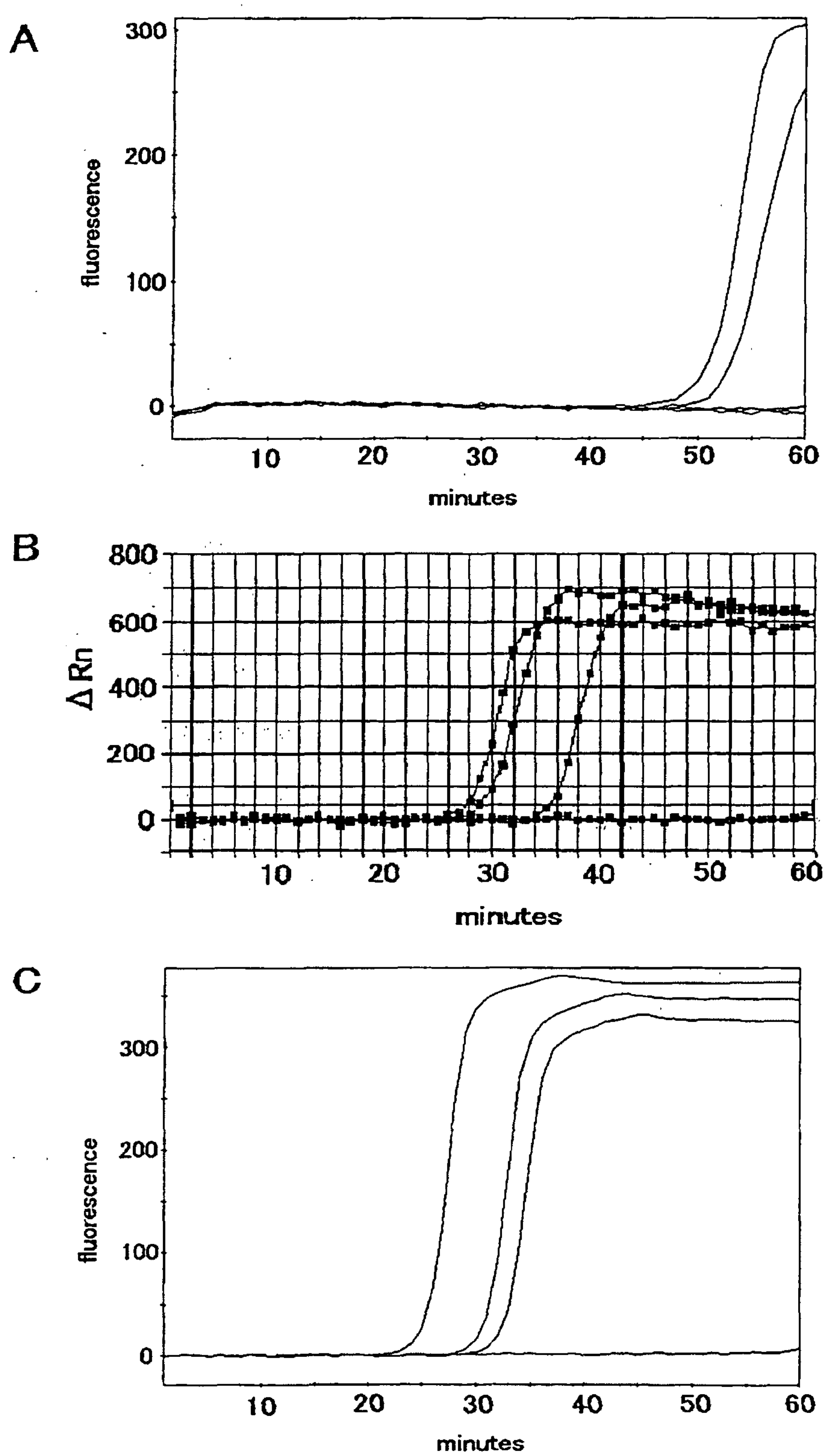
A
300
200
100
0
fluorescence
10
20
30
40
50
60
minutes
B
800
600
400
200
0
ΔRn
10
20
30
40
50
60
minutes
C
300
200
100
0
fluorescence
10
20
30
40
50
60
minutes

Figure 3

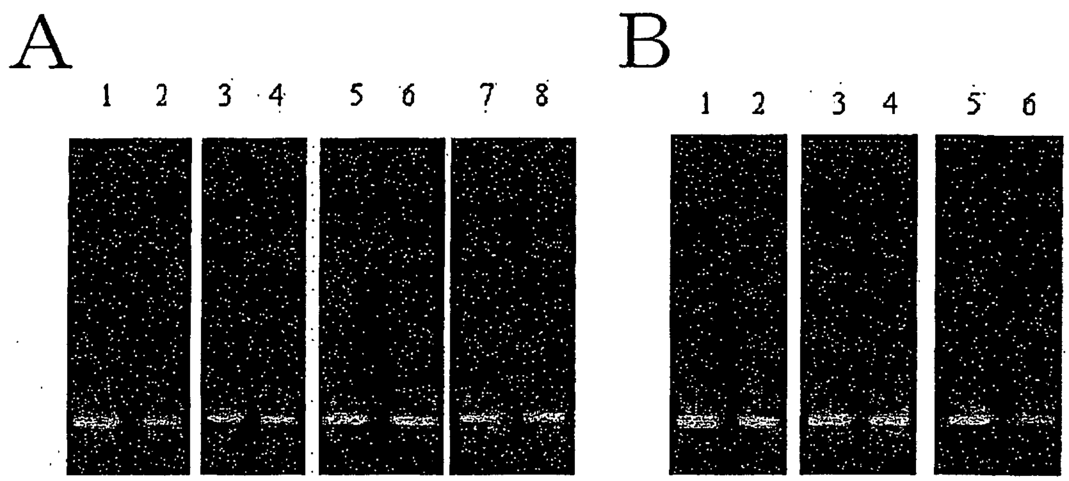

Figure 4

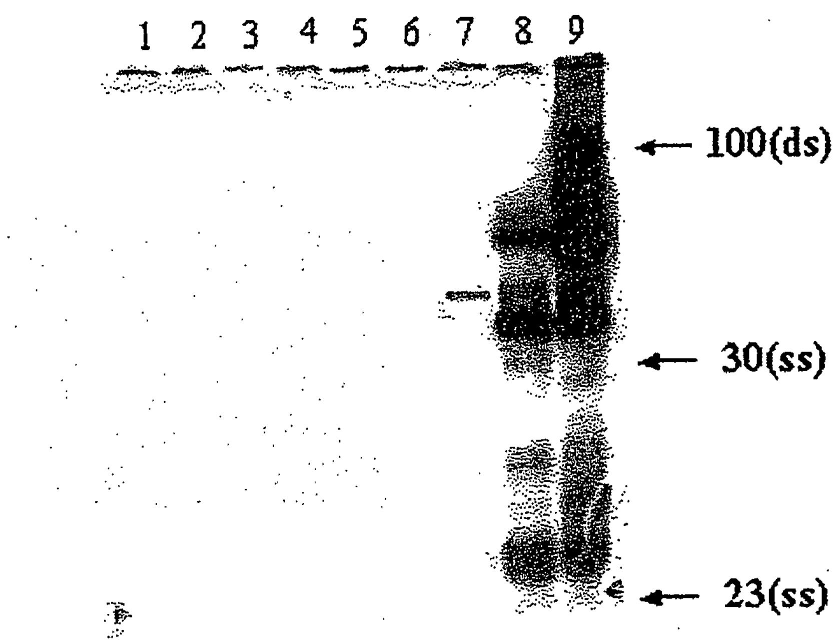

Figure 5

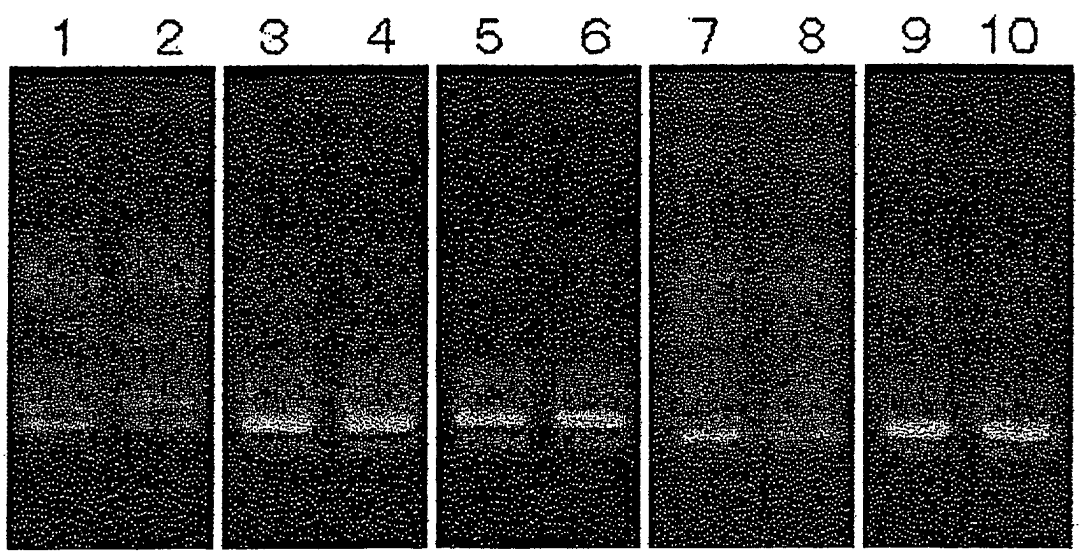

Figure 6

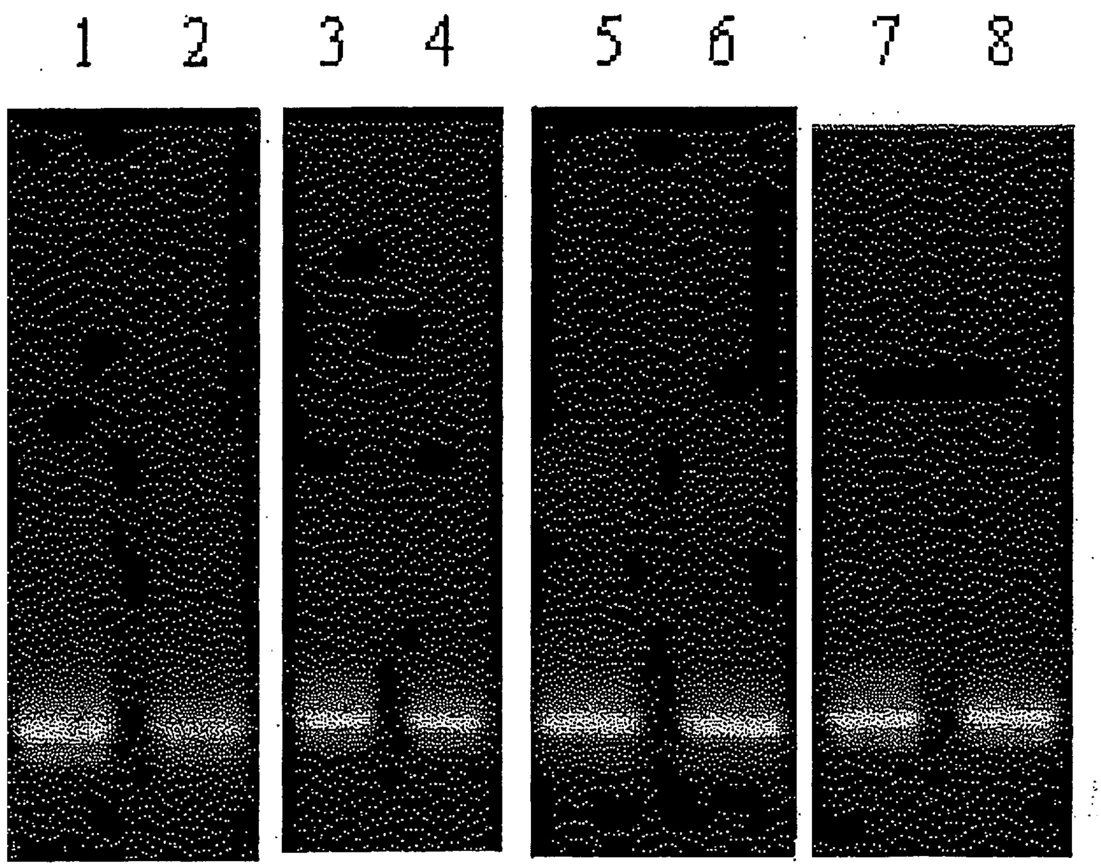

Figure 7

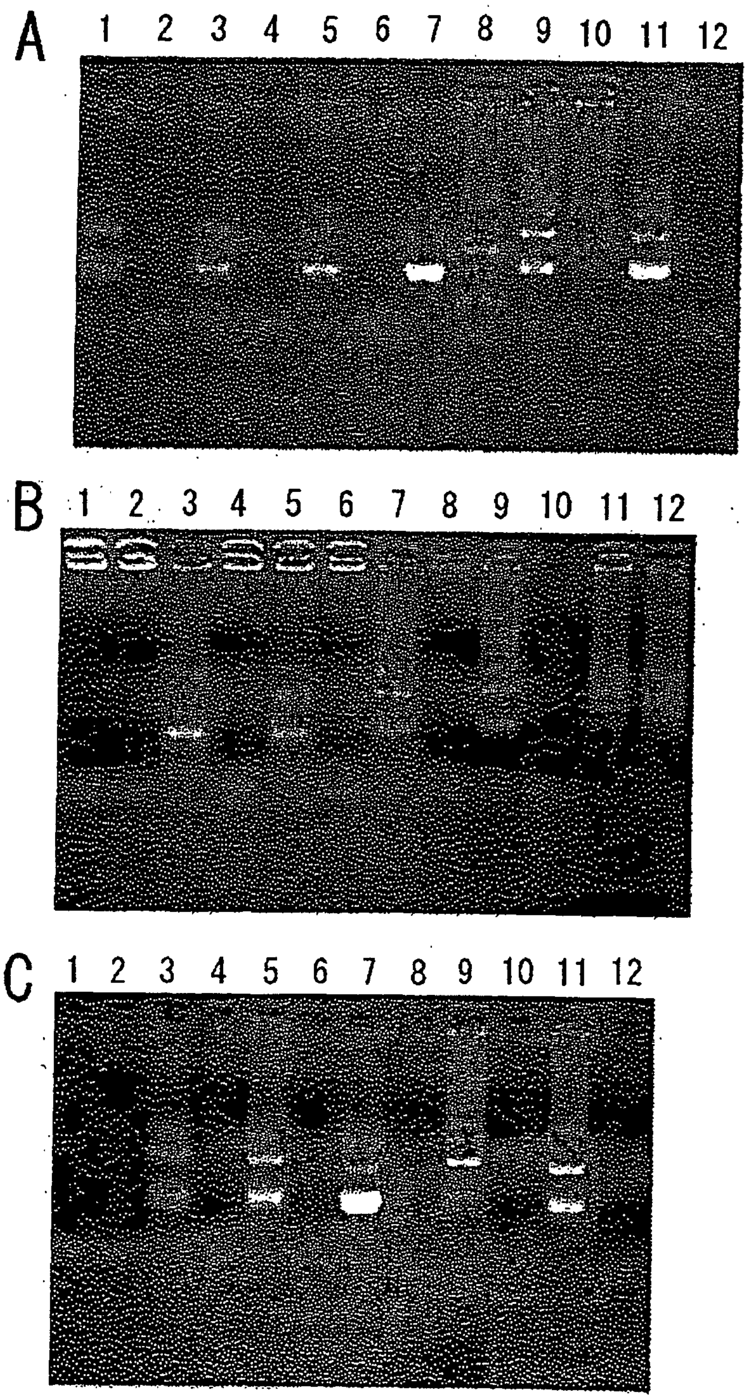

**INTERNATIONAL SEARCH REPORT**

International application No.
PCT/JP02/05832

A. CLASSIFICATION OF SUBJECT MATTER
Int.Cl[7] C12N15/09, C12Q1/68

According to International Patent Classification (IPC) or to both national classification and IPC

B. FIELDS SEARCHED
Minimum documentation searched (classification system followed by classification symbols)
Int.Cl[7] C12N15/09, C12Q1/68

Documentation searched other than minimum documentation to the extent that such documents are included in the fields searched

Electronic data base consulted during the international search (name of data base and, where practicable, search terms used)
BIOSIS(DIALOG), WPI(DIALOG), PubMed

C. DOCUMENTS CONSIDERED TO BE RELEVANT

| Category* | Citation of document, with indication, where appropriate, of the relevant passages | Relevant to claim No. |
|---|---|---|
| X | Supervised by Toshiharu MAKI, "Saibo Kogaku Bessatsu Tips Series Kaitei PCR Tips - Kanosei o Hirogeru Kotsu to Hint -", second edition, first print, Shujunsha Co., Ltd., 4-15-21 Nishiazabu Minato-Ku Tokyo-To, published by Haruki SUMA, 10 December, 1999 (10.12.99), ISBN4-87962-215-X, pages 182 to 188 | 1-11 |
| X | Hiroyuki MUKAI, La PCR, Protein, nucleic acid and enzyme. 1996, Vol.41, No.5, pages 179 to 188 | 1-11 |
| A | EP 590327 A2 (F.Hoffmann-La Roche AG), 06 April, 1994 (06.04.94), & US 5501963 A & JP 6-277062 A & CA 2105944 A | 1-11 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 09 July, 2002 (09.07.02) | 23 July, 2002 (23.07.02) |
| Name and mailing address of the ISA/ Japanese Patent Office | Authorized officer |
| Facsimile No. | Telephone No. |

Form PCT/ISA/210 (second sheet) (July 1998)